# EUROPEAN PATENT APPLICATION

(11) **EP 4 674 843 A1**
(43) Date of publication of application: **07.01.2026**
(21) Application number: 24186769.6
(22) Date of filing: 05.07.2024
(51) Int. Cl.: C07D 257/12, C07B 59/00, A61K 51/04, A61K 101/02, C07F 7/02, C07F 7/22, C07F 7/30

(54) **TETRAZINE ANALOGUES FOR THERANOSTIC APPLICATION**

(71) Applicant: University of Copenhagen, 1165 Copenhagen K (DK); Rigshospitalet, 2100 Copenhagen Ø (DK)
(72) Inventor: HERTH, Matthias Manfred, 21121 Malmö (SE); BATTISTI, Umberto Maria, 2100 Copenhagen (DK); KJÆR, Andreas, 2000 Frederiksberg (DK); HVASS, Lars, 1958 Frederiksberg C (DK)
(74) Representative: Budde Schou A/S

(57) **Abstract**

The present invention provides bioorthogonal tetrazine reaction entities for use in pretargeting approaches comprising imaging, therapy and diagnostics specifically pretargeting theranostics. The present invention also provides precursors of the tetrazine reaction entities and kits comprising the tetrazine reaction entity or the precursor of the tetrazine reaction entity and a dienophile with inverse electron demand Diels-Alder cycloaddition reactivity.

## Description

### FIELD

The present invention is within the field of bioorthogonal reaction entities for use in pretargeting approaches comprising imaging, therapy and diagnostics specifically pretargeting theranostics. Pretargeting imaging and radioimmunotherapy is efficiently combining the selectivity of antibodies and the properties of medicinal radioisotopes for high-contrast imaging and high-therapeutic-index. Unlike standard radioimmunoconjugates, pretargeted approaches separate the targeting step from the imaging/therapeutic step, which amplifies the targeting, such as tumor uptake. The tetrazine ligation is currently the fastest bioorthogonal reaction for this strategy. The present invention relates to tetrazine analogues that can be used for imaging when labelled with a bromine (⁷⁶Br) or fluorine radionuclide and for therapy when labelled with a bromine (⁷⁷Br), iodine or astatine radionuclide.

### BACKGROUND

Recently, theranostic radiopharmaceuticals have witnessed a surge in importance. This strategy relies on the utilization of matching pairs of radiopharmaceuticals for both diagnostic imaging and targeted radiotherapy. The application of companion nuclear imaging agents based on the same platform as the radiotherapeutic can aid in identifying patients who are more likely to respond positively to the treatment, as well as in optimizing the dosage of the radiotherapeutic and monitoring patients during treatment. This personalized approach has been successfully implemented in the clinic, with examples including the use DOTATATE and PSMA-617. This methodology is not limited to small molecules and peptides but can also be extended to monoclonal antibodies (mAbs). However, mAbs require radiolabeling with long-lived radionuclides to match their slow pharmacokinetics and ensure adequate radioactivity in the radioimmunoconjugate at the target site. While a few therapeutic radionuclides, such as 177Lu (6.7 days), 131I (8.0 days), and 225Ac (9.9 days), exhibit half-lives of a few days, they pose a risk of high radiation doses to healthy tissues due to their slow pharmacokinetics and long half-lives. In contrast, other radionuclides such as astatine, has a half-life of approximately 7.2 hours. This is considered ideal for pretargeted theranostics because it provides a sufficient window for the radiolabeled compound to localize to the target site while limiting the patient's exposure to radiation over time. It balances the need for rapid targeting with a manageable decay period.

The technical features of astatine moreover includes high linear energy transfer deposit meaning that a large amount of energy is deposited over a short distance, typically a few cell diameters. This is very effective in causing double-strand breaks in DNA, leading to cell death, which is particularly useful in targeting and killing cancer cells. The short path length of alpha particles (50-100 micrometers) ensures that the radiation damage is localized to the targeted cells, minimizing damage to surrounding healthy tissues. However, it is difficult to label tetrazines with astatine to a sufficient amount. Astatine is a halogen but behaves very differently from other halogens like fluorine and iodine due to its larger atomic radius and relatively low electronegativity which affects its binding to organic molecules. ²¹¹At is an alpha emitter emitting very high-energy particles. Compounds with astatine are often less stable compared to those with other halogens since the high-energy alpha emitter particles may cause damage to the molecules astatine is bound to, such as a tetrazine or an antibody. The actual chemical labeling process of ²¹¹At onto tetrazine is moreover challenging requiring optimal reaction conditions such as pH, temperature, and reaction time to ensure astatine binds effectively and stably to the tetrazine. These concerns and the fact that ²¹¹At is a rare isotope and is produced only in small amounts which limits the amount of material available for experiments and requires efficient methods for synthesis and labeling. Consequently, the widespread clinical application of antibodies in radiotherapy has been challenging.

Pretargeting is a technique that can help overcome some of the issues associated with traditional radioimmunotherapy. This approach involves the decoupling of the antibody and the radionuclide and injecting them separately. First, a modified antibody that can bind selectively both to a target such as a cancer antigen and to a radiolabeled small molecule is injected. After this antibody has had several days to accumulate in the target, such as in a tumor, and clear from the blood, the radiolabeled small molecule is administered. This second component travels through the bloodstream quickly and either interacts with the antibody remaining at the target site or rapidly leaves the body. This means that a radionuclide with a multiday physical half-life is no longer needed, which makes it possible to use short-lived nuclides that are normally incompatible with mAbs. This approach also reduces radiation dose rates to healthy tissues.

In the last years, new approaches for in vivo pretargeting has been developed exploiting the inverse electron demand Diels-Alder (IEDDA) ligation between a trans-cyclooctene (TCO)-modified antibody and a radiolabeled tetrazine (Tz). This bioorthogonal reaction is extremely fast and selective and allow the formation of stable dihydropyridazine cycloadducts even at radiotracers concentration in a living organism.

In particular, focus has been on developing new radiolabeling methods specifically tailored for the tetrazines, as well as improving their physicochemical properties for pretargeting imaging approaches. WO2022/189304 describes a method to radiolabel base sensitive tetrazine structures wherein tetrazines that were previously not accessible by applying standard aliphatic ¹⁸F-labeling strategies can be radiolabeled. WO2021/228992 describe H-tetrazine compounds comprising a radionuclide of F, I or At and one or more polar groups that can be used in pretargeted cancer diagnostics and cancer therapy. Even though key properties for tetrazines in pretargeting have been identified and new labeling methodologies tailored for tetrazines using ¹⁸F have been provided recently, there is, however, still a need for F, I or At labeled tetrazines that show high uptake at the target site, favorable pharmacokinetics as well as sufficient target-to-background ratios. Accordingly, the compounds provided will have improved use in particularly theranostics pretargeting approaches.

### SUMMARY

The present invention provides in a first aspect tetrazine compounds of formula I: 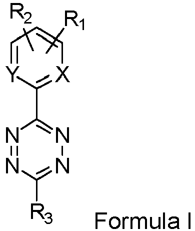 wherein R₁ is 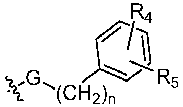
wherein G is selected from -O-, -S-, -NH-, and -N-alkyl-;
n is a digit from 0 to 4;
R₄ is a radioisotope selected from ¹⁸F, ¹⁹F, ⁷⁶Br, ⁷⁷Br, ¹²⁵I, ¹²³I, ¹³¹I, ¹²⁴I, ¹²⁷I and ²¹¹At;
R₅ is -H- or a polar group (PG) selected from: 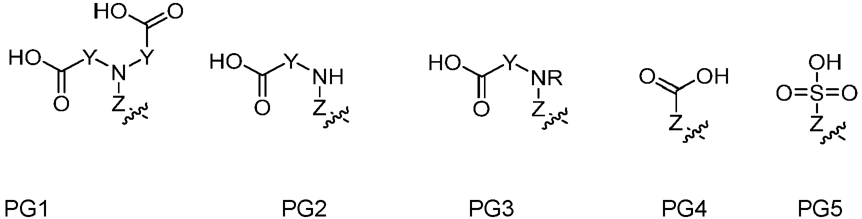
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 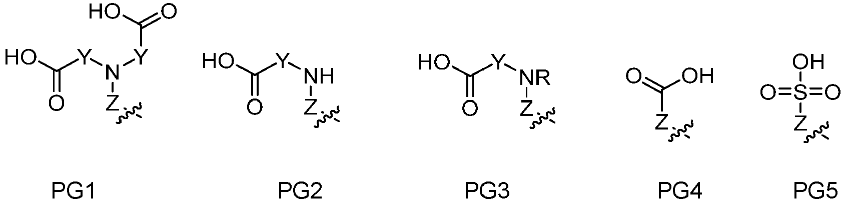
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 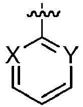
wherein X and Y are independently selected from: -CH₂- and -N-;
   and
wherein the tetrazine compound of formula I has a lipophilicity of cLogD7.4 < -0.5.

In a second aspect, the present invention relates compounds of formula I or use as a medicament in pretargeting based therapy, diagnostic, theranostic or imaging.

In a third aspect, the present invention relates to compounds of formula I for use in a method of diagnosing and/or treating cancer diseases, cardiovascular diseases, infectious diseases, neurological disorders, inflammatory diseases, bone disorders, thyroid diseases, renal diseases, or lymphatic disorders.

In a fourth aspect, the present invention relates to Method comprising:
a) administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream
c) Administering to the same subject a compound of formula I: 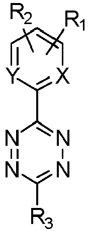 wherein R₁ is 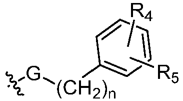
   wherein G is selected from -O-, -S-, -NH-, and -N-alkyl-;
   n is a digit from 0 to 4;
   R₄ is a radioisotope selected from ¹⁸F, ⁷⁶Br, ⁷⁷Br, ¹²⁵I, ¹²³I, ¹³¹I, ¹²⁴I, and ²¹¹At;
   R₅ is -H- or a polar group (PG) selected from: 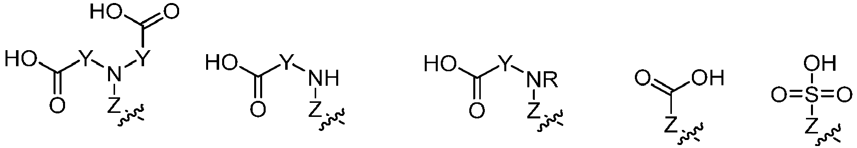 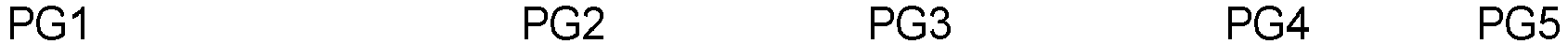
   wherein Z is CO(CH₂)ₙ, SO₂(CH₂)ₙ, (CH₂)ₙ or (OCH₂CH2)ₙ and n = 0, 1, 2 or 3; and
   wherein Y is (CH₂)ₙ, (OCH₂CH₂)ₙ or CO(CH₂)ₙ and n is a digit from 0 to 3; and
   wherein R is (CH₂)ₙCH₃, (OCH₂CH₂)ₙOH, CO(CH₂)ₙCOOH, (OCH₂CH₂)ₙOCH₂COOH and n is a digit from 0 to 3;
   and wherein the curly sign indicates the link to the aromatic ring;
   R₂ is -H- or a polar group (PG) selected from: 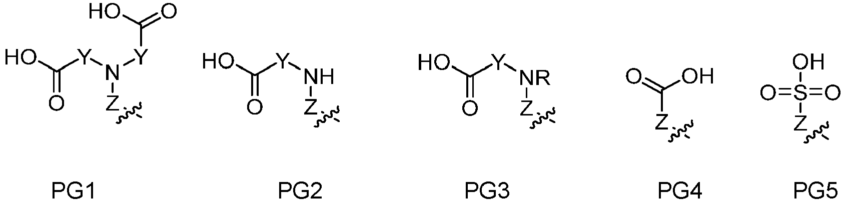
   wherein Z is CO(CH₂)ₙ, SO₂(CH₂)ₙ, (CH₂)ₙ or (OCH₂CH₂)ₙ and n = 0, 1, 2 or 3; and
   wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
   R₃ is -H or 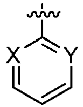
   wherein X and Y are independently selected from: -CH₂- and -N-;
      and
      wherein the tetrazine compound of formula I has a lipophilicity of cLogD7.4 < - 0.5, and have complementary inverse electron demand Diels-Alder cycloaddition reactivity to the dienophile.

In a fifth aspect, the present invention relates to a precursor of formula II. The precursor of formula II is a precursor that can be used to provide compounds of formula I. The precursors of formula II have the following structure: 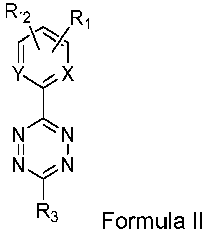 wherein R₁ is 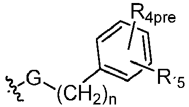
wherein G is selected from -O-, -S-, -NH-, and -N-alkyl-;
n is a digit from 0 to 4;
R₄ₚᵣₑ is a group selected from T₃Sn, T₃Si or T₃Ge, wherein T is a; straight chain or branched alkyl group, independently selected from methyl, ethyl, propyl, butyl, pentyl, hexyl, heptyl, octyl, nonyl, and decyl,
R'₅ is -H- or a polar group (PG) selected from: 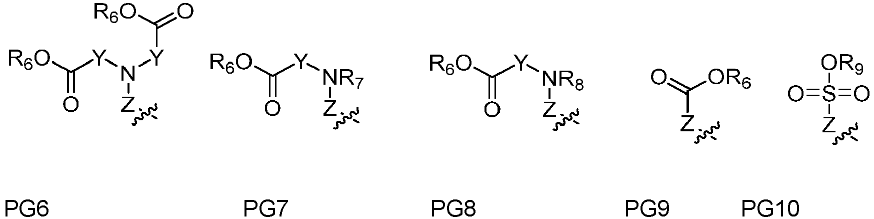
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, (OCH₂CH₂)ₙOCH₂COOC(CH₃)₃, and n is a digit from 0 to 3;
and wherein R₆ is selected from -H, tert-butyl, and trityl;
R₇ is -H or Boc;
R₉ is selected from -H, neopentyl, and α-trifluoromethylbenzyl;
and wherein the curly sign indicates the link to the aromatic ring,
R'₂ is -H- or a polar group (PG) selected from: 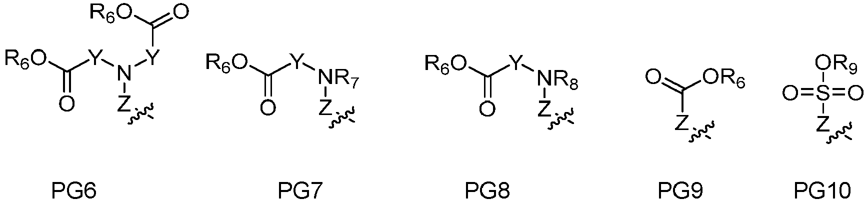
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH2)ₙ or CO(CH₂)ₙ and n is a digit from 0 to 3; and
wherein R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, (OCH₂CH₂)ₙOCH₂COOC(CH₃)₃, and n is a digit from 0 to 3;
and wherein R₆ is selected from -H, tert-butyl, and trityl;
R₇ is -H or Boc;
R₉ is selected from -H, neopentyl, and α-trifluoromethylbenzyl;
and wherein the curly sign indicates the link to the aromatic ring,
R₃ is -H or 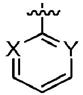
wherein X and Y are independently selected from: -CH₂- and -N-.

In a sixth aspect, the present invention relates to a kit comprising either a compound of formula I or a compound of formula II, and a dienophile having complementary inverse electron demand Diels-Alder cycloaddition reactivity to the compound of formula I or to the compound of formula II, respectively.

### BRIEF DESCRIPTION OF DRAWINGS

Figure 1: Schematic representation of blocking experiment
Figure 2: Table comprising the structural scaffolds, calculated physicochemical properties (clogD7.4), rate constants (M⁻¹ s⁻¹), and blocking efficiencies of tetrazine derivatives.
Figure 3: **A** reaction sequence of the radiolabeling of [¹⁸F]**27**; **B** analytical chromatograms of [¹⁸F]**27** and the reference **27**; **C** analytical chromatogram of [¹⁸F]**27** after TCO-PNB addition.
Figure 4: Astatination of [²¹¹At]**28** with silyl precursor **47**.
Figure 5: Semipreparative HPLC (A) and analytical radio-HPLC (B) of [¹³¹I]**28** .
Figure 6: **A** graph of PET-derived biodistribution of [¹⁸F]**27** as %ID/g for tumor uptake at 1, 2 and 3h P.I.; **B** graph of PET-derived biodistribution of [¹⁸F]**27** in tumor, heart (blood surrogate) and muscle tissue %ID/g 3h P.I.; **C** scatter diagram of PET-derived biodistribution of [¹⁸F]**27** in various tissues as %ID/g.
Figure 7: Diagram showing biodistribution of [²¹¹At]28 in various organs and tissues expressed as %ID/g.

### DEFINITIONS

**Lipophilicity cLogD7.4** is a measure of a compound's lipophilicity at a specific pH of 7.4, which is the physiological pH of human blood plasma. Lipophilicity refers to the affinity of a compound for lipid environments, which influences its ability to dissolve in fats, oils, and non-polar solvents. The term "cLogD" represents the calculated logarithm of the distribution coefficient (D) of a compound between a lipid (oil) phase and an aqueous (water) phase.

The distribution coefficient (D) takes into account both the neutral and ionized forms of the compound in the given pH environment. At pH 7.4, this measure is particularly relevant for understanding how a drug or chemical compound behaves, as it indicates how the compound partitions between hydrophilic and lipophilic environments. This property is critical for absorption, distribution, metabolism, excretion, and overall bioavailability. The lipophilicity cLogD7.4 value of a compound can be determined by experimental methods for example by chromatographic methods and/or using computational methods known in the art.

**Blocking efficiency** is - in the assay used herein - a measure of the ability of an unlabeled tetrazine to react with a dienophile targeting vector in vivo. If the tetrazine reacts well with the pretargeting vector, subsequent reaction between the pretargeting vector and a subsequently administered radiolabeled tetrazine known to react well with the pretargeting vector will be blocked. If the unlabeled tetrazine does not react well with the pretargeting vector, the radiolabeled tetrazine will react with the pretargeting vector and no blocking will be observed. In the present assay a dienophile with inverse electron demand Diels-Alder cycloaddition reactivity being conjugated to a pretargeting vector, CC49-TCO, is injected into a mouse comprising the tumor target. After 72 hours, the unlabeled tetrazine to be tested is administered to the same mouse. After 22 hours, a ¹¹¹In labelled DOTA-tetrazine known to react well with CC49-TCO is administered to the same mouse. The tumor blocking effect of the unlabeled tetrazine derivatives is then determined by ex vivo biodistribution and normalized to the binding of [¹¹¹In]DOTA-Tz without any blocking.

### DETAILED DESCRIPTION OF THE INVENTION

The present invention provides radiolabeled tetrazines wherein the radionuclide is linked to the tetrazine via an aromatic linker. It was surprisingly found that such compounds show improved properties over previous radiolabeled tetrazines. The tetrazines disclosed were found to be labelable with radionuclides selected from ¹⁸F, ¹⁹F, ⁷⁶Br, ⁷⁷Br ¹²⁵I, ¹²³I, ¹³¹I, ¹²⁴I, ¹²⁷I, or ²¹¹At in an amount that allows for the efficient use in pretargeting approaches within theranostics, therapy, diagnostics and imaging. It is shown herein that when the radionuclide is linked to the tetrazine via an aromatic linker, a surprisingly improved tumor uptake and increased excretion was observed.

The new class of tetrazines provided here resulted from a desire of providing compounds suitable for labeling not only with fluorine but also with three other important radionuclides, bromine, iodine and astatine, respectively. Such compounds would be applicable in theranostics because the same structure could be applied as an imaging agent / diagnostic agent and as a therapeutic agent depending on which radionuclide the compound would be labeled with. Compounds [¹⁸F]**1** and [¹⁸F]**2** and were chosen as control compounds because these compounds showed good tumor-to-background ratios in previous in vivo experiments.

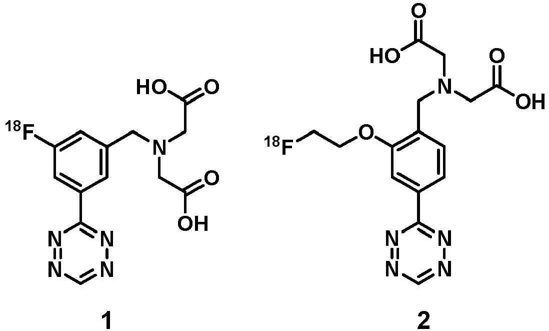

However, we found that when these compounds were labeled with Iodine, and tested in blocking assays, they did not block as well as the ¹⁸F-labeled compounds. Labeling the structures with astatine would possibly block even less efficiently than the iodine compounds because Astatine-labeled compounds are more unstable in vivo than Iodine-labeled compounds.

As shown herein, a tetrazine structure wherein the radionuclide is linked to the tetrazine via an aromatic linker surprisingly proved to be suitable for the desired theranostic purposes, because such structures provided sufficient rate constant values, good blocking effects and sufficient radiochemical yield when labeled with ¹⁸F, 131I and ²¹¹astatine, respectively. Further, as also shown herein, such structure provided surprisingly improved tumor uptake and increased excretion when tested in *ex vivo* PET-imaging experiments.

In a first aspect, the compound provided herein is a tetrazine compound having the structure of formula I: 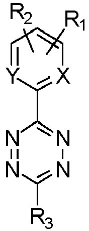 wherein R₁ is 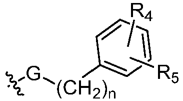
wherein G is selected from -O-, -S-, -NH-, and -N-alkyl-;
n is a digit from 0 to 4;
R₄ is a radioisotope selected from ¹⁸F, ¹⁹F, ⁷⁶Br, ⁷⁷Br, ¹²⁵I, ¹²³I, ¹³¹I, ¹²⁴I, ¹²⁷I and ²¹¹At;
R₅ is -H- or a polar group (PG) selected from: 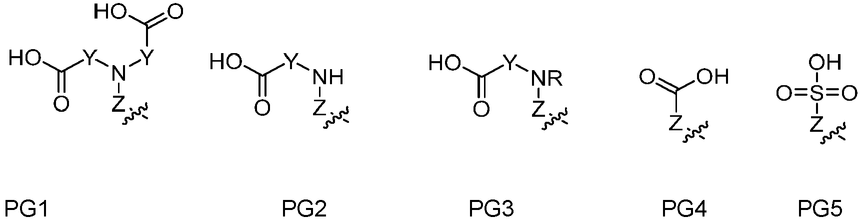
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 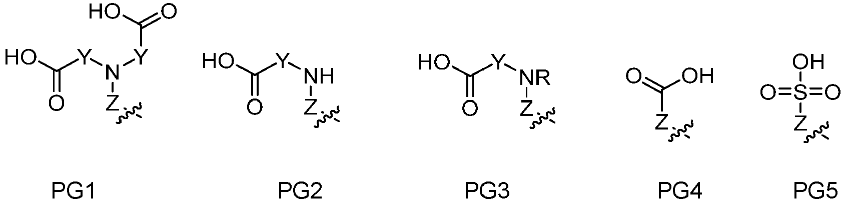
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 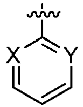
wherein X and Y are independently selected from: -CH₂- and -N-;
   and
wherein the tetrazine compound of formula I has a lipophilicity of cLogD7.4 < -0.5.

The tetrazine compounds according Formula I comprises at least one group that provides the compounds according to Formula I with a lipophilicity of clogD_{7.4} < -0.5. When the tetrazine compounds of Formula I have a sufficiently high polarity, they will reach pretargeting vectors which do no internalize. This is beneficial for their use as imaging agents, as diagnostic agents and as therapeutic agents in relation to certain diseased tissue in animals and humans, such as cancer tissue, cancerous cells, infected cells or pathogens.

The lipophilicity of compounds of formula I suited for such purposes have a lipophilicity clogD_{7.4} of approximately less than -0.5. It has previously been found for other tetrazine structures that their blocking effect increased the lower the lipophilicity value. Thus, the compounds of Formula I preferably have a lipophilicity clogD_{7.4} of less than -2. Even more preferred, the compounds of Formula I preferably have a lipophilicity clogD_{7.4} of less than less than -3 and most preferred, the compounds of Formula I have a lipophilicity clogD_{7.4} of less than -6. A lipophilicity of is clogD_{7.4} of approximately less than -0.5 needed to obtain reasonable tumor accumulation (normalized blocking effect > 70), in standardly applied tumor models for pretargeted strategies.

The present compound of formula I can be used for imaging when labelled with either ¹⁸F, ⁷⁶Br, ¹²³I, ¹²⁴I or ¹³¹I and for therapy when labelled with either ⁷⁷Br, ¹²⁵I, ¹³¹I, or ²¹¹At. When the compound of formula I is labelled with either ¹⁹F or ¹²⁷I, the compound is suitable for use in laboratory experiments. Since the structure of formula I can be used both in imaging which is often for diagnostic purposes and in therapy depending on the specific radionuclide selected as the R₄-entity in a compound of formula I, compounds of formula I are suitable for theranostic purposes where the same compound except from the specific radionuclide can be administered to the subject. Thus, the compound of formula I can be used in theranostic approaches when applied as pairs in diagnostic and therapy by selecting between the radionuclides applicable for a specific purpose.

The application of compounds of formula I is based on their inverse electron demand Diels-Alder cycloaddition reactivity. Compounds of formula I effectively reacts with dienophiles having complementary inverse electron demand Diels-Alder cycloaddition reactivity, a reaction commonly referred to as a click-reaction. Accordingly, compounds of formula I are very suitable for use in pre-targeting approaches. Such approaches would comprise firstly, administering to a subject a dienophile with inverse electron demand Diels-Alder cycloaddition reactivity being conjugated to targeting vector, such as a human. After binding to the target, and clearing or excretion of any excess dienophile-targeting conjugate, a compound of formula I having complementary inverse electron demand Diels-Alder cycloaddition reactivity would then be administered to the same subject. The dienophile-targeting conjugate and the compound of formula I would bind to each other via the click-reaction. Unbound compound of formula I would readily be excreted from the subject.

Accordingly, in a second aspect, the Compound of formula I 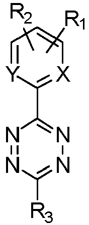 wherein R₁ is 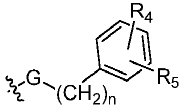
wherein G is selected from -O-, -S-, -NH-, and -N-alkyl-;
n is a digit from 0 to 4;
R₄ is a radioisotope selected from ¹⁸F, ⁷⁶Br, ⁷⁷Br, ¹²⁵I, ¹²³I, ¹³¹I, ¹²⁴I, and ²¹¹At;
R₅ is -H- or a polar group (PG) selected from: 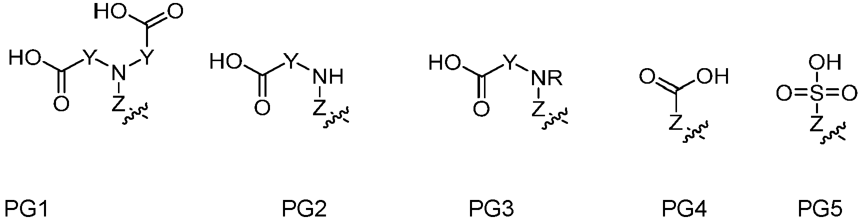
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH2)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 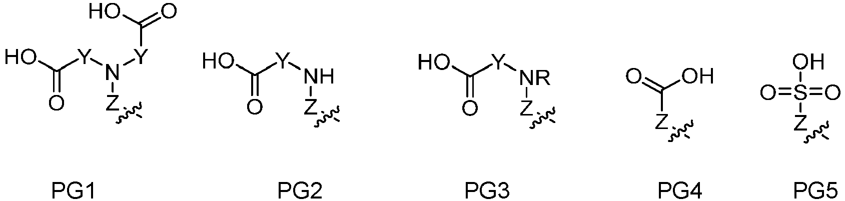
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH2)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 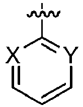
wherein X and Y are independently selected from: -CH₂- and -N-;
   and
   wherein the tetrazine compound of formula I has a lipophilicity of cLogD7.4 < - 0.5
   for use as a medicament in pretargeting based therapy, pretargeting based diagnostic, pretargeting based theranostic or pretargeting based imaging is provided.

When the radionuclide R₄ in formula I is selected from ⁷⁷Br, ¹²⁵I, ¹³¹I, ²¹¹At, the compound of formula I can be used in therapy. The radionuclides have different applicability in therapy that are well known in the art. Depending on the antigen targeted by the dienophile-targeting vector conjugate that the compound of formula I will click with, the radionuclide will come into close contact with the desired target. The target can thus be any target characteristic of a certain disease, cell type or tissue that a dienophile-targeting vector can be targeted at, such as proteins, including cell surface receptors, antigens such as specific antigens on cancer cells, enzymes, such as enzymes that are upregulated in certain diseases, cell membrane transporters, DNA, RNA and cell adhesion molecules. The target could also be cells with specific metabolic characteristics involved in metabolic pathways known to be relevant in certain diseases.

The diseases that can be targeted by existing targeting vectors and therefore also possible targets in imaging, diagnostic, treatment or theranostic approaches based on compounds of formula I includes cancer, such as prostate cancer, neuroendocrine tumors, Non-Hodgkin's Lymphoma, colorectal cancer, liver cancer, brain tumors, lung cancer, thyroid cancer, pancreatic cancer and melanoma; cardiovascular diseases such as atherosclerosis and assessment of general functions such as myocardial perfusion imaging; Infectious diseases such as tuberculosis and other bacterial or fungal infections; neurological disorders such as Alzheimer's Disease and Parkinson's Disease; inflammatory diseases such as Rheumatoid Arthritis and inflammatory Bowel Disease; bone disorders such as osteomyelitis and bone metastases; thyroid diseases such as Grave's disease; Renal diseases such as pyelonephritis and assessment of renal function in general; lymphatic disorders such as lymphedema.

Accordingly, the invention relates in a second aspect to a compound of formula I 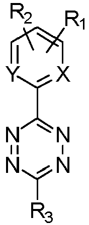 wherein R₁ is 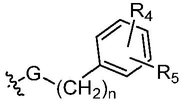
wherein G is selected from -O-, -S-, -NH-, and -N-alkyl-;
n is a digit from 0 to 4;
R₄ is a radioisotope selected from ¹⁸F, ⁷⁶Br, ⁷⁷Br, ¹²⁵I, ¹²³I, ¹³¹I, ¹²⁴I, and ²¹¹At;
R₅ is -H- or a polar group (PG) selected from: 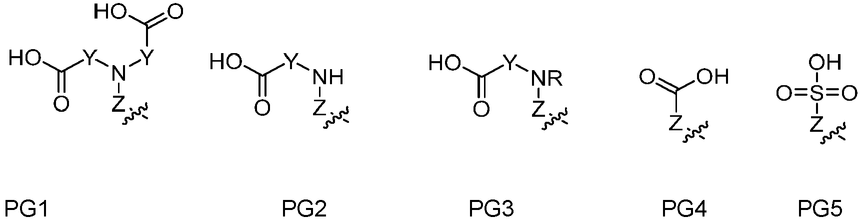
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 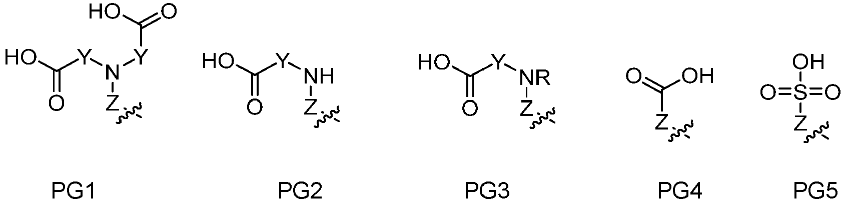
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 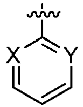
wherein X and Y are independently selected from: -CH₂- and -N-;
   and
   wherein the tetrazine compound of formula I has a lipophilicity of cLogD7.4 < - 0.5
   for use in a method of diagnosing and/or treating cancer diseases, cardiovascular diseases, infectious diseases, neurological disorders, inflammatory diseases, bone disorders, thyroid diseases, renal diseases, or lymphatic disorders.

Since compounds of formula I needs to click with a dienophile-targeting vector conjugate in order to provide its imaging, diagnostic, or therapeutic effect at the target site, use of compounds of formula I in such methods would require, that the dienophile-targeting vector conjugate is already situated at the target once compounds of formula I is administered to a subject, i.e. a pre-targeting approach.
In a preferred embodiment, the compound of formula I 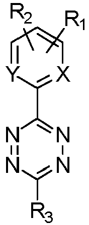 wherein R₁ is 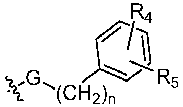
wherein G is selected from -O-, -S-, -NH-, and -N-alkyl-;
n is a digit from 0 to 4;
R₄ is a radioisotope selected from ¹⁸F, ⁷⁶Br, ⁷⁷Br, ¹²⁵I, ¹²³I, ¹³¹I, ¹²⁴I, and ²¹¹At;
R₅ is -H- or a polar group (PG) selected from: 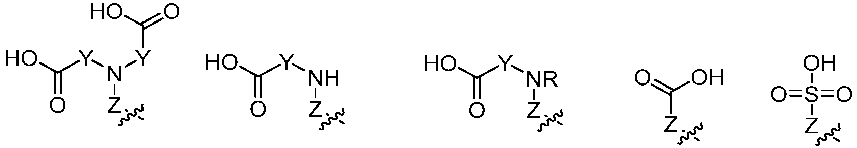 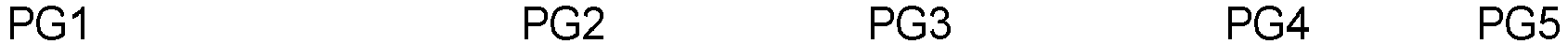
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 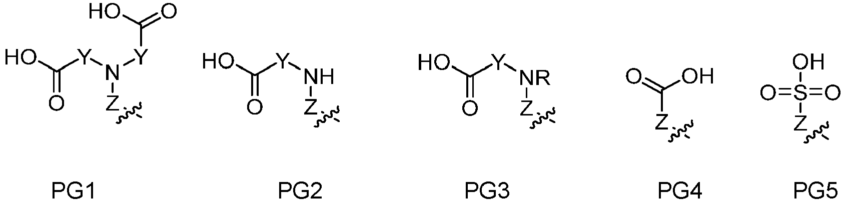
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 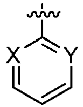
wherein X and Y are independently selected from: -CH₂- and -N-;

   and
   wherein the tetrazine compound of formula I has a lipophilicity of cLogD7.4 < - 0.5,
   is for use in a method wherein said method comprises:
   a) administering to a subject a dienophile with inverse electron demand Diels-Alder cycloaddition reactivity being conjugated to a targeting vector targeting specific antigens in the subject,
   b) Allowing the targeting vector time to bind to the target, and for unbound targeting vectors to clear from the bloodstream
   c) Administering to the same subject a compound of formula I having complementary inverse electron demand Diels-Alder cycloaddition reactivity to the dienophile.

For diagnostic purposes, the radionuclide R₄ in formula I must be identified by imaging techniques, such as PET and SPECT. Accordingly, when compounds of formula I are for use in a diagnostic method or in imaging, the radionuclide must be selected from ¹⁸F, ¹²³I, ¹³¹I, ¹²⁴I.

Thus, in a preferred embodiment, the compound of formula I is a compound wherein the radioisotope R₄ I is selected from ¹⁸F, ⁷⁶Br, ¹²³I, ¹³¹I, ¹²⁴I, and wherein the method is diagnostic and further comprises subjecting the subject to imaging, such as PET-scanning or SPECT-scanning.

For PET-scanning the radioisotope R₄ in the compound of formula I must be selected from ¹⁸F, ⁷⁶Br, or ¹²⁴I .

For SPECT-scanning the radioisotope R₄ in formula I must be selected from ¹²³I or ¹³¹I.

For therapeutic purposes, the radionuclide R₄ in formula I must be able to deliver therapeutic radiation doses to target tissues. ⁷⁷Br emits beta particles, which are useful for therapeutic applications. ⁷⁷Br has a half-life of about 57 hours, which allows for a practical timeframe for both synthesis and administration in therapeutic settings. ¹²⁵I emits low-energy gamma rays and has a relatively long half-life, which is suitable for localized, prolonged radiation delivery. It is commonly used in brachytherapy, particularly for treating prostate cancer and certain types of brain tumors. 131I emits both beta particles and gamma rays and is widely used in the treatment of thyroid disorders, including hyperthyroidism and thyroid cancer. ²¹¹At emits alpha particles, which have high linear energy transfer and are effective in killing for example cancer cells while minimizing damage to surrounding healthy tissue.

Thus, in a preferred embodiment, the compound of formula I is a compound wherein the radioisotope R₄ is selected from ⁷⁷Br, ¹²⁵I ¹³¹I, ²¹¹A and wherein the method is therapeutic.

In some embodiments, the compound of formula I could be for use in a method wherein the compound of formula I is administered twice to the same subject. This is relevant for theranostic purposes, wherein first, the compound of formula I comprises a radioisotope R₄ that is suitable for imaging / diagnostic purposes is administered and wherein afterwards, the compound of formula I comprising a radioisotope R₄ that is suitable for therapy is administered. In this way, the compound of formula I is applied as a theranostic pair where the only structural difference is the identity of the radioisotope. This approach ensures that the therapeutic agent precisely targets the disease site identified by the diagnostic agent.

Therefore, administering a compound of formula I wherein R₄ is selected from ¹⁸F, ⁷⁶Br, ¹²³I, ¹³¹I, ¹²⁴I, followed by administering the same compound of formula I wherein wherein R₄ is selected from ⁷⁷Br, ¹²⁵I ¹³¹I, ²¹¹A is suitable for theranostic purposes where the same compound except from the specific radionuclide can be administered to the subject. Thus, the compound of formula I can be used in theranostic approaches when applied as pairs in diagnostic and therapy by selecting between the radionuclides applicable for a specific purpose.

The benefit of using theranostic pairs includes improved personalized treatment in that the diagnostic scan confirms the presence and extent of the disease, ensuring that the therapeutic agent is administered to patients who will most likely benefit from it. It also improves efficacy and safety by targeting the therapeutic agent precisely to the disease site identified by the diagnostic agent, the theranostic approach enhances treatment efficacy and minimizes damage to healthy tissues. Moreover, theranostic pairs is a mean for monitoring response because the diagnostic component can be used to monitor the response to therapy, allowing adjustments to be made to the treatment regimen if necessary.

In a preferred embodiment, the compound of formula I 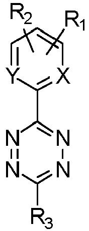 wherein R₁ is 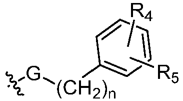
wherein G is selected from -O-, -S-, -NH-, and -N-alkyl-;
n is a digit from 0 to 4;
R₄ is a radioisotope selected from ¹⁸F, ⁷⁶Br, ⁷⁷Br, ¹²⁵I, ¹²³I, ¹³¹I, ¹²⁴I, and ²¹¹At;
R₅ is -H- or a polar group (PG) selected from: 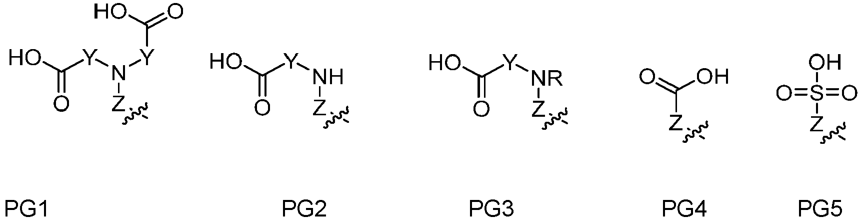
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 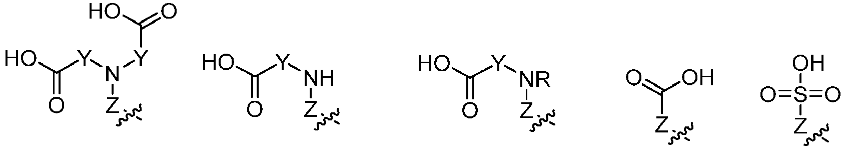 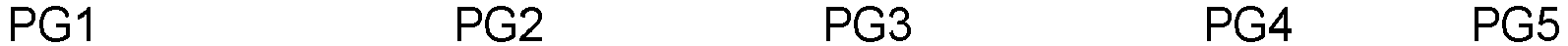
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 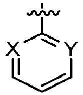
wherein X and Y are independently selected from: -CH₂- and -N-;
   and
   wherein the tetrazine compound of formula I has a lipophilicity of cLogD7.4 < - 0.5
   is for use in a method comprising:
   a) administering to a subject a dienophile with inverse electron demand Diels-Alder cycloaddition reactivity being conjugated to a targeting vector targeting specific antigens in the subject,
   b) Allowing the targeting vector time to bind to the target, and for unbound targeting vectors to clear from the bloodstream
   c) Administering to the same subject as in step a) a compound of formula I having complementary inverse electron demand Diels-Alder cycloaddition reactivity to the dienophile, wherein R₄ in formula I is a radioisotope selected from ¹⁸F, ¹²³I, ¹³¹I, ¹²⁴I, and
   d) Optionally repeating step a) and step b)
   e) Administering to the same subject as in step c) a compound of formula I and having complementary inverse electron demand Diels-Alder cycloaddition reactivity to the dienophile, and wherein R₄ in formula I is a radioisotope selected from ¹²⁵I, ¹³¹I, and ²¹¹At.

In another preferred embodiment, the compound of formula I is for use in a method comprising:
a) Administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream,
c) Administering to the same subject as in step a) a compound of formula I having complementary inverse electron demand Diels-Alder cycloaddition reactivity to the dienophile, wherein R₄ in formula I is a radioisotope selected from ¹⁸F, ⁷⁶Br, ¹²³I, ¹³¹I, ¹²⁴I, and subjecting the subject to PET or SPECT,
d) Optionally repeating step a) and step b),
e) Administering to the same subject as in step c) a compound of formula I and having complementary inverse electron demand Diels-Alder cycloaddition reactivity to the dienophile, and wherein R₄ in formula I is a radioisotope selected from ⁷⁷Br ¹²⁵I, ¹³¹I, and ²¹¹At,
f) Administering to the same subject as in step e) a dienophile with inverse electron demand Diels-Alder cycloaddition reactivity being conjugated to a targeting vector targeting specific antigens in the subject,
g) Allowing the targeting vector time to bind to the target, and for unbound targeting vectors to clear from the bloodstream,
h) Administering to the same subject as in step f) a compound of formula I having complementary inverse electron demand Diels-Alder cycloaddition reactivity to the dienophile, wherein R₄ in formula I is a radioisotope selected from ¹⁸F, ¹²³I, ¹³¹I, ¹²⁴I, and subjecting the subject to PET or SPECT,
i) Optionally, repeating step e) or steps e) to h).

The targeting vector can be any kind of targeting vector that is suitable for use in therapy, imaging, or diagnostics. Such commonly used targeting vectors that are suitable in the diagnostic, imaging, therapeutic, and theranostic methods mentioned herein and in the present kits include antibodies, nanobodies, polymers, nanomedicines, cells, proteins, peptides, and small molecules.

Commonly applied targeting vectors, that are suitable in the present methods and kits includes: peptides such as Octreotide, Octreotate, AE105; small molecules such as FAPI derivatives and PSMA derivatives.

The subject mentioned in the methods herein could be any living subject, such as a mammal. In a preferred embodiment, the mammal is an individual such as a human. In another aspect, the present invention provides a pre-targeting method wherein compounds of formula I is used for imaging, in therapy, in diagnosis or in theranostic.

Thus, the present invention provides a method comprising:
a) administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream
c) Administering to the same subject a compound of formula I: 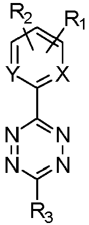 wherein R₁ is 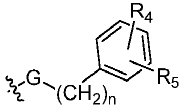
   wherein G is selected from -O-, -S-, -NH-, and -N-alkyl-;
   n is a digit from 0 to 4;
   R₄ is a radioisotope selected from ¹⁸F, ⁷⁶Br, ⁷⁷Br, ¹²⁵I, ¹²³I, ¹³¹I, ¹²⁴I, and ²¹¹At;
   R₅ is -H- or a polar group (PG) selected from: 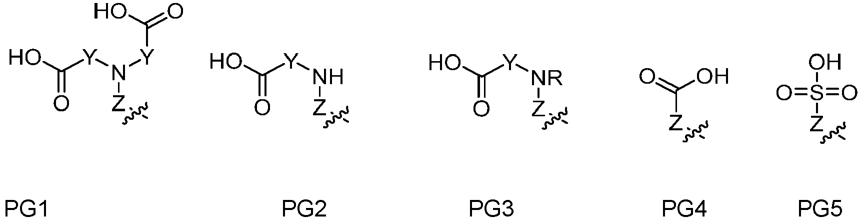
   wherein Z is CO(CH₂)ₙ, SO₂(CH₂)ₙ, (CH₂)ₙ or (OCH₂CH2)ₙ and n = 0, 1, 2 or 3; and
   wherein Y is (CH₂)ₙ, (OCH₂CH₂)ₙ or CO(CH₂)ₙ and n is a digit from 0 to 3; and
   wherein R is (CH₂)ₙCH₃, (OCH₂CH₂)ₙOH, CO(CH₂)ₙCOOH, (OCH₂CH₂)ₙOCH₂COOH and n is a digit from 0 to 3;
   and wherein the curly sign indicates the link to the aromatic ring;
   R₂ is -H- or a polar group (PG) selected from: 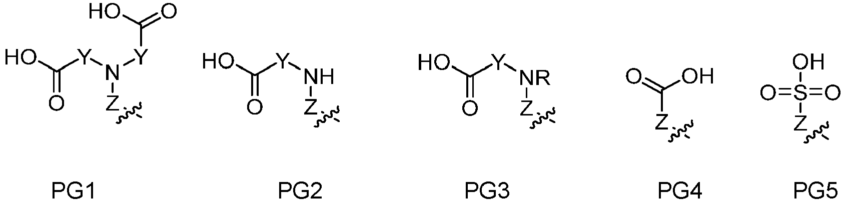
   wherein Z is CO(CH₂)ₙ, SO₂(CH₂)ₙ, (CH₂)ₙ or (OCH₂CH₂)ₙ and n = 0, 1, 2 or 3; and
   wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
   R₃ is -H or 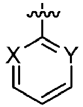
   wherein X and Y are independently selected from: -CH₂- and -N-;
      and
      wherein the tetrazine compound of formula I has a lipophilicity of cLogD7.4 < - 0.5
      having complementary inverse electron demand Diels-Alder cycloaddition reactivity to the dienophile.

In a preferred embodiment, a method is provided wherein R₄ in the compound of formula I is a radioisotope selected from ¹⁸F, ⁷⁶Br, ¹²³I, ¹³¹I, ¹²⁴I, and wherein the method is diagnostic further comprising subjecting the subject to PET-scanning when the radioisotope in formula I is ¹⁸F, ⁷⁶Br, or ¹²⁴I, or subjecting the subject to SPECT-scanning when the radioisotope in formula I is ¹²³I or ¹³¹I.

In another preferred embodiment, a method is provided wherein R₄ in the compound of formula I is a radioisotope selected from ¹⁸F, ⁷⁶Br, ¹²³I, ¹³¹I, ¹²⁴I, and wherein the method is a pre-targeting theranostic method comprising:
a) administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream
c) Administering to the same subject a compound of formula I: 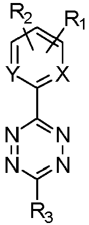 wherein R₁ is 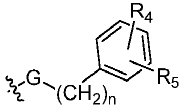
   wherein G is selected from -O-, -S-, -NH-, and -N-alkyl-;
   n is a digit from 0 to 4;
   R₄ is a radioisotope selected from ¹⁸F, ⁷⁶Br, ⁷⁷Br, ¹²⁵I, ¹²³I, ¹³¹I, ¹²⁴I, and ²¹¹At;
   R₅ is -H- or a polar group (PG) selected from: 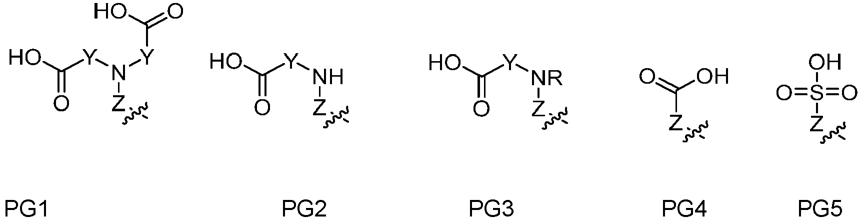
   wherein Z is CO(CH₂)ₙ, SO₂(CH₂)ₙ, (CH₂)ₙ or (OCH₂CH2)ₙ and n = 0, 1, 2 or 3; and
   wherein Y is (CH₂)ₙ, (OCH₂CH₂)ₙ or CO(CH₂)ₙ and n is a digit from 0 to 3; and
   wherein R is (CH₂)ₙCH₃, (OCH₂CH₂)ₙOH, CO(CH₂)ₙCOOH, (OCH₂CH₂)ₙOCH₂COOH and n is a digit from 0 to 3;
   and wherein the curly sign indicates the link to the aromatic ring;
   R₂ is -H- or a polar group (PG) selected from: 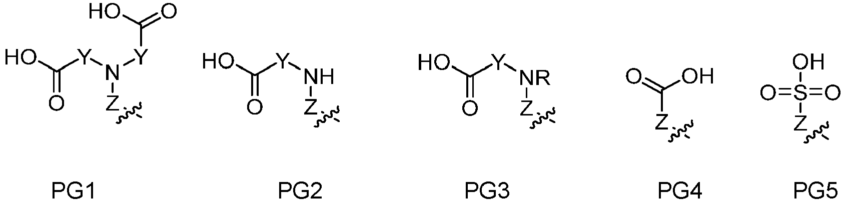
   wherein Z is CO(CH₂)ₙ, SO₂(CH₂)ₙ, (CH₂)ₙ or (OCH₂CH₂)ₙ and n = 0, 1, 2 or 3; and
   wherein Y is (CH₂)ₙ, (OCH₂CH2)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
   R₃ is -H or 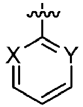
   wherein X and Y are independently selected from: -CH₂- and -N-;
      and
      wherein the tetrazine compound of formula I has a lipophilicity of cLogD7.4 < - 0.5
      having complementary inverse electron demand Diels-Alder cycloaddition reactivity to the dienophile.
d) Optionally repeating step a) and step b)
e) Administering to the same subject a compound of formula I and having complementary inverse electron demand Diels-Alder cycloaddition reactivity to the dienophile, and wherein R₄ in formula I is a radioisotope selected from ⁷⁷Br¹²⁵I, ¹³¹I, and ²¹¹At.

In another preferred embodiment, the method includes diagnostic/imaging steps followed by one or more therapy steps allowing for the monitoration of progression the therapy and possible adjustment of the treatment comprising:
a) Administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream,
c) Administering to the same subject as in step a) a compound of formula I having complementary inverse electron demand Diels-Alder cycloaddition reactivity to the dienophile, wherein R₄ in formula I is a radioisotope selected from ¹⁸F, ⁷⁶Br, ¹²³I, ¹³¹I, ¹²⁴I, and subjecting the subject to PET or SPECT,
d) Optionally repeating step a) and step b),
e) Administering to the same subject as in step c) a compound of formula I and having complementary inverse electron demand Diels-Alder cycloaddition reactivity to the dienophile, and wherein R₄ in formula I is a radioisotope selected from ⁷⁷Br¹²⁵I, ¹³¹I, and ²¹¹At,
f) Administering to the same subject as in step e) a dienophile with inverse electron demand Diels-Alder cycloaddition reactivity being conjugated to a targeting vector targeting specific antigens in the subject,
g) Allowing the targeting vector time to bind to the target, and for unbound targeting vectors to clear from the bloodstream,
h) Administering to the same subject as in step f) a compound of formula I having complementary inverse electron demand Diels-Alder cycloaddition reactivity to the dienophile, wherein R₄ in formula I is a radioisotope selected from ¹⁸F, ⁷⁶Br, ¹²³I, ¹³¹I, ¹²⁴I, and subjecting the subject to PET or SPECT,
i) Optionally, repeating step e) or steps e) to h).

In another aspect, the present invention relates to precursors of the tetrazine compounds of formula I. These precursors have the structure of formula II: 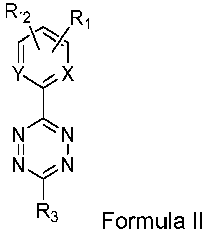 wherein R₁ is 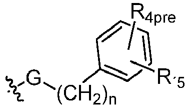
wherein G is selected from -O-, -S-, -NH-, and -N-alkyl-;
n is a digit from 0 to 4;
R₄ₚᵣₑ is a group selected from T₃Sn, T₃Si or T₃Ge, wherein T is a; straight chain or branched alkyl group, independently selected from methyl, ethyl, propyl, butyl, pentyl, hexyl, heptyl, octyl, nonyl, and decyl,
R'₅ is -H- or a polar group (PG) selected from: 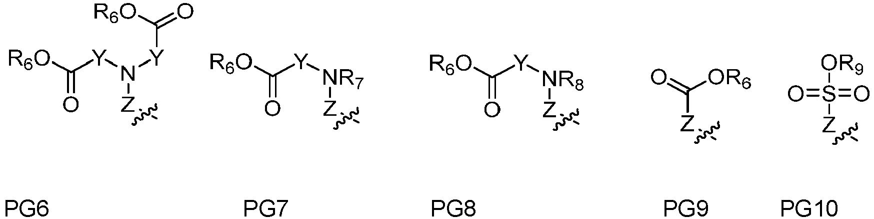
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or (OCH₂CH₂)ₙOCH₂COOC(CH₃)₃, wherein n is a digit from 0 to 3;
R₉ is selected from -H, neopentyl, and α-trifluoromethylbenzyl;
and wherein the curly sign indicates the link to the aromatic ring,
R'₂ is -H- or a polar group (PG) selected from: 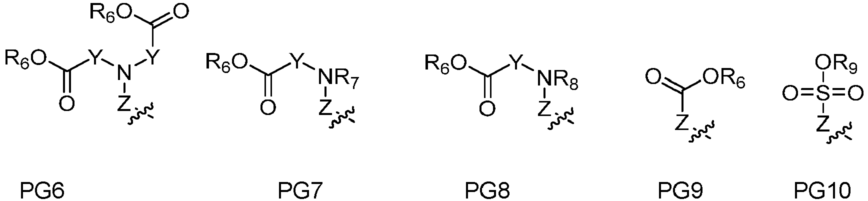
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring,
and wherein R₆ is selected from -H; tert-butyl, and trityl;
R₇ is -H, or Boc,
R₈ is selected from -H, neopentyl, and α-trifluoromethylbenzyl
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or
(OCH₂CH₂)ₙOCH₂COOC(CH₃)₃, wherein n is a digit from 0 to 3;
R₉ is selected from -H, neopentyl, and α-trifluoromethylbenzyl;
R₃ is -H or 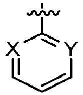
wherein X and Y are independently selected from: -CH₂- and -N-, and wherein the curly sign indicates the link to the aromatic ring.

The precursors of formula II are suitable for providing the tetrazines of formula I by subjecting the precursors of formula II to labeling with a radioactive isotope selected from ¹⁸F, ⁷⁶Br, ⁷⁷Br, ¹²⁵I, ¹²³I, ¹³¹I, ¹²⁴I, and ²¹¹At at position R₄. Thus, the R₄ group of formula I is named R₄ₚᵣₑ in the compounds of formula II. The difference between R₄ in formula I and R₄ₚᵣₑ in formula II is that R₄ₚᵣₑ comprises a stannyl, silicium, or germanyl leaving group which is replaced by the radioactive isotope in order to provide the R₄ group in compounds of formula I.

Such precursor compounds for formula II are easier to handle and more suitable for storage than the labelled versions of the same compound of formula I since they are non-radioactive.

Therefore, compounds of formula II wherein R₄ₚᵣₑ comprising is a group selected from T₃Sn, T₃Si or T₃Ge, wherein T is a; straight chain or branched alkyl group, independently selected from methyl, ethyl, propyl, butyl, pentyl, hexyl, heptyl, octyl, nonyl, and decyl, is beneficial in for example use in a kit where the labelling with a radionuclide replacing the leaving group can be prepared shortly before administering the compound to a subject such as a patient.

Compounds of formula II may comprise one or more protecting groups on position R'₂ and/or R'₅, denoted R₆, R₇, R₈ and R₉, respectively in formula II. The difference between R₂ and R₅ in structures of formula I and R'₂ and R'₅ in structures of formula II is thus the optional presence of one or more protecting groups on R'₂ and/or R'₅.

R₆ in R'₂ and/or R'₅ when R'₂ and/or R'₅ comprises any of the polar groups PG6, PG7, PG8 or PG9 is selected from -H, and the protecting groups tert-butyl, and trityl. R₇ in R'₂ and/or R'₅ when R'₂ and/or R'₅ comprises the polar group PG7 is either -H or the protecting group Boc.

R₈ in R'₂ and/or R'₅ when R'₂ and/or R'₅ comprises PG8 is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or (OCH₂CH₂)ₙOCH₂COOC(CH₃)₃, wherein n is a digit from 0 to 3;
R₉ in R'₂ and/or R'₅ when R'₂ and/or R'₅ comprises the polar group PG10 is selected from -H, and the protecting groups neopentyl, and α-trifluoromethylbenzyl.

In another aspect, the present invention relates to a kit comprising either a tetrazine compound of formula I or a precursor compound of formula II, and a dienophile having complementary inverse electron demand Diels-Alder cycloaddition reactivity to the compound of formula I or of formula II, respectively.

Thus, in this aspect the invention relates to a kit comprising a tetrazine compound according to formula I: 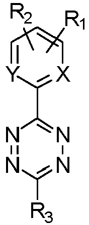 wherein R₁ is 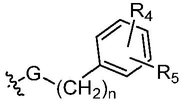
wherein G is selected from -O-, -S-, -NH-, and -N-alkyl-;
n is a digit from 0 to 4;
R₄ is a radioisotope selected from ¹⁸F, ¹⁹F, ⁷⁶Br, ⁷⁷Br, ¹²⁵I, ¹²³I, ¹³¹I, ¹²⁴I, ¹²⁷I and ²¹¹At;
R₅ is -H- or a polar group (PG) selected from: 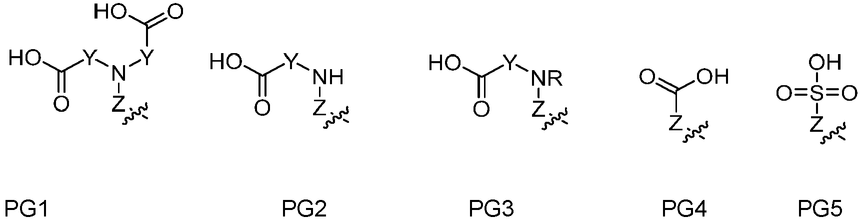
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 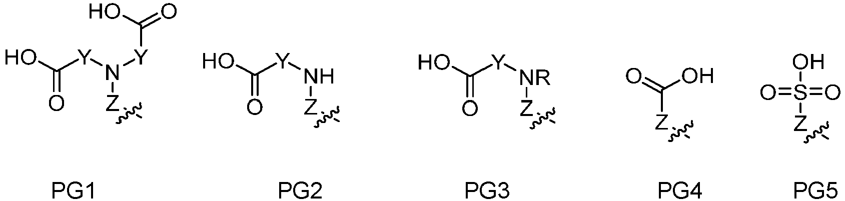
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 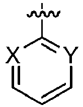
wherein X and Y are independently selected from: -CH₂- and -N-;
   and
   wherein the tetrazine compound of formula I has a lipophilicity of cLogD7.4 < -0.5,
      or
   a precursor of formula II: 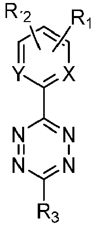 wherein R₁ is 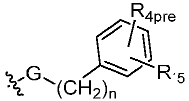
   wherein G is selected from -O-, -S-, -NH-, and -N-alkyl-;
   n is a digit from 0 to 4;
   R₄ₚᵣₑ is a group selected from T₃Sn, T₃Si or T₃Ge, wherein T is a; straight chain or branched alkyl group, independently selected from methyl, ethyl, propyl, butyl, pentyl, hexyl, heptyl, octyl, nonyl, and decyl,
   R'₅ is -H- or a polar group (PG) selected from: 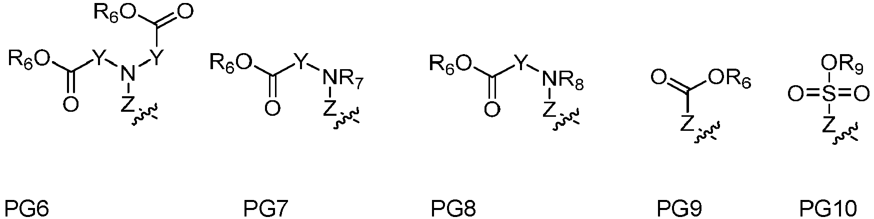
   wherein Z is CO(CH₂)ₙ, SO₂(CH₂)ₙ, (CH₂)ₙ or (OCH₂CH2)ₙ and n = 0, 1, 2 or 3; and
   wherein Y is (CH₂)ₙ, (OCH₂CH2)ₙ or CO(CH₂)ₙ and n is a digit from 0 to 3;
   and wherein R₆ is selected from -H, tert-butyl, and trityl;
   R₇ is -H or Boc;
   R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, (OCH₂CH₂)ₙOCH₂COOC(CH₃)₃, and n is a digit from 0 to 3;
   R₉ is selected from -H, neopentyl, and α-trifluoromethylbenzyl;
   and wherein the curly sign indicates the link to the aromatic ring,
   R'₂ is -H- or a polar group (PG) selected from: 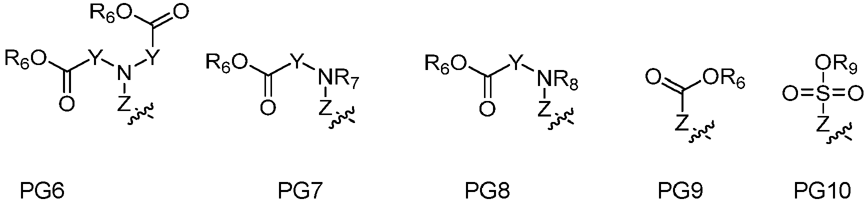
   wherein Z is CO(CH₂)ₙ, SO₂(CH₂)ₙ, (CH₂)ₙ or (OCH₂CH2)ₙ and n = 0, 1, 2 or 3; and
   wherein Y is (CH₂)ₙ, (OCH₂CH₂)ₙ or CO(CH₂)ₙ and n is a digit from 0 to 3;
   and wherein R₆ is selected from -H, tert-butyl, and trityl;
   R₇ is -H or Boc;
   R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or
   (OCH₂CH₂)ₙOCH₂COOC(CH₃)₃, wherein n is a digit from 0 to 3;
   R₉ is selected from -H, neopentyl, and α-trifluoromethylbenzyl;
   and wherein the curly sign indicates the link to the aromatic ring,
   R₃ is -H or 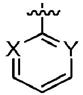
   wherein X and Y are independently selected from: -CH₂- and -N--, and wherein the curly sign indicates the link to the aromatic ring;
   and a dienophile having complementary inverse electron demand Diels-Alder cycloaddition reactivity to the tetrazine compound of formula I.

In a preferred embodiment, the kit comprises a compound of formula I: 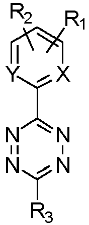 wherein R₁ is 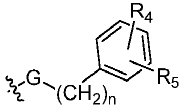
wherein G is selected from -O-, -S-, -NH-, and -N-alkyl-;
n is a digit from 0 to 4;
R₄ is a radioisotope selected from ¹⁸F, ¹⁹F, ⁷⁶Br, ⁷⁷Br, ¹²⁵I, ¹²³I, ¹³¹I, ¹²⁴I, ¹²⁷I and ²¹¹At;
R₅ is -H- or a polar group (PG) selected from: 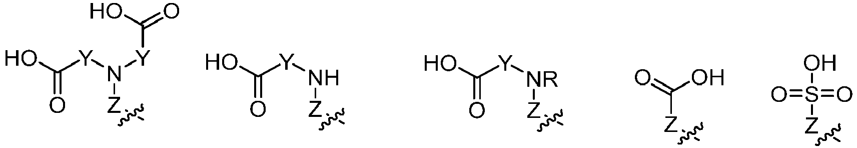 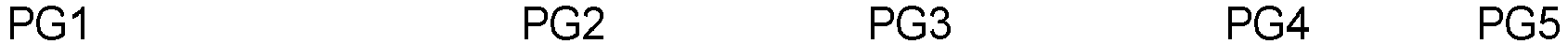
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 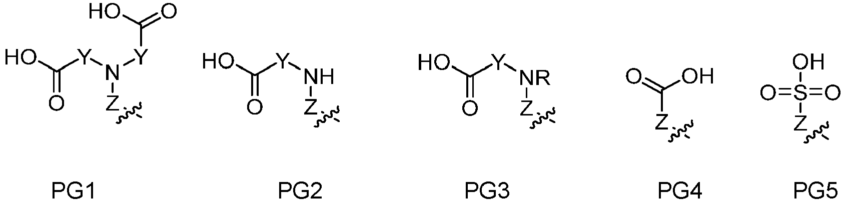
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 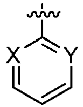
wherein X and Y are independently selected from: -CH₂- and -N-;
   and
   wherein the tetrazine compound of formula I has a lipophilicity of cLogD7.4 < -0.5,
   and a dienophile having complementary inverse electron demand Diels-Alder cycloaddition reactivity to the tetrazine compound of formula I.

In another preferred embodiment, the kit comprises a precursor of formula II: 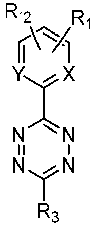 wherein R₁ is 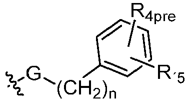
wherein G is selected from -O-, -S-, -NH-, and -N-alkyl-;
n is a digit from 0 to 4;
R₄ₚᵣₑ is a group selected from T₃Sn, T₃Si or T₃Ge, wherein T is a; straight chain or branched alkyl group, independently selected from methyl, ethyl, propyl, butyl, pentyl, hexyl, heptyl, octyl, nonyl, and decyl,
R'₅ is -H- or a polar group (PG) selected from: 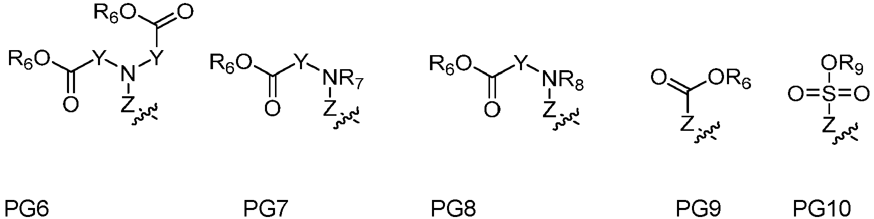
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH2)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, (OCH₂CH₂)ₙOCH₂COOC(CH₃)₃, and n is a digit from 0 to 3;
R₉ is selected from -H, neopentyl, and α-trifluoromethylbenzyl;
and wherein the curly sign indicates the link to the aromatic ring,
R'₂ is -H- or a polar group (PG) selected from: 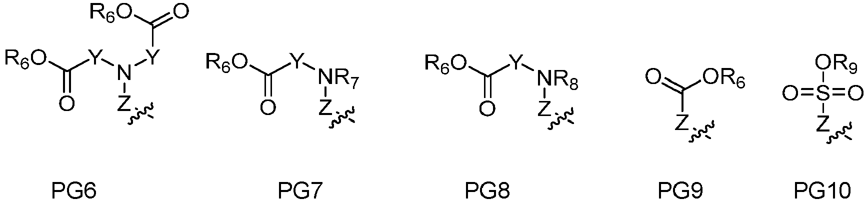
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or (OCH₂CH₂)ₙOCH₂COOC(CH₃)₃, wherein n is a digit from 0 to 3;
R₉ is selected from -H, neopentyl, and α-trifluoromethylbenzyl;
and wherein the curly sign indicates the link to the aromatic ring,
R₃ is -H or 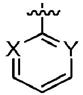
wherein X and Y are independently selected from: -CH₂- and -N-, and wherein the curly sign indicates the link to the aromatic ring;
and a dienophile having complementary inverse electron demand Diels-Alder cycloaddition reactivity.

For all embodiments, the targeting vector can be any suitable vector directed at a specific target and includes antibodies, nanobodies, polymers, nanomedicines, cells, proteins, peptides, and small molecules.

In a preferred embodiment, the dienophile is conjugated to a targeting vector, thus providing a kit wherein said dienophile having complementary inverse electron demand Diels-Alder cycloaddition reactivity is conjugated to a targeting vector.

### EXAMPLES

### Example 1 - Synthesis of compounds of formula I and precursors

### 1.1 Synthesis overview

The shortest linker derivatives **12**, **13** and their precursor **14** were obtained as shown in Scheme 1. Initially 3-hydroxy-4-methylbenzonitrile was reacted with 4-fluoro, or 4-iodoboronic acid under Chan-Lam coupling conditions to give **4** and **5.** The latters were brominated and converted to compounds **8** and **9.** Tetrazines **10** and **11** were then obtained using a Pinner-like, sulfur-mediated procedure reported by Qu et al.³² Finally deprotection in acidic conditions gave **12** and **13.** The precursor **14** was instead obtained under Stille coupling conditions from **11.**

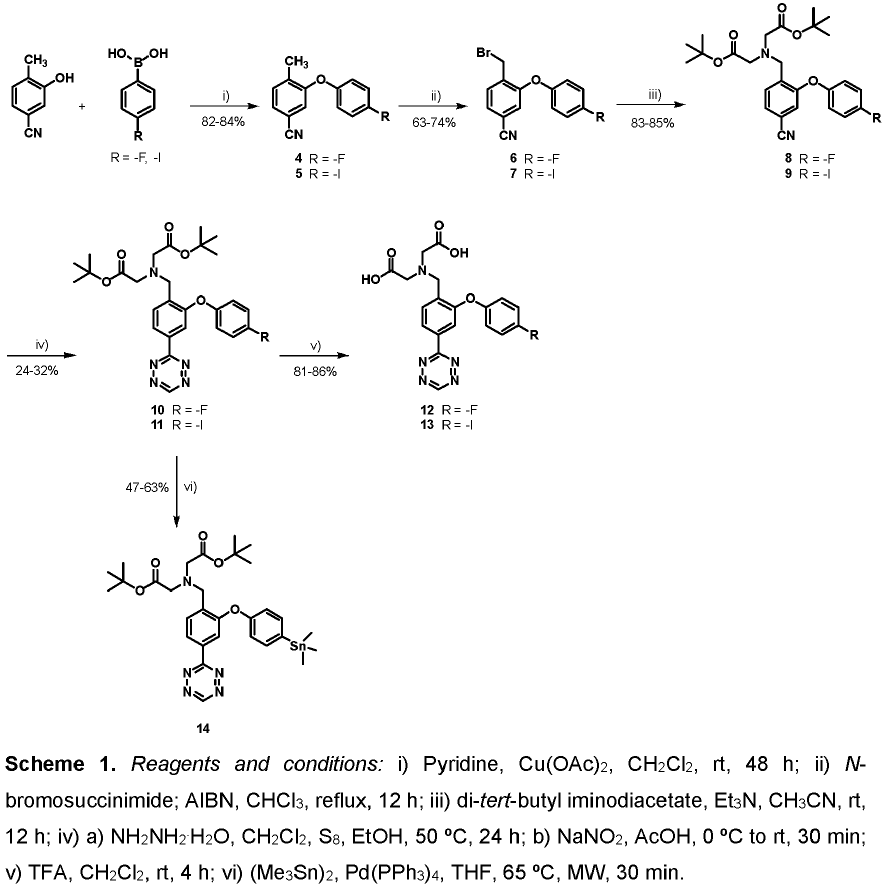

The next derivatives in the homologue series, with one or two carbon units in the linker were obtained as shown in Scheme 2. The common intermediate **18** was synthesized from 3-hydroxy-4-methylbenzonitrile in 4 steps as previously described.²⁰ Compounds **19-22** were isolated after reacting **18** with the selected benzyl bromide or iodophenethyl tosylate derivatives. Tetrazines **23-26** were then synthesized as described above. Finally, deprotection in TFA/CH₂Cl₂ gave the desired compounds **27-30.** The corresponding trimethylstannyl analogues **31** and **32** were obtained by Pd-mediated Stille coupling.

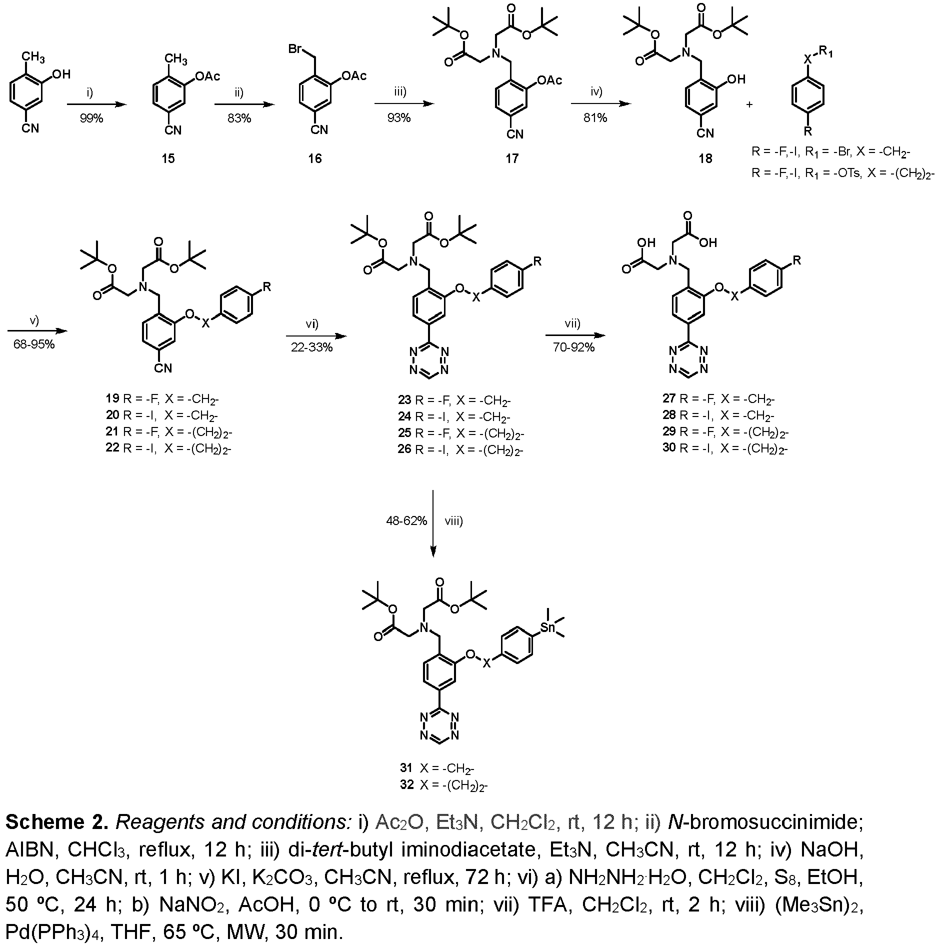

The last derivatives **41** and **42** and their precursor **43** were synthesized as described in Scheme 3. *Tert*-butyl protected benzyl bromides **35** and **36** were obtained from the corresponding acids by esterification and bromination. Alkylation of **18** gave **37** and **38**; the latter were subsequently converted to the corresponding tetrazines 39 and **40.** These were deprotected under acidic conditions to yield **41** and **42.** Stannyl **43** was again obtained from the iodine **40.**

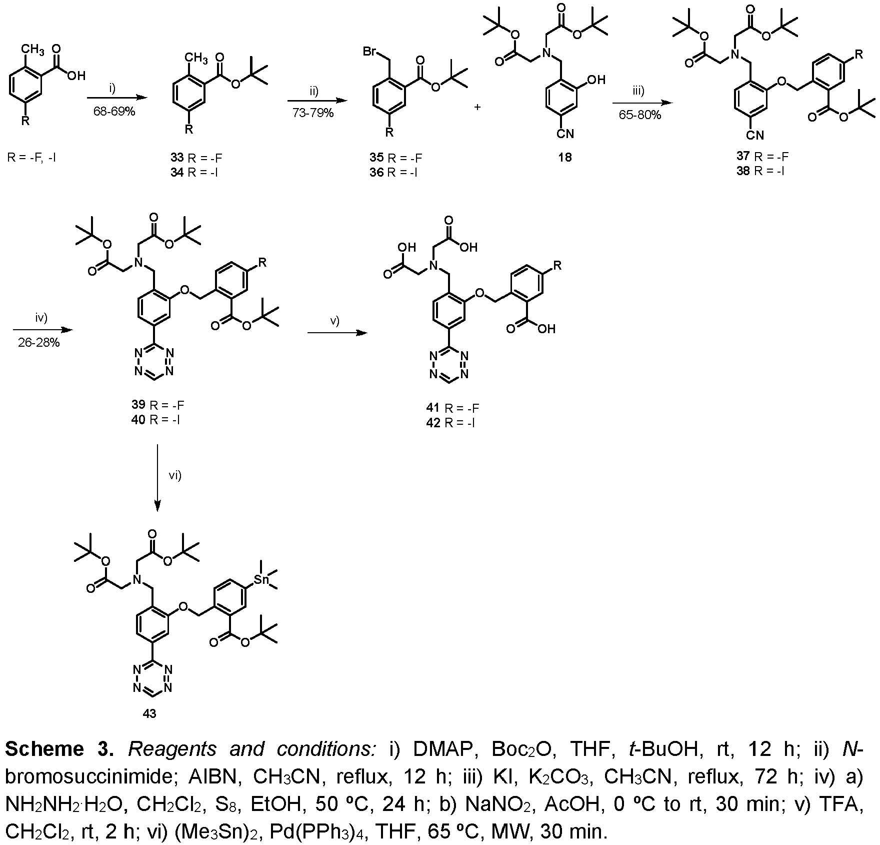

All reagents and solvents were dried prior to use according to standard methods. Commercial reagents were used without further purification. Analytical TLC was performed using silica gel 60 F254 (Merck) with detection by UV absorption and/or by charring following immersion in a 7% ethanolic solution of sulfuric acid or KMnO₄-solution (1.5 g of KMnO₄, 10 g K₂CO₃, and 1.25 mL 10% NaOH in 200 mL water). Purification of compounds was carried out by column chromatography on silica gel (40-60 µm, 60 Å) or employing a CombiFlash NextGen 300+ (Teledyne ISCO). ¹H and ¹³C NMR spectra were recorded on Brucker (400 and 600 MHz instruments), using Chloroform-*d*, Methanol-*d*₄ or DMSO-*d*₆ as deuterated solvent and with the residual solvent as the internal reference. For all NMR experiments the deuterated solvent signal was used as the internal lock. Chemical shifts are reported in δ parts per million (ppm). Coupling constants (*J* values) are given in Hertz (Hz). Multiplicities of ¹H NMR signals are reported as follows: s, singlet; d, doublet; dd, doublet of doublets; ddd, doublet of doublets of doublets; dt, doublet of triplets; t, triplet; q, quartet; m, multiplet; br, broad signal. NMR spectra of all compounds are reprocessed in MestReNova software (version 12.0.22023) from original FID's files. Mass spectra analysis was performed using MS-Acquity-A: Waters Acquity UPLC with QDa-detector. Purification by preparative HPLC was performed on Agilent 1260 infinity system, column SymmetryPrep-C18, 17 mL/min H₂O-MeCN gradient 50-100% 15 min with 0.1% trifluoroacetic acid. All final compounds were >95% pure as determined by analytical HPLC. Analytical HPLC method: (Thermo Fisher^{®} UltiMate 3000) with a C-18 column (Luna^{®} 5u C18(2) 100Å, 150 × 4.6 mm), eluents: A: H2O with 0.1% TFA, B: MeCN with 0.1% TFA. Gradient from 100% A -> 100% B over 15minutes, back to 100% A over 4 minutes, flow rate 1.5 mL/min. Detection by UV-absorption at λ = 254 nm on a UVD 170U detector.

### 1.2 3-(4-Fluorophenoxy)-4-methylbenzonitrile (4)

The compound was synthesized following a published procedure for similar molecules.⁴⁰ A mixture of 3-hydroxy-4-methylbenzonitrile (1.33 g, 10.0 mmol), 4-flourophenylboronic acid (2.09 g, 15.0 mmol), anhydrous Cu(OAc)₂ (2.0 g, 11.0 mmol), pyridine (4.02 mL, 50 mmol) and powdered 4 Å molecular sieves (1 g) in anhydrous CH₂Cl₂ (70 mL) was stirred at room temperature for 48 h. The mixture was filtered and concentrated under reduced pressure. Purification by flash chromatography (90/10 n-Heptane/EtOAc) afforded 1.92 g (84%) of the desired product as a colorless oil. Rf = 0.39 (90/10 n-Heptane/EtOAc); ¹H NMR (400 MHz, CDCl₃) δ 7.28 - 7.15 (m, 2H), 7.04 - 6.91 (m, 2H), 6.88 (d, *J* = 1.5 Hz, 1H), 6.86 - 6.71 (m, 2H), 2.25 (s, 3H); ¹³C NMR (101 MHz, CDCl₃) δ 159.20 (d, *J* = 242.9 Hz), 156.06, 151.89 (d, *J* = 2.7 Hz), 134.99, 132.24, 126.86, 120.36 (d, *J* = 8.4 Hz), 120.18, 116.85, 116.61, 110.68, 16.57.

### 1.3 3-(4-lodophenoxy)-4-methylbenzonitrile (5)

The compound was synthesized following a published procedure for similar molecules.⁴⁰ A mixture of 3-hydroxy-4-methylbenzonitrile (1.33 g, 10.0 mmol), 4-iodophenylboronic acid (3.71 g, 15.0 mmol), anhydrous Cu(OAc)₂ (2.0 g, 11.0 mmol), pyridine (4.02 mL, 50 mmol) and powdered 4 Å molecular sieves (1 g) in anhydrous CH₂Cl₂ (70 mL) was stirred at room temperature for 48 h. The mixture was filtered and concentrated under reduced pressure. Purification by flash chromatography (90/10 n-Heptane/EtOAc) afforded 2.76 g (82%) of the desired product as a colorless oil. Rf = 0.41 (90/10 n-Heptane/EtOAc); ¹H NMR (400 MHz, CDCl₃) δ 7.65 (d, *J* = 8.8 Hz, 2H), 7.34 (d, *J* = 1.1 Hz, 2H), 7.09 (s, 1H), 6.70 (d, *J* = 8.8 Hz, 2H), 2.32 (s, 3H); ¹³C NMR (101 MHz, CDCl₃) δ 156.39, 154.92, 139.06, 135.71, 132.41, 127.58, 121.67, 120.41, 110.88, 86.80, 16.61.

### 1.4 4-(Bromomethyl)-3-(4-fluorophenoxy)benzonitrile (6)

To a solution of 4 (1.92 g, 8.45 mmol) and N-bromo succinimide (1.65 g, 9.29 mmol) in CHCl₃ (30 mL) was added AIBN (0.55 g, 3.37 mmol). The resulting solution was refluxed for 12 h. The reaction was cooled down and the organic layer was washed with water (2 × 30 mL) and brine (2 × 30 mL), dried over anhydrous Na₂SO₄, filtered and concentrated under reduced pressure. Purification by flash chromatography (90/10 n-Heptane/EtOAc) afforded 1.92 g (74%) of the desired product as a white solid. Rf = 0.31 (90/10 n-Heptane/EtOAc); ¹H NMR (400 MHz, CDCl₃) δ 7.53 (d, *J* = 7.8 Hz, 1H), 7.33 (dd, *J* = 7.8, 1.5 Hz, 1H), 7.20 - 7.09 (m, 2H), 7.09 - 7.00 (m, 2H), 6.95 (d, *J* = 1.5 Hz, 1H), 4.60 (s, 2H); ¹³C NMR (101 MHz, CDCl₃) δ 159.84 (d, *J* = 244.4 Hz), 156.44, 150.95 (d, *J* = 2.7 Hz), 133.18, 132.01, 126.55, 121.66 (d, *J* = 8.5 Hz), 119.64, 117.81, 117.06 (d, *J* = 23.5 Hz), 113.50, 26.26.

### 1.5 4-(Bromomethyl)-3-(4-iodophenoxy)benzonitrile (7)

To a solution of **5** (2.71 g, 8.05 mmol) and N-bromo succinimide (1.57 g, 8.86 mmol) in CHCl₃ (30 mL) was added AIBN (0.53 g, 3.22 mmol). The resulting solution was refluxed for 12 h. The reaction was cooled down and the organic layer was washed with water (2 × 30 mL) and brine (2 × 30 mL), dried over anhydrous Na₂SO₄, filtered and concentrated under reduced pressure. Purification by flash chromatography (90/10 n-Heptane/EtOAc) afforded 2.1 g (63%) of the desired product as a white solid. Rf = 0.33 (90/10 n-Heptane/EtOAc); ¹H NMR (600 MHz, CDCl₃) δ 7.81 - 7.67 (m, 1.7H), 7.63 - 7.50 (m, 1.3H), 7.37 (dd, *J* = 7.9, 1.5 Hz, 1H), 7.11 - 7.01 (m, 1H), 6.98 - 6.94 (m, 0.3H), 6.89 - 6.78 (m, 1.7H), 4.57 (s, 0.6H), 4.57 (s, 1.4H); ¹³C NMR (151 MHz, CDCl₃) δ 155.53, 155.44, 139.37, 133.81, 132.09, 127.19, 121.87, 120.78, 117.66, 113.60, 88.39, 26.07.

### 1.6 Di-tert-butyl 2,2'-((4-cyano-2-(4-fluorophenoxy)benzyl)azanediyl)diacetate (8)

To a solution of **6** (1.81 g, 5.88 mmol) in CH₃CN (50 mL) was added K₂CO₃ (2.43 g, 17.64 mmol) and di-*tert*-butyl iminodiacetate (1.51 g, 6.17 mmol). The reaction mixture was stirred at room temperature overnight and then the solvent was concentrated under reduced pressure. The resulting mixture was diluted with water (20 mL), extracted with EtOAc (2 × 25 mL), washed with brine (30 mL), dried over MgSO₄, filtered and concentrated under reduced pressure. Purification by flash chromatography (90/10 n-Heptane/EtOAc) afforded 2.36 g (85%) of the desired product as a white solid. Rf = 0.27 (90/10 n-Heptane/EtOAc); ¹H NMR (400 MHz, CDCl₃) δ 7.83 (d, *J* = 7.9 Hz, 1H), 7.34 (dd, *J* = 7.9, 1.6 Hz, 1H), 7.08 - 6.98 (m, 2H), 6.98 - 6.88 (m, 3H), 4.01 (s, 2H), 3.43 (s, 4H), 1.39 (s, 18H); ¹³C NMR (101 MHz, CDCl₃) δ 170.41, 159.33 (d, *J* = 243.2 Hz), 156.08, 151.63 (d, *J* = 2.7 Hz), 135.40, 131.73, 120.85 (d, *J* = 8.3 Hz), 118.30, 116.86, 116.63, 111.64, 81.11, 68.15, 55.77, 51.22, 28.10.

### 1.7 Di-tert-butyl 2,2'-((4-cyano-2-(4-fluorophenoxy)benzyl)azanediyl)diacetate (9)

To a solution of **7** (1.81 g, 4.35 mmol) in CH₃CN (50 mL) was added K₂CO₃ (1.81 g, 13.04 mmol) and di-tert-butyl iminodiacetate (1.12 g, 4.56 mmol). The reaction mixture was stirred at room temperature overnight and then the solvent was concentrated under reduced pressure. The resulting mixture was diluted with water (20 mL), extracted with EtOAc (2 × 25 mL), washed with brine (30 mL), dried over MgSO₄, filtered and concentrated under reduced pressure. Purification by flash chromatography (90/10 n-Heptane/EtOAc) afforded 2.1 g (83%) of the desired product as a white solid. Rf = 0.32 (90/10 n-Heptane/EtOAc); ¹H NMR (400 MHz, CDCl₃) δ 7.83 (d, *J* = 7.9 Hz, 1H), 7.66 - 7.56 (m, 1.7H), 7.44 - 7.31 (m, 1.3H), 7.02 - 6.92 (m, 1H), 6.85 - 6.73 (m, 0.3H), 6.68 - 6.54 (m, 1.7H), 3.92 (s, 2H), 3.37 (s, 4H), 1.35 (s, 18H); ¹³C NMR (101 MHz, CDCl₃) δ 170.40, 156.21, 154.96, 139.08, 136.22, 133.11, 131.85, 127.58, 121.15, 120.89, 120.56, 118.19, 111.81, 81.23, 55.78, 50.94, 28.14.

### 1.8 Di-tert-butyl 2,2'-((2-(4-fluorophenoxy)-4-(1,2,4,5-tetrazin-3-yl)benzyl)azanediyl) diacetate (10)

The compound was obtained following the reported procedure.³² CH₂Cl₂ (0.061 mL, 0.95 mmol), sulfur (0.061 g, 0.24 mmol), hydrazine monohydrate (0.37 mL, 7.65 mmol) and ethanol (4.0 mL) along with **8** (0.45 g, 0.95 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNO₂ (0.66 g, 9.56 mmol) in water (10 ml) were added to the now yellow mixture followed by dropwise addition of acetic acid (4 mL), producing a mixture red in color. The reaction mixture was extracted with CH₂Cl₂, washed with brine, dried with MgSO₄ and filtered before concentrating *in vacuo.* The crude was purified using flash chromatography (95/5 n-Heptane/EtOAc) to yield 0.12 g (24%) of **10** as a red solid. Rf = 0.22 (80/20 n-Heptane/EtOAc); ¹H NMR (400 MHz, CDCl₃) δ 10.16 (s, 1H), 8.37 (dd, *J* = 8.1, 1.8 Hz, 1H), 8.00 (d, *J* = 1.8 Hz, 1H), 7.95 (d, *J* = 8.1 Hz, 1H), 7.08 - 6.92 (m, 4H), 4.09 (s, 2H), 3.51 (s, 4H), 1.43 (s, 18H);
¹³C NMR (101 MHz, CDCl₃) δ 170.55, 166.00, 159.01 (d, *J* = 242.2 Hz), 157.73, 156.53, 152.58 (d, *J* = 2.6 Hz), 135.45, 132.04, 131.78, 123.40, 120.31 (d, *J* = 8.4 Hz), 116.98, 116.56 (d, *J* = 23.5 Hz), 81.12, 55.77, 51.40, 28.15.

### 1.9 Di-tert-butyl 2,2'-((2-(4-iodophenoxy)-4-(1,2,4,5-tetrazin-3-yl)benzyl)azanediyl) diacetate (11)

The compound was obtained following the reported procedure.³² CH₂Cl₂ (0.22 mL, 3.46 mmol), sulfur (0.22 g, 0.86 mmol), hydrazine monohydrate (1.35 mL, 27.66 mmol) and ethanol (4.0 mL) along with **9** (2.0 g, 3.45 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNO₂ (2.38 g, 34.57 mmol) in water (30 ml) were added to the now yellow mixture followed by dropwise addition of acetic acid (7 mL), producing a mixture red in color. The reaction mixture was extracted with CH₂Cl₂, washed with brine, dried with MgSO₄ and filtered before concentrating *in vacuo.* The crude was purified using flash chromatography (95/5 n-Heptane/EtOAc) to yield 0.71 g (32%) of **11** as a red solid. Rf = 0.39 (80/20 n-Heptane/EtOAc); ¹H NMR (600 MHz, CDCl₃) δ 10.16 (s, 1H), 8.39 (dd, *J* = 8.1, 1.8 Hz, 1H), 8.17 - 8.01 (m, 1H), 7.97 (d, *J* = 8.1 Hz, 1H), 7.77 - 7.53 (m, 1.7H), 7.41 (d, *J* = 8.9 Hz, 0.3H), 6.87 (d, *J* = 8.9 Hz, 0.3H), 6.87 - 6.52 (m, 1.7H), 4.03 (s, 2H), 3.47 (s, 4H), 1.41 (s, 18H); ¹³C NMR (151 MHz, CDCl₃) δ 170.48, 165.88, 157.79, 157.11, 155.33, 138.85, 136.21, 132.89, 132.09, 131.90, 124.03, 120.44, 120.08, 118.28, 86.33, 81.10, 55.74, 51.39, 28.16.

### 1.10 2,2'-((2-(4-Fluorophenoxy)-4-(1,2,4,5-tetrazin-3-yl)benzyl)azanediyl)diacetic acid (12)

To a solution of **10** (0.09 g, 0.17 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.08 g (86%) of **12** (TFA salt) as a pink solid.
¹H NMR (400 MHz, MeOD) δ 10.36 (s, 1H), 8.41 (dd, *J* = 8.0, 1.7 Hz, 1H), 8.25 - 7.82 (m, 2H), 7.52 - 6.40 (m, 5H), 4.87 (s, 2H), 4.32 (s, 4H); ¹³C NMR (101 MHz, MeOD) δ 167.46, 165.34, 160.08 (d, *J* = 242.9 Hz), 158.48, 158.13, 150.58 (d, *J* = 2.6 Hz), 135.88, 134.76, 123.07, 122.21, 122.01 (d, *J* = 8.5 Hz), 116.59 (d, *J* = 23.9 Hz), 114.50, 53.75, 52.99.

### 1.11 2,2'-((2-(4-lodophenoxy)-4-(1,2,4,5-tetrazin-3-yl)benzyl)azanediyl)diacetic acid (13)

To a solution of **11** (0.08 g, 0.12 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.06 g (81%) of **13** (TFA salt) as a pink solid. ¹H NMR (600 MHz, DMSO) δ 10.58 (s, 1H), 8.33 (dd, *J* = 8.0, 1.7 Hz, 1H), 7.93 (d, *J* = 8.0 Hz, 1H), 7.88 (d, *J* = 1.7 Hz, 1H), 7.77 - 7.72 (m, 2H), 6.94 - 6.87 (m, 2H), 4.03 (s, 2H), 3.52 (s, 4H); ¹³C NMR (151 MHz, DMSO) δ 172.70, 165.33, 158.64, 156.99, 155.53, 139.28, 135.57, 132.77, 132.42, 123.76, 121.45, 117.50, 87.94, 54.57, 51.52.

### 1.12 Di-tert-butyl 2,2'-((4-(1,2,4,5-tetrazin-3-yl)-2-(4-(trimethylstannyl)phenoxy)benzyl) azanediyl)diacetate (14)

Palladium tetrakis (0.01 g, 0.009 mmol, 10%), hexamethylditin (0.05 mL, 0.24 mmol,) and dry THF (2 mL) were successively added to a microwave vial equipped with a stir bar which was then sealed and purged with N₂.⁴¹ A solution of **11** (0.06 g, 0.09 mmol) in dry THF (2 mL) was added via a syringe and the reaction allowed to stir at 65 °C in a microwave for 30 minutes. The reaction was allowed to cool to room temperature and unsealed before being quenched with saturated aqueous KF (1 mL). The solution was extracted with CH₂Cl₂ (3 × 5 mL), washed with brine (2 × 10 mL), dried over MgSO₄, filtered and concentrated under reduced pressure. The crude was purified using flash chromatography (90/10 n-Heptane/EtOAc) to yield 0.25 g (40%) of a pink solid. Rf = 0.42 (80/20 n-Heptane/EtOAc); ¹H NMR (400 MHz, CDCl₃) δ 10.16 (s, 1H), 8.40 (dd, *J* = 8.1, 1.7 Hz, 1H), 8.11 (d, *J* = 1.7 Hz, 1H), 8.00 (d, *J* = 8.1 Hz, 1H), 7.51 - 7.39 (m, 2H), 7.04 - 6.91 (m, 2H), 4.09 (s, 2H), 3.51 (s, 4H), 1.43 (s, 18H), 0.29 (s, 9H); ¹³C NMR (101 MHz, CDCl₃) δ 170.57, 166.11, 157.70, 157.34, 155.91, 137.30, 136.36, 136.12, 131.89, 131.68, 123.57, 118.21, 118.10, 81.07, 55.79, 51.42, 28.16, -9.48.

### 1.13 5-Cyano-2-methylphenyl acetate (15)

To a solution of 3-hydroxy-4-methylbenzonitrile (3.11 g, 23.28 mmol) and Et₃N (9.74 mL, 7.07 mmol) in CH₂Cl₂ (30 mL)was added acetic anhydride (2.64 mL, 27.93 mmol). The resulting mixture was stirred at room temperature for 12 h. The organic layer was then washed with water (2 × 30 mL) and brine (2 × 30 mL), dried over anhydrous Na₂SO₄, filtered and concentrated under reduced pressure to give 4.05 g (99%) of compound **15** as a white solid. Rf = 0.37 (80/20 n-Heptane/EtOAc); ¹H NMR (400 MHz, CDCl₃) δ 7.77 - 7.34 (m, 1H), 7.33 - 7.03 (m, 2H), 2.45 - 2.27 (m, 3H), 2.26 - 2.16 (m, 3H); ¹³C NMR (101 MHz, CDCl₃) δ 168.55, 149.41, 136.64, 132.04, 129.62, 125.74, 118.05, 110.64, 20.64, 16.57.

### 1.14 2-(Bromomethyl)-5-cyanophenyl acetate (16)

To a solution of **15** (3.00 g, 17.12 mmol) and N-bromo succinimide (4.57 g, 25.68 mmol) in CHCl₃ (50 mL) was added AIBN (1.12 g, 6.85 mmol). The resulting solution was refluxed for 12 h. The reaction was cooled down and the organic layer was washed with water (2 × 30 mL) and brine (2 × 30 mL), dried over anhydrous Na₂SO₄, filtered and concentrated under reduced pressure. Purification by flash chromatography (90/10 n-Heptane/EtOAc) afforded 3.6 g (83%) of **16** as a white solid. Rf = 0.35 (80/20 n-Heptane/EtOAc); ¹H NMR (400 MHz, CDCl₃) δ 7.66-7.41 (m, 3H), 4.40 (s, 2H), 2.39 (s, 3H); ¹³C NMR (101 MHz, CDCl₃) δ 168.16, 148.95, 135.16, 131.68, 129.83, 126.90, 117.46, 113.41, 25.86, 20.87.

### 1.15 Di-tert-butyl 2,2'-((2-acetoxy-4-cyanobenzyl)azanediyl)diacetate (17)

To a solution of **16** (3.61 g, 14.17 mmol) in CH₃CN (50 mL) was added Et₃N (5.92 mL, 42.51 mmol) and di-tert-butyl iminodiacetate (3.65 g, 14.87 mmol). The reaction mixture was stirred at room temperature overnight and then the solvent was concentrated under reduced pressure. The resulting mixture was diluted with water (100 mL), extracted with EtOAc (3 × 40 mL), washed with brine (50 mL), dried over MgSO₄, filtered and concentrated under reduced pressure. Purification by flash chromatography (90/10 n-Heptane/EtOAc) afforded 5.51 g (93%) of **17** as a yellow oil. Rf = 0.39 (80/20 n-Heptane/EtOAc); ¹H NMR (400 MHz, CDCl₃) δ 7.83 (d, *J* = 8.0 Hz, 1H), 7.52 (dd, *J* = 8.0, 1.6 Hz, 1H), 7.32 (d, *J* = 1.6 Hz, 1H), 3.90 (s, 2H), 3.37 (s, 4H), 2.33 (s, 3H), 1.45 (s, 9H); ¹³C NMR (101 MHz, CDCl₃) δ 168.36, 167.03, 147.56, 135.47, 129.90, 127.91, 124.34, 116.09, 110.08, 79.34, 53.25, 49.75, 26.27, 18.77.

### 1.16 Di-tert-butyl 2,2'-((4-cyano-2-hydroxybenzyl)azanediyl)diacetate (18)

To a solution of **17** (5.5 g, 13.14 mmol) in CH₃CN (50 mL) was added a 1M NaOH solution (20 mL). The mixture was stirred at room temperature for 12 h. The mixture then concentrated under reduced pressure and neutralized with a 1M HCl solution. The resulting slurry was extracted with EtOAc (3 × 40 mL), washed with brine (50 mL), dried over MgSO₄, filtered and concentrated under reduced pressure to give 4.02 g (81%) of **18** as a beige solid. Rf = 0.36 (80/20 n-Heptane/EtOAc); ¹H NMR (600 MHz, CDCl₃) δ 10.05 (s, 1H), 7.14 (d, *J* = 1.5 Hz, 1H), 7.07 (dd, *J* = 7.7, 1.6 Hz, 1H), 7.04 (d, *J* = 7.7 Hz, 1H), 3.98 (s, 2H), 3.37 (s, 4H), 1.47 (s, 9H); ¹³C NMR (151 MHz, CDCl₃) δ 169.99, 157.98, 130.17, 127.17, 122.90, 120.02, 118.82, 112.86, 82.38, 55.54, 54.94, 28.11.

### 1.17 Di-tert-butyl 2,2'-((4-cyano-2-((4-fluorobenzyl)oxy)benzyl)azanediyl)diacetate (19)

To a solution of **18** (0.60 g, 1.59 mmol) in in CH₃CN (50 mL) was added 4-fluorobenzylbromide (0.25 mL, 2.04 mmol), K₂CO₃ (0.77 g, 5.58 mmol) and KI (cat.). The resulting suspension was refluxed for 72 hours. The mixture was cooled to room temperature, filtered and concentrated under reduced pressure. The crude was directly purified by flash chromatography (90/10 n-Heptane/EtOAc) to give 0.61 g (78%) of the desired product as a colorless oil. Rf = 0.39 (80/20 n-Heptane/EtOAc); ¹H NMR (400 MHz, CDCl₃) δ 7.75 (d, *J* = 7.8 Hz, 1H), 7.45 - 7.36 (m, 2H), 7.28 (dd, *J* = 7.7, 1.4 Hz, 1H), 7.12 - 7.03 (m, 3H), 5.05 (s, 2H), 4.00 (s, 2H), 3.44 (s, 4H), 1.43 (s, 18H); ¹³C NMR (101 MHz, CDCl₃) δ 170.48, 162.59 (d, *J* = 246.9 Hz), 156.47, 134.00, 131.83 (d, *J* = 3.3 Hz), 130.65, 129.14 (d, *J=* 8.2 Hz), 125.20, 118.98, 115.61 (d, *J* = 21.6 Hz), 114.44, 111.28, 81.05, 69.74, 55.78, 51.76, 28.12.

### 1.18 Di-tert-butyl 2,2'-((4-cyano-2-((4-iodobenzyl)oxy)benzyl)azanediyl)diacetate (20)

To a solution of **18** (1.20 g, 3.18 mmol) in in CH₃CN (70 mL) was added 4-iodobenzylbromide (1.04 g, 3.50 mmol), K₂CO₃ (1.32 g, 9.56 mmol) and KI (cat.). The resulting suspension was refluxed for 72 hours. The mixture was cooled to room temperature, filtered and concentrated under reduced pressure. The crude was directly purified by flash chromatography (90/10 n-Heptane/EtOAc) to give 1.41 g (74%) of the desired product as a colorless oil. Rf = 0.48 (80/20 n-Heptane/EtOAc); ¹H NMR (400 MHz, CDCl₃) δ 7.73 (d, *J* = 7.8 Hz, 1H), 7.71 - 7.61 (m, 2H), 7.25 (dd, *J* = 7.8, 1.4 Hz, 1H), 7.16 (d, *J* = 8.1 Hz, 2H), 7.07 (d, *J* = 1.5 Hz, 1H), 5.01 (s, 2H), 3.99 (s, 2H), 3.43 (s, 4H), 1.42 (s, 18H); ¹³C NMR (101 MHz, CDCl₃) δ 170.41, 156.38, 137.76, 135.76, 133.94, 130.70, 129.10, 125.23, 118.89, 114.40, 111.33, 93.75, 81.00, 69.69, 55.76, 51.78, 28.14.

### 1.19 Di-tert-butyl 2,2'-((4-cyano-2-(4-fluorophenethoxy)benzyl)azanediyl)diacetate (21)

To a solution of **18** (0.50 g, 1.33 mmol) in in CH₃CN (70 mL) was added 4-fluorophenethyl 4-methylbenzenesulfonate (0.43 g, 1.46 mmol), K₂CO₃ (0.55 g, 3.98 mmol) and KI (cat.). The resulting suspension was refluxed for 72 hours. The mixture was cooled to room temperature, filtered and concentrated under reduced pressure. The crude was directly purified by flash chromatography (90/10 n-Heptane/EtOAc) to give 0.45 g (68%) of the desired product as a colorless oil. Rf = 0.21 (80/20 n-Heptane/EtOAc); ¹H NMR (400 MHz, CDCl₃) δ 7.62 (d, *J* = 7.8 Hz, 1H), 7.21 - 7.13 (m, 3H), 6.97 - 6.87 (m, 3H), 4.07 (t, *J* = 6.6 Hz, 2H), 3.85 (s, 2H), 3.35 (s, 4H), 3.01 (t, *J* = 6.6 Hz, 2H), 1.38 (s, 18H); ¹³C NMR (101 MHz, CDCl₃) δ 170.49, 161.78 (d, *J* = 244.7 Hz), 156.62, 133.76, 133.54 (d, *J* = 3.2 Hz), 130.50 (d, *J* = 8.0 Hz), 130.32, 124.92, 119.05, 115.37 (d, *J* = 21.3 Hz), 113.74, 111.18, 81.08, 69.07, 55.77, 51.87, 34.81, 28.17.

### 1.20 Di-tert-butyl 2,2'-((4-cyano-2-(4-fluorophenethoxy)benzyl)azanediyl)diacetate (22)

To a solution of **18** (1.85 g, 2.26 mmol) in in CH₃CN (70 mL) was added 4-iodophenethyl 4-methylbenzenesulfonate (1.00 g, 2.48 mmol), K₂CO₃ (0.94 g, 6.77 mmol) and KI (cat.). The resulting suspension was refluxed for 72 hours. The mixture was cooled to room temperature, filtered and concentrated under reduced pressure. The crude was directly purified by flash chromatography (90/10 n-Heptane/EtOAc) to give 0.45 g (95%) of the desired product as a colorless oil (pure enough for the next step). Rf = 0.25 (80/20 n-Heptane/EtOAc); ¹H NMR (400 MHz, CDCl₃) δ 7.70 (d, *J* = 7.8 Hz, 1H), 7.63 (d, *J* = 8.2 Hz, 2H), 7.24 (dd, *J* = 7.8, 1.4 Hz, 1H), 7.09 - 6.94 (m, 3H), 4.15 (t, *J* = 6.5 Hz, 2H), 3.92 (s, 2H), 3.43 (s, 4H), 3.05 (t, *J* = 6.5 Hz, 2H), 1.46 (s, 18H); ¹³C NMR (101 MHz, CDCl₃) δ 170.48, 156.56, 137.64, 137.58, 133.79, 131.16, 130.32, 124.93, 119.01, 113.73, 111.16, 91.93, 81.02, 68.71, 55.78, 51.87, 35.11, 28.21.

### 1.21 Di-tert-butyl 2,2'-((2-((4-fluorobenzyl)oxy)-4-(1,2,4,5-tetrazin-3-yl)benzyl)azanediyl) diacetate (23)

The compound was obtained following the reported procedure.³² CH₂Cl₂ (0.08 mL, 1.24 mmol), sulfur (0.08 g, 0.31 mmol), hydrazine monohydrate (0.48 mL, 9.90 mmol) and ethanol (3.0 mL) along with **19** (0.6 g, 1.24 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNO₂ (0.85 g, 12.38 mmol) in water (10 ml) were added to the now yellow mixture followed by dropwise addition of acetic acid (4 mL), producing a mixture red in color. The reaction mixture was extracted with CH₂Cl₂, washed with brine, dried with MgSO₄ and filtered before concentrating *in vacuo.* The crude was purified using flash chromatography (90/10 n-Heptane/EtOAc) to yield 0.15 g (22%) of **23** as a red oil. Rf = 0.40 (80/20 n-Heptane/EtOAc); ¹H NMR (400 MHz, CDCl₃) δ 10.18 (s, 1H), 8.27 (dd, *J* = 7.9, 1.6 Hz, 1H), 8.16 (d, *J* = 1.6 Hz, 1H), 7.83 (d, *J* = 7.9 Hz, 1H), 7.50 - 7.39 (m, 2H), 7.07 (t, *J* = 8.7 Hz, 2H), 5.18 (s, 2H), 4.08 (s, 2H), 3.49 (s, 4H), 1.44 (s, 18H), ¹³C NMR (101 MHz, CDCl₃) δ 170.61, 166.30, 162.51 (d, *J* = 246.2 Hz), 157.74, 157.27, 133.76, 132.41 (d, *J* = 3.3 Hz), 131.22, 131.06, 129.23 (d, *J* = 8.3 Hz), 121.37, 115.50 (d, *J* = 21.5 Hz), 110.67, 81.01, 69.65, 55.77, 51.93, 28.16.

### 1.22 Di-tert-butyl 2,2'-((2-((4-iodobenzyl)oxy)-4-(1,2,4,5-tetrazin-3-yl)benzyl)azanediyl) diacetate (24)

The compound was obtained following the reported procedure.³² CH₂Cl₂ (0.14 mL, 2.19 mmol), sulfur (0.14 g, 0.55 mmol), hydrazine monohydrate (0.85 mL, 17.55 mmol) and ethanol (4.0 mL) along with **20** (1.3 g, 2.19 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNO₂ (1.51 g, 21.94 mmol) in water (10 ml) were added to the now yellow mixture followed by dropwise addition of acetic acid (7 mL), producing a mixture red in color. The reaction mixture was extracted with CH₂Cl₂, washed with brine, dried with MgSO₄ and filtered before concentrating *in vacuo.* The crude was purified using flash chromatography (90/10 n-Heptane/EtOAc) to yield 0.47 g (33%) of **24** as a red oil. Rf = 0.44 (80/20 n-Heptane/EtOAc); ¹H NMR (600 MHz, CDCl₃) δ 10.17 (s, 1H), 8.26 (dd, *J* = 7.9, 1.6 Hz, 1H), 8.12 (d, *J* = 1.6 Hz, 1H), 7.82 (d, *J* = 7.9 Hz, 1H), 7.72 - 7.67 (m, 2H), 7.23 (d, *J* = 8.1 Hz, 2H), 5.15 (s, 2H), 4.07 (s, 2H), 3.49 (s, 4H), 1.43 (s, 18H); ¹³C NMR (151 MHz, CDCl₃) δ 170.59, 166.24, 157.75, 157.74, 157.16, 137.69, 136.34, 133.71, 131.25, 131.12, 129.23, 121.41, 110.59, 93.53, 81.01, 69.58, 55.75, 51.92, 28.18.

### 1.23 Di-tert-butyl 2,2'-((2-(4-fluorophenethoxy)-4-(1,2,4,5-tetrazin-3-yl)benzyl)azanediyl) diacetate (25)

The compound was obtained following the reported procedure.³² CH₂Cl₂ (0.05 mL, 0.86 mmol), sulfur (0.05 g, 0.21 mmol), hydrazine monohydrate (0.33 mL, 6.90 mmol) and ethanol (4.0 mL) along with **21** (0.43 g, 0.86 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNO₂ (0.59 g, 8.62 mmol) in water (10 ml) were added to the now yellow mixture followed by dropwise addition of acetic acid (4 mL), producing a mixture red in color. The reaction mixture was extracted with CH₂Cl₂, washed with brine, dried with MgSO₄ and filtered before concentrating *in vacuo.* The crude was purified using flash chromatography (90/10 n-Heptane/EtOAc) to yield 0.13 g (27%) of **25** as a red oil. Rf = 0.33 (80/20 n-Heptane/EtOAc); ¹H NMR (400 MHz, CDCl₃) δ 10.09 (s, 1H), 8.16 (dd, *J* = 7.9, 1.6 Hz, 1H), 7.98 (d, *J* = 1.6 Hz, 1H), 7.71 (d, *J* = 7.9 Hz, 1H), 7.24 - 7.15 (m, 2H), 7.00 - 6.87 (m, 2H), 4.22 (t, *J* = 6.7 Hz, 2H), 3.93 (s, 2H), 3.41 (s, 4H), 3.06 (t, *J* = 6.6 Hz, 2H), 1.39 (s, 18H); ¹³C NMR (101 MHz, CDCl₃) δ 170.65, 166.32, 161.72 (d, *J* = 244.5 Hz), 157.71, 157.43, 133.89 (d, *J* = 3.4 Hz), 133.56, 131.10, 130.75, 130.55 (d, *J* = 7.8 Hz), 121.04, 115.30 (d, *J* = 21.3 Hz), 110.05, 80.95, 69.04, 55.77, 51.99, 34.96, 28.19.

### 1.24 Di-tert-butyl 2,2'-((2-(4-iodophenethoxy)-4-(1,2,4,5-tetrazin-3-yl)benzyl)azanediyl) diacetate (26)

The compound was obtained following the reported procedure.³² CH₂Cl₂ (0.07 mL, 1.15 mmol), sulfur (0.07 g, 0.29 mmol), hydrazine monohydrate (0.45 mL, 9.23 mmol) and ethanol (4.0 mL) along with **23** (0.71 g, 1.15 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNO₂ (0.79 g, 11.54 mmol) in water (15 ml) were added to the now yellow mixture followed by dropwise addition of acetic acid (7 mL), producing a mixture red in color. The reaction mixture was extracted with CH₂Cl₂, washed with brine, dried with MgSO₄ and filtered before concentrating *in vacuo.* The crude was purified using flash chromatography (90/10 n-Heptane/EtOAc) to yield 0.33 g (24%) of **26** as a red oil. Rf = 0.29 (80/20 n-Heptane/EtOAc); ¹H NMR (400 MHz, CDCl₃) δ 10.16 (s, 1H), 8.22 (dd, *J* = 7.9, 1.6 Hz, 1H), 8.03 (d, *J* = 1.7 Hz, 1H), 7.78 (d, *J* = 7.9 Hz, 1H), 7.69 - 7.55 (m, 2H), 7.14 - 7.00 (m, 2H), 4.30 (t, *J* = 6.6 Hz, 2H), 4.00 (s, 2H), 3.49 (s, 4H), 3.09 (t, *J* = 6.6 Hz, 2H), 1.46 (s, 18H); ¹³C NMR (101 MHz, CDCl₃) δ 170.59, 166.29, 157.72, 157.38, 137.95, 137.57, 133.47, 131.20, 131.14, 130.81, 121.09, 110.05, 91.80, 81.00, 68.70, 55.75, 52.00, 35.28, 28.22.

### 1.25 2,2'-((2-((4-Fluorobenzyl)oxy)-4-(1,2,4,5-tetrazin-3-yl)benzyl)azanediyl)diacetic acid (27)

To a solution of **23** (0.08 g, 0.15 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.06 g (70%) of **27** (TFA salt) as a pink solid. ¹H NMR (600 MHz, MeOD) δ 10.28 (s, 1H), 8.26 (d, *J* = 1.5 Hz, 1H), 8.19 (dd, *J* = 7.9, 1.5 Hz, 1H), 7.66 (d, *J* = 7.9 Hz, 1H), 7.55 - 7.48 (m, 2H), 7.10 - 6.98 (m, 2H), 5.27 (s, 2H), 4.57 (s, 2H), 4.03 (s, 4H); ¹³C NMR (151 MHz, MeOD) δ 167.81, 165.69, 162.81 (d, *J* = 245.2 Hz), 158.13, 135.53, 133.96, 131.97 (d, *J* = 3.3Hz), 130.00(d, *J* = 8.2 Hz), 122.92, 120.74, 115.13(d, *J* = 21.9Hz), 111.53, 70.03, 54.10, 53.82.

### 1.26 2,2'-((2-((4-Iodobenzyl)oxy)-4-(1,2,4,5-tetrazin-3-yl)benzyl)azanediyl)diacetic acid (28)

To a solution of **24** (0.05 g, 0.08 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.04 g (70%) of **28** (TFA salt) as a pink solid. ¹H NMR (600 MHz, MeOD) δ 10.38 (s, 1H), 8.33 (d, *J* = 1.6 Hz, 1H), 8.30 (dd, *J* = 7.8, 1.5 Hz, 1H), 7.77 (d, *J* = 7.9 Hz, 3H), 7.37 (d, *J* = 8.0 Hz, 2H), 5.36 (s, 2H), 4.69 (s, 2H), 4.15 (s, 4H); ¹³C NMR (151 MHz, MeOD) δ 167.83, 165.64, 158.12, 158.05, 137.65, 135.76, 135.51, 133.98, 129.66, 122.96, 120.81, 111.49, 93.29, 70.03, 53.75.

### 1.27 2,2'-((2-(4-Fluorophenethoxy)-4-(1,2,4,5-tetrazin-3-yl)benzyl)azanediyl)diacetic acid (29)

To a solution of **25** (0.07 g, 0.12 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.06 g (92%) of **29** (TFA salt) as a pinksolid. ¹H NMR (400 MHz, MeOD) δ 10.38 (s, 1H), 8.30 - 8.19 (m, 2H), 7.74 (d, *J* = 7.8 Hz, 1H), 7.40 (dd, *J* = 8.4, 5.4 Hz, 2H), 7.05 (t, *J* = 8.6 Hz, 2H), 4.68 (s, 2H), 4.48 (t, *J* = 6.8 Hz, 2H), 4.17 (s, 4H), 3.25 (t, *J* = 6.6 Hz, 2H); ¹³C NMR (101 MHz, MeOD) δ 167.35, 165.63, 161.79 (d, *J* = 243.3 Hz), 158.37, 158.13, 135.76, 134.15, 133.90 (d, *J* = 3.1 Hz), 130.42 (d, *J* = 7.9 Hz), 121.68, 120.55, 114.90 (d, *J* = 21.4 Hz), 110.94, 69.67, 53.74, 53.37, 33.96.

### 1.28 2,2'-((2-(4-Iodophenethoxy)-4-(1,2,4,5-tetrazin-3-yl)benzyl)azanediyl)diacetic acid (30)

To a solution of **27** (0.05 g, 0.08 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.04 g (80%) of **30** (TFA salt) as a pink solid. ¹H NMR (600 MHz, DMSO) δ 10.59 (s, 1H), 8.12 (dd, *J* = 7.8, 1.6 Hz, 1H), 8.03 (d, *J* = 1.7 Hz, 1H), 7.72 (d, *J* = 7.9 Hz, 1H), 7.69 - 7.63 (m, 2H), 7.23 - 7.16 (m, 1H), 4.33 (s, 2H), 4.03 (s, 2H), 3.59 (s, 4H), 3.07 (s, 2H); ¹³C NMR (151 MHz, DMSO) δ 172.02, 165.75, 158.66, 158.58, 158.35, 157.72, 138.83, 137.50, 132.56, 132.07, 120.68, 110.60, 92.59, 69.13, 54.51, 51.93, 34.79.

### 1.29 Di-tert-butyl 2,2'-((4-(1,2,4,5-tetrazin-3-yl)-2-((4-(trimethylstannyl)benzyl)oxy)benzyl) azanediyl)diacetate (31)

Palladium tetrakis (0.01 g, 0.009 mmol, 10%), hexamethylditin (0.05 mL, 0.24 mmol,) and dry THF (2 mL) were successively added to a microwave vial equipped with a stir bar which was then sealed and purged with N₂.⁴¹ A solution of **24** (0.06 g, 0.09 mmol) in dry THF (2 mL) was added via a syringe and the reaction allowed to stir at 65 °C in a microwave for 30 minutes. The reaction was allowed to cool to room temperature and unsealed before being quenched with saturated aqueous KF (1 mL). The solution was extracted with CH₂Cl₂ (3 × 5 mL), washed with brine (2 × 10 mL), dried over MgSO₄, filtered and concentrated under reduced pressure. The crude was purified using flash chromatography (90/10 n-Heptane/EtOAc) to yield 0.40 g (62%) of a pink solid. Rf = 0.46 (80/20 n-Heptane/EtOAc); ¹H NMR (400 MHz, CDCl₃) δ 10.19 (s, 1H), 8.27 (dd, *J* = 7.9, 1.5 Hz, 1H), 8.19 (d, *J* = 1.6 Hz, 1H), 7.86 (d, *J* = 7.9 Hz, 1H), 7.54 - 7.49 (m, 2H), 7.46 (d, *J* = 7.8 Hz, 2H), 5.21 (s, 2H), 4.11 (s, 2H), 3.52 (s, 4H), 1.44 (s, 18H), 0.29 (s, 9H); ¹³C NMR (101 MHz, CDCl₃) δ 166.38, 157.72, 157.45, 142.11, 136.55, 136.04, 135.23, 130.95, 130.23, 127.64, 127.07, 121.26, 110.58, 81.03, 70.28, 55.81, 52.00, 28.18, -9.59.

### 1.30 Di-tert-butyl 2,2'-((4-(1,2,4,5-tetrazin-3-yl)-2-(4-(trimethylstannyl)phenethoxy)benzyl) azanediyl)diacetate (32)

Palladium tetrakis (0.01 g, 0.009 mmol, 10%), hexamethylditin (0.05 mL, 0.24 mmol,) and dry THF (2 mL) were successively added to a microwave vial equipped with a stir bar which was then sealed and purged with N₂.⁴¹ A solution of **24** (0.05 g, 0.08 mmol) in dry THF (2 mL) was added via a syringe and the reaction allowed to stir at 65 °C in a microwave for 30 minutes. The reaction was allowed to cool to room temperature and unsealed before being quenched with saturated aqueous KF (1 mL). The solution was extracted with CH₂Cl₂ (3 × 5 mL), washed with brine (2 × 10 mL), dried over MgSO₄, filtered and concentrated under reduced pressure. The crude was purified using flash chromatography (90/10 n-Heptane/EtOAc) to yield 0.25 g (48%) of a pink solid. Rf = 0.39 (80/20 n-Heptane/EtOAc); ¹H NMR (600 MHz, CDCl₃) δ 10.10 (s, 1H), 8.16 (dd, *J* = 7.9, 1.6 Hz, 1H), 7.99 (d, *J* = 1.6 Hz, 1H), 7.73 (d, *J* = 7.9 Hz, 1H), 7.45 - 7.33 (m, 2H), 7.26 - 7.20 (m, 2H), 4.24 (t, *J* = 7.0 Hz, 2H), 3.97 (s, 2H), 3.43 (s, 4H), 3.08 (t, *J* = 7.0 Hz, 2H), 1.40 (s, 18H), 0.21 (s, 9H); ¹³C NMR (151 MHz, CDCl₃) δ 170.70, 166.38, 157.71, 157.54, 140.06, 138.10, 136.07, 133.58, 131.08, 130.74, 128.89, 120.97, 110.10, 80.96, 69.26, 55.83, 52.00, 35.76, 28.22, -9.58.

### 1.31 Tert-butyl 5-fluoro-2-methylbenzoate (33)

5-Fluoro-2-methylbenzoic acid (1.00 g, 6.54 mmol) was dissolved in t-BuOH (9 mL) and THF (3 mL). Boc anhydride (2.90 g, 13.27 mmol) was added followed by DMAP (0.24 g, 1.99 mmol). The mixture was stirred at room temperature under N₂ for 12 h. The solvents were removed under reduced pressure. The residue was dissolved in EtOAc (50 mL) and washed with saturated aqueous NaHCO₃ (2 × 30 mL) and brine (2 × 30 mL). The organic phase was dried over MgSO₄, filtered and concentrated under reduced pressure to give 0.95 g (69%) of the desired product as colorless oil. Rf = 0.41 (90/10 n-Heptane/EtOAc); ¹H NMR (400 MHz, CDCl₃) δ 7.51 (dd, *J* = 9.5, 2.9 Hz, 1H), 7.16 (dd, *J* = 8.4, 5.6 Hz, 1H), 7.05 (td, *J* = 8.2, 2.8 Hz, 1H), 2.52 (s, 3H), 1.59 (s, 9H); ¹³C NMR (101 MHz, CDCl₃) δ 165.96 (d, *J* = 2.7 Hz), 160.60 (d, *J* = 244.0 Hz), 135.01 (d, *J* = 3.4 Hz), 133.01 (d, *J* = 6.7 Hz), 132.89 (d, *J* = 7.3 Hz), 118.21 (d, *J* = 20.8 Hz), 116.93 (d, *J* = 22.8 Hz), 81.60, 28.21, 20.92.

### 1.32 Tert-butyl 5-iodo-2-methylbenzoate (34)

5-lodo-2-methylbenzoic acid (1.71 g, 6.54 mmol) was dissolved in t-BuOH (9 mL) and THF (3 mL). Boc anhydride (2.90 g, 13.27 mmol) was added followed by DMAP (0.24 g, 1.99 mmol). The mixture was stirred at room temperature under N₂ for 12 h. The solvents were removed under reduced pressure. The residue was dissolved in EtOAc (50 mL) and washed with saturated aqueous NaHCOs (2 × 30 mL) and brine (2 × 30 mL). The organic phase was dried over MgSO₄, filtered and concentrated under reduced pressure to give 1.42 g (68%) of the desired product as colorless oil. Rf = 0.42 (90/10 n-Heptane/EtOAc); ¹H NMR (600 MHz, CDCl₃) δ 8.10 (d, *J* = 2.0 Hz, 1H), 7.65 (dd, *J* = 8.1, 2.0 Hz, 1H), 6.95 (dd, *J* = 8.1, 0.9 Hz, 1H), 2.50 (s, 3H), 1.59 (s, 9H); ¹³C NMR (151 MHz, CDCl₃) δ 165.67, 140.06, 138.85, 138.81, 133.74, 133.35, 89.93, 81.76, 28.23, 21.27.

### 1.33 Tert-butyl 2-(bromomethyl)-5-fluorobenzoate (35)

To a solution of **33** (0.91 g, 4.33 mmol) and N-bromo succinimide (0.85 g, 4.76 mmol) in CH₃CN (50 mL) was added AIBN (0.28 g, 1.73 mmol). The resulting solution was refluxed for 12 h. The reaction was cooled down and the organic layer was washed with water (2 × 20 mL) and brine (2 × 20 mL), dried over anhydrous Na₂SO₄, filtered and concentrated under reduced pressure. Purification by flash chromatography (90/10 n-Heptane/EtOAc) afforded 0.91 g (73%) of **35** as a white solid. Rf = 0.39 (90/10 n-Heptane/EtOAc); ¹H NMR (400 MHz, CDCl₃) δ 7.57 (dd, *J* = 9.3, 2.8 Hz, 1H), 7.40 (ddd, *J* = 8.3, 5.3, 2.1 Hz, 1H), 7.13 (td, *J* = 8.1, 2.6 Hz, 1H), 4.89 (s, 2H), 1.63 (s, 9H); ¹³C NMR (101 MHz, CDCl₃) δ 164.82, 162.03 (d, *J* = 249.2 Hz), 134.53 (d, *J* = 3.5 Hz), 133.36 (d, *J* = 7.2 Hz), 133.33 (d, *J* = 7.8 Hz), 118.82 (d, *J* = 21.5 Hz), 118.13 (d, *J* = 23.5 Hz), 82.70, 30.78, 28.10.

### 1.34 Tert-butyl 2-(bromomethyl)-5-iodobenzoate (36)

To a solution of **34** (1.11 g, 3.45 mmol) and N-bromo succinimide (0.68 g, 3.80 mmol) in CH₃CN (50 mL) was added AIBN (0.23 g, 1.38 mmol). The resulting solution was refluxed for 12 h. The reaction was cooled down and the organic layer was washed with water (2 × 20 mL) and brine (2 × 20 mL), dried over anhydrous Na₂SO₄, filtered and concentrated under reduced pressure. Purification by flash chromatography (90/10 n-Heptane/EtOAc) afforded 0.91 g (79%) of **36** as a white solid. Rf = 0.40 (90/10 n-Heptane/EtOAc); ¹H NMR (400 MHz, CDCl₃) δ 8.17 (d, *J* = 1.9 Hz, 1H), 7.76 (dd, *J* = 8.1, 2.0 Hz, 1H), 7.14 (d, *J* = 8.1 Hz, 1H), 4.84 (s, 2H), 1.63 (s, 9H); ¹³C NMR (101 MHz, CDCl₃) δ 164.59, 140.74, 139.81, 138.04, 133.14, 132.95, 93.71, 82.81, 30.79, 28.14.

### 1.35 Di-tert-butyl 2,2'-((2-((2-(tert-butoxycarbonyl)-4-fluorobenzyl)oxy)-4-cyanobenzyl) azanediyl)diacetate (37)

To a solution of **18** (0.40 g, 1.06 mmol) in in CH₃CN (40 mL) was added **35** (0.34 g, 1.16 mmol), K₂CO₃ (0.44 g, 3.19 mmol). The resulting suspension was refluxed for 12 hours. The mixture was cooled to room temperature, filtered and concentrated under reduced pressure. The crude was directly purified by flash chromatography (90/10 n-Heptane/EtOAc) to give 0.5 g (80%) of the desire product as a colorless oil (pure enough for the next step). Rf = 0.43 (80/20 n-Heptane/EtOAc); ¹H NMR (400 MHz, CDCl₃) δ 7.68 (d, *J* = 7.8 Hz, 1H), 7.65 - 7.55 (m, 2H), 7.21 (dd, *J* = 7.6, 1.5 Hz, 1H), 7.15 (td, *J* = 8.2, 2.8 Hz, 1H), 7.04 (d, *J* = 1.5 Hz, 1H), 5.38 (s, 2H), 3.98 (s, 2H), 3.38 (s, 4H), 1.52 (s, 9H), 1.35 (s, 18H); ¹³C NMR (101 MHz, CDCl₃) δ 170.46, 164.75 (d, *J* = 2.5 Hz), 161.63 (d, *J* = 247.1 Hz), 156.47, 133.98 (d, *J* = 3.3 Hz), 133.75, 131.39 (d, *J* = 7.0 Hz), 130.59, 129.13 (d, *J* = 7.7 Hz), 125.13, 119.19, 118.98, 117.71 (d, *J* = 23.5 Hz), 114.43, 111.42, 82.33, 81.03, 68.18, 55.72, 51.87, 28.14, 28.12.

### 1.36 Di-tert-butyl 2,2'-((2-((2-(tert-butoxycarbonyl)-4-iodobenzyl)oxy)-4-cyanobenzyl) azanediyl)diacetate (38)

To a solution of **18** (0.53 g, 1.41 mmol) in in CH₃CN (40 mL) was added **36** (0.61 g, 1.55 mmol), K₂CO₃ (0.58 g, 4.22 mmol). The resulting suspension was refluxed for 12 hours. The mixture was cooled to room temperature, filtered and concentrated under reduced pressure. The crude was directly purified by flash chromatography (90/10 n-Heptane/EtOAc) to give 0.63 g (65%) of the desire product as a colorless oil (pure enough for the next step). Rf = 0.33 (80/20 n-Heptane/EtOAc); ¹H NMR (400 MHz, CDCl₃) δ 8.47 (d, *J* = 1.9 Hz, 1H), 8.05 (dd, *J* = 8.3, 1.9 Hz, 1H), 7.97 (d, *J* = 7.8 Hz, 1H), 7.66 (d, *J* = 8.3 Hz, 1H), 7.49 (dd, *J* = 7.8, 1.4 Hz, 1H), 7.31 (d, *J* = 1.5 Hz, 1H), 5.64 (s, 2H), 4.27 (s, 2H), 3.67 (s, 4H), 1.81 (s, 9H), 1.64 (s, 18H); ¹³C NMR (101 MHz, CDCl₃) δ 170.42, 164.47, 156.38, 141.10, 139.44, 137.89, 133.74, 131.26, 130.66, 128.93, 125.18, 118.91, 114.41, 111.45, 92.54, 82.45, 81.02, 68.24, 55.70, 51.87, 28.17, 28.14.

### 1.37 Di-tert-butyl 2,2'-((2-((2-(tert-butoxycarbonyl)-4-fluorobenzyl)oxy)-4-(1,2,4,5-tetrazin-3-yl)benzyl)azanediyl)diacetate (39)

The compound was obtained following the reported procedure.³² CH₂Cl₂ (0.05 mL, 0.86 mmol), sulfur (0.05 g, 0.21 mmol), hydrazine monohydrate (0.33 mL, 6.90 mmol) and ethanol (4.0 mL) along with **37** (0.50 g, 0.86 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNOz (0.59 g, 8.62 mmol) in water (10 ml) were added to the now yellow mixture followed by dropwise addition of acetic acid (4 mL), producing a mixture red in color. The reaction mixture was extracted with CH₂Cl₂, washed with brine, dried with MgSO₄ and filtered before concentrating *in vacuo.* The crude was purified using flash chromatography (90/10 n-Heptane/EtOAc) to yield 0.14 g (26%) of **39** as a red oil. Rf = 0.26 (80/20 n-Heptane/EtOAc); ¹H NMR (400 MHz, CDCl₃) δ 10.17 (s, 1H), 8.27 (dd, *J* = 7.9, 1.6 Hz, 1H), 8.17 (d, *J* = 1.7 Hz, 1H), 7.85 (d, *J* = 7.9 Hz, 1H), 7.79 (dd, *J* = 8.7, 5.5 Hz, 1H), 7.67 (dd, *J* = 9.4, 2.8 Hz, 1H), 7.23 (td, *J* = 8.2, 2.8 Hz, 1H), 5.57 (s, 2H), 4.14 (s, 2H), 3.51 (s, 4H), 1.61 (s, 9H), 1.44 (s, 18H); ¹³C NMR (101 MHz, CDCl₃) δ 170.56, 166.37, 164.92 (d, *J* = 2.6 Hz), 161.64 (d, *J* = 246.7 Hz), 157.72, 157.34, 134.30 (d, *J* = 3.2 Hz), 133.45, 131.74 (d, *J* = 7.0 Hz), 131.44, 131.03, 129.51 (d, *J* = 7.6 Hz), 121.34, 118.97 (d, *J* = 21.0 Hz), 117.65 (d, *J* = 23.3 Hz), 110.65, 82.39, 81.03, 68.14, 55.70, 52.06, 28.20, 28.16.

### 1.38 Di-tert-butyl 2,2'-((2-((2-(tert-butoxycarbonyl)-4-iodobenzyl)oxy)-4-(1,2,4,5-tetrazin-3-yl)benzyl)azanediyl)diacetate (40)

The compound was obtained following the reported procedure.³² CH₂Cl₂ (0.06 mL, 0.91 mmol), sulfur (0.06 g, 0.23 mmol), hydrazine monohydrate (0.35 mL, 7.28 mmol) and ethanol (4.0 mL) along with **38** (0.63 g, 0.91 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNOz (0.63 g, 9.01 mmol) in water (10 ml) were added to the now yellow mixture followed by dropwise addition of acetic acid (5 mL), producing a mixture red in color. The reaction mixture was extracted with CH₂Cl₂, washed with brine, dried with MgSO₄ and filtered before concentrating *in vacuo.* The crude was purified using flash chromatography (90/10 n-Heptane/EtOAc) to yield 0.19 g (28%) of **40** as a red oil. Rf = 0.31 (80/20 n-Heptane/EtOAc); ¹H NMR (400 MHz, CDCl₃) δ 10.18 (s, 1H), 8.31 - 8.24 (m, 2H), 8.15 (d, *J* = 1.6 Hz, 1H), 7.90 - 7.80 (m, 2H), 7.55 (d, *J* = 8.2 Hz, 1H), 5.55 (s, 2H), 4.13 (s, 2H), 3.51 (s, 4H), 1.61 (s, 9H), 1.44 (s, 18H); ¹³C NMR (101 MHz, CDCl₃) δ 170.60, 169.97, 166.36, 164.66, 157.73, 157.24, 141.01, 139.42, 138.25, 131.62, 131.43, 131.07, 129.29, 121.40, 110.64, 92.39, 82.53, 81.03, 68.18, 55.71, 52.06, 28.22, 28.18.

### 1.39 2,2'-((2-((2-Carboxy-4-iodobenzyl)oxy)-4-(1,2,4,5-tetrazin-3-yl)benzyl)azanediyl) diacetic acid (41)

To a solution of **39** (0.1 g, 0.15 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.07 g (76%) of **41** (TFA salt) as a pink solid. ¹H NMR (600 MHz, DMSO) δ 10.58 (s, 1H), 8.16 (dd, *J* = 7.8, 1.5 Hz, 1H), 8.06 (d, *J* = 1.6 Hz, 1H), 7.85 - 7.81 (m, 1H), 7.81 - 7.77 (m, 1H), 7.71 (dd, *J* = 9.4, 2.9 Hz, 1H), 7.48 (td, *J* = 8.4, 2.9 Hz, 1H), 5.60 (s, 2H), 4.07 (s, 2H), 3.53 (s, 4H); ¹³C NMR (151 MHz, DMSO) δ 172.72, 167.40, 167.39, 165.74, 161.50 (d, *J* = 244.8 Hz), 158.61, 157.39, 134.84 (d, *J* = 3.1 Hz), 132.26, 131.84 (d, *J* = 7.0 Hz), 131.29, 130.63 (d, *J* = 7.8 Hz), 120.96, 119.55 (d, *J* = 20.9 Hz), 117.53 (d, *J* = 23.0 Hz), 110.78, 67.95, 54.63, 52.17.

### 1.40 2,2'-((2-((2-Carboxy-4-iodobenzyl)oxy)-4-(1,2,4,5-tetrazin-3-yl)benzyl)azanediyl) diacetic acid (42)

To a solution of **40** (0.03 g, 0.04 mmol) in CH₂Cl₂ (5 mL) was added trifluoroacetic acid (5 mL). The reaction was stirred at room temperature for 2 hours. The solvent was then removed under reduced pressure to obtain a pink solid. NMR of the crude shows full conversion. Purification by preparative HPLC afforded 0.015 g (64%) of **42** (TFA salt) as a pink solid. ¹H NMR (600 MHz, DMSO) δ 10.59 (s, 1H), 8.25 (d, *J* = 1.9 Hz, 1H), 8.17 (dd, *J* = 7.8, 1.6 Hz, 1H), 8.04 (d, *J* = 1.6 Hz, 1H), 7.98 (dd, *J* = 8.2, 1,9 Hz, 1H), 7.80 (d, *J* = 7.8 Hz, 1H), 7.58 (d, *J* = 8.2 Hz, 1H), 5.60 (s, 2H), 4.16 (s, 2H), 3.62 (s, 4H); ¹³C NMR (151 MHz, DMSO) δ 172.11, 167.13, 165.67, 158.62, 157.44, 141.33, 139.19, 138.46, 132.73, 131.97, 131.44, 130.17, 120.99, 110.85, 93.82, 68.10, 54.59, 52.36.

### 1.41 Di-tert-butyl 2,2'-((2-((2-(isopropoxycarbonyl)-4-(trimethylstannyl)benzyl)oxy)-4-(1,2,4,5-tetrazin-3-yl)benzyl)azanediyl)diacetate (43)

Palladium tetrakis (0.09 g, 0.007 mmol, 10%), hexamethylditin (0.05 mL, 0.24 mmol,) and dry THF (2 mL) were successively added to a microwave vial equipped with a stir bar which was then sealed and purged with N₂.⁴¹ A solution of **39** (0.05 g, 0.07 mmol) in dry THF (2 mL) was added via a syringe and the reaction allowed to stir at 65 °C in a microwave for 30 minutes. The reaction was allowed to cool to room temperature and unsealed before being quenched with saturated aqueous KF (1 mL). The solution was extracted with CH₂Cl₂ (3 × 5 mL), washed with brine (2 × 10 mL), dried over MgSO₄, filtered and concentrated under reduced pressure. The crude was purified using flash chromatography (90/10 n-Heptane/EtOAc) to yield 0.25 g (48%) of a pink solid. Rf = 0.24 (80/20 n-Heptane/EtOAc); ¹H NMR (600 MHz, CDCl₃) δ 10.17 (s, 1H), 8.32 - 8.24 (m, 1H), 8.23 - 8.17 (m, 1H), 8.08 (s, 1H), 7.88 (d, *J* = 7.9 Hz, 1H), 7.72 (d, *J* = 7.4 Hz, 1H), 7.65 (d, *J* = 7.5 Hz, 1H), 5.58 (s, 2H), 4.14 (s, 2H), 3.52 (s, 4H), 1.61 (s, 9H), 1.44 (s, 18H), 0.32 (s, 9H); 13C NMR (101 MHz, CDCl₃) δ 170.60, 169.98, 166.75, 166.45, 157.72, 157.46, 141.53, 139.60, 138.25, 137.88, 131.30, 130.83, 129.40, 126.85 121.22, 110.49, 81.83, 80.99, 68.68, 55.82, 52.05, 28.30, 28.18, -9.54.

### 1.42 (4-(Bromomethyl)phenyl)trimethylsilane (44)

The compound was synthesized according to the literature.⁴² To a mixture of N-bromosuccinimide (1.09 g, 6.11 mmol), 2, 2'-azobisisobutyronitrile (0.015 g, 0.09 mmol), and tetrachloromethane (10.0 mL), a tetrachloromethane solution (3.0mL) of the trimethyl(p-tolyl)silane was added with a syringe at room temperature. The mixture was stirred for 8 h at 80°C. The reaction was cooled to room temperature and the reacrion quenched with water (30 mL). The organic phase was separated, washed with brine (2 × 20 mL), dried over MgSO₄, filtered and concentrated under reduced pressure to give 1.22 g (82%) the desired compound as yellow liquid. Rf = 0.63 (n-Hexane); ¹H NMR (400 MHz, CDCl₃) δ 7.39 (d, *J* = 8.1 Hz, 2H), 7.26 (d, *J* = 8.0 Hz, 2H), 4.37 (s, 2H), 0.15 (s, 9H); ¹³C NMR (101 MHz, CDCl₃) δ 142.31, 139.35, 135.01, 129.45, 34.74, 0.00.

### 1.43 Di-tert-butyl 2,2'-((4-cyano-2-((4-(trimethylsilyl)benzyl)oxy)benzyl)azanediyl)diacetate (45)

To a solution of 18 (1.51 g, 3.98 mmol) in in CH₃CN (40 mL) was added **45** (1.26 g, 5.18 mmol), K₂CO₃ (1.65 g, 11.95 mmol). The resulting suspension was refluxed for 12 hours. The mixture was cooled to room temperature, filtered and concentrated under reduced pressure. The crude was directly purified by flash chromatography (90/10 n-Heptane/EtOAc) to give 1.89 g (88%) of the desire product as a white solid. Rf = 0.29 (90/10 n-Heptane/EtOAc); ¹H NMR (400 MHz, CDCl₃) δ 7.76 (d, *J* = 7.8 Hz, 1H), 7.57 - 7.50 (m, 2H), 7.38 (d, *J* = 7.7 Hz, 2H), 7.28 (dd, *J* = 7.7, 1.4 Hz, 1H), 7.10 (d, *J* = 1.5 Hz, 1H), 5.08 (s, 2H), 4.02 (s, 2H), 3.45 (s, 4H), 1.43 (s, 18H), 0.28 (s, 9H); ¹³C NMR (101 MHz, CDCl₃) δ 171.64, 157.77, 141.79, 137.63, 134.87, 131.74, 127.65, 126.29, 120.26, 115.58, 112.42, 82.30, 71.51, 56.98, 52.93, 29.31.

### 1.44 Di-tert-butyl 2,2'-((4-(1,2,4,5-tetrazin-3-yl)-2-((4-(trimethylsilyl)benzyl)oxy)benzyl) azanediyl)diacetate (46)

The compound was obtained following the reported procedure.³² CH₂Cl₂ (0.23 mL, 3.62 mmol), sulfur (0.23 g, 0.90 mmol), hydrazine monohydrate (1.41 mL, 28.95 mmol) and ethanol (4.0 mL) along with **45** (1.95 g, 3.62 mmol) were added to a Biotage microwave vial (10-20 mL) equipped with a stir bar. The vessel was sealed, and the reaction mixture was heated to 50 °C for 24 hours, before being allowed to cool to room temperature and unsealed. Then 3 ml of CH₂Cl₂ and NaNOz (2.50 g, 36.19 mmol) in water (20 ml) were added to the now yellow mixture followed by dropwise addition of acetic acid (8 mL), producing a mixture red in color. The reaction mixture was extracted with CH₂Cl₂, washed with brine, dried with MgSO₄ and filtered before concentrating *in vacuo.* The crude was purified using flash chromatography (90/10 n-Heptane/EtOAc) to yield 0.45 g (21%) of **46** as a red oil. Rf = 0.30 (80/20 n-Heptane/EtOAc); ¹H NMR (400 MHz, CDCl₃) δ 10.18 (s, 1H), 8.28 (dd, *J* = 7.9, 1.6 Hz, 1H), 8.19 (d, *J* = 1.6 Hz, 1H), 7.86 (d, *J* = 7.9 Hz, 1H), 7.57 - 7.53 (m, 2H), 7.51 - 7.44 (m, 2H), 5.22 (s, 2H), 4.12 (s, 2H), 3.53 (s, 4H), 1.44 (s, 18H), 0.27 (s, 9H); ¹³C NMR (101 MHz, CDCl₃) δ 171.66, 167.50, 158.87, 158.60, 141.42, 138.24, 134.73, 132.40, 132.15, 127.88, 127.62, 122.42, 111.71, 82.23, 71.40, 56.92, 53.12, 29.31, 0.00.

### 1.45 2,2'-((4-(1,2,4,5-Tetrazin-3-yl)-2-((4-(trimethylsilyl)benzyl)oxy)benzyl)azanediyl)diacetic acid (47)

Compound 46 (0.05 g, 0.08 mmol) was solubilized in 4M HCl in dioxane (2 mL). The solution was stirred at room temperature for 4 hours. The volatiles were removed under reduced pressure and the residue purified by preparative HPLC to give 0.03 g (52%) of the desired compound as a red solid (TFA salt). ¹H NMR (600 MHz, CD₃CN) δ 10.29 (s, 1H), 8.23 - 8.15 (m, 2H), 7.65 (d, *J* = 7.8 Hz, 1H), 7.59 (d, *J* = 7.9 Hz, 2H), 7.53 (d, *J* = 7.7 Hz, 2H), 5.38 (s, 2H), 4.46 (s, 2H), 3.91 (s, 4H), 0.25 (s, 9H); ¹³C NMR (151 MHz, CD₃CN) δ 169.71, 166.32, 158.70, 158.55, 140.99, 137.51, 135.33, 134.24, 134.02, 127.51, 125.79, 117.90, 111.94, 70.96, 56.19, 55.40, -1.55.

### Blocking Assay and Ex Vivo Studies

### Establishing Tumor Xenografts in Mice

All animal studies were approved by the Danish Animal Welfare Council, Ministry of Justice. Five week old female nude BALB/c mice (Charles River, Sulzfeld, Germany) were allowed to acclimatize for 1 week. At all time, the animals had access to water and chow ad libitum. The human colon cancer cell line, LS174T (ATCC, VA, USA), was cultured in minimum essential medium (MEM) supplemented with 10% fetal bovine serum, 1% I-glutamine, 1% sodium pyruvate, 1% non-essential amino acids, and 1% penicillin-streptomycin. At a confluence of 70-90%, the cells were harvested by trypsinization, and subcutaneous tumors were established in the flank of the animals by inoculation of ~5 × 10⁶ LS174T cells (in 100 µL of sterile PBS). The tumors were allowed to grow for 7-10 days and were measured using a caliper. The tumor volume was estimated using the formula: volume = 1/2 (length × width²).

### Example 2 - Blocking Experiments

### 2.1 Blocking Assay

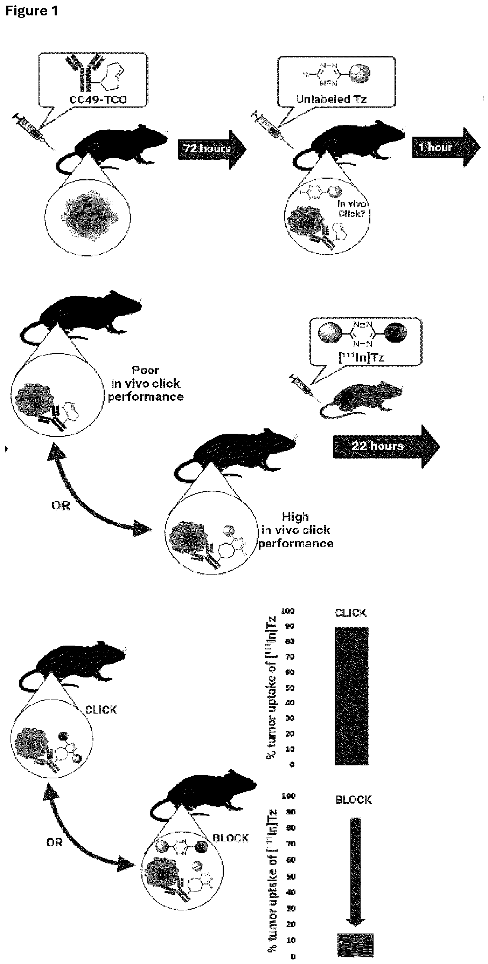

The cold tetrazines were screened using a blocking assay. This assay eliminates the need to radiolabel all tetrazines in order to evaluate their click efficacy in vivo. Moreover, iodine analogues can be tested as well in order to predict the in vivo behavior of the ²¹¹At radiolabeled tetrazines. This approach is based on the ability of tetrazines to block the binding of pretargeted imaging agent [¹¹¹In]**DOTA-Tz**, to the pretargeting vector CC49-TCO (administered 72 h prior) in tumor bearing mice as previously described in Stéen et al., ACS Pharmacology and Translational Science 2021, 824 - 833. The tumor blocking effect of the unlabeled tetrazine derivatives is then determined by *ex vivo* biodistribution and normalized to the binding of [¹¹¹In]**DOTA-Tz** without any blocking. A schematic representation of the experiment is shown in Figure 1.

### 2.2 Establishing Tumor Xenografts in Mice

All animal studies were approved by the Danish Animal Welfare Council, Ministry of Justice. Five week old female nude BALB/c mice (Charles River, Sulzfeld, Germany) were allowed to acclimatize for 1 week. At all time, the animals had access to water and chow ad libitum. The human colon cancer cell line, LS174T (ATCC, VA, USA), was cultured in minimum essential medium (MEM) supplemented with 10% fetal bovine serum, 1% I-glutamine, 1% sodium pyruvate, 1% non-essential amino acids, and 1% penicillin-streptomycin. At a confluence of 70-90%, the cells were harvested by trypsinization, and subcutaneous tumors were established in the flank of the animals by inoculation of ~5 × 10⁶ LS174T cells (in 100 µL of sterile PBS). The tumors were allowed to grow for 7-10 days and were measured using a caliper. The tumor volume was estimated using the formula: volume = 1/2 (length × width²).

### 2.3 Ex vivo blocking assay

Tumor bearing animals were matched in groups based on their tumor volume (tumor volumes of ~100-300 mm³ and *n* = 3 in each group) and were administered 100 µg/100 µL of CC49-TCO (~7 TCO/mAb) per mouse. After 3 days, animals were first injected with non-radioactive Tz (39 nmol), and after 1 h, they were administered with [111In]**DOTA-Tz** (3-5 MBq/100 µL and 3.9 nmol) *via* the tail vein. Tz [¹¹¹In]**DOTA-Tz** was radiolabeled as previously described. 22 h later, the mice were euthanized, and the tumor, blood, heart, lung, liver, spleen, kidney, and muscle tissue were resected and weighed, and the radioactivity was measured using a gamma counter (Wizard2, PerkinElmer). The data were corrected for decay and normalized to tissue mass. Tumor uptake of [¹¹¹In]**16** in the animals receiving the non-radiolabeled Tz was normalized to a control group of animals receiving [¹¹¹In]**DOTA-Tz** exclusively. The precursor of [¹¹¹In]**DOTA-Tz** was included as a positive control.

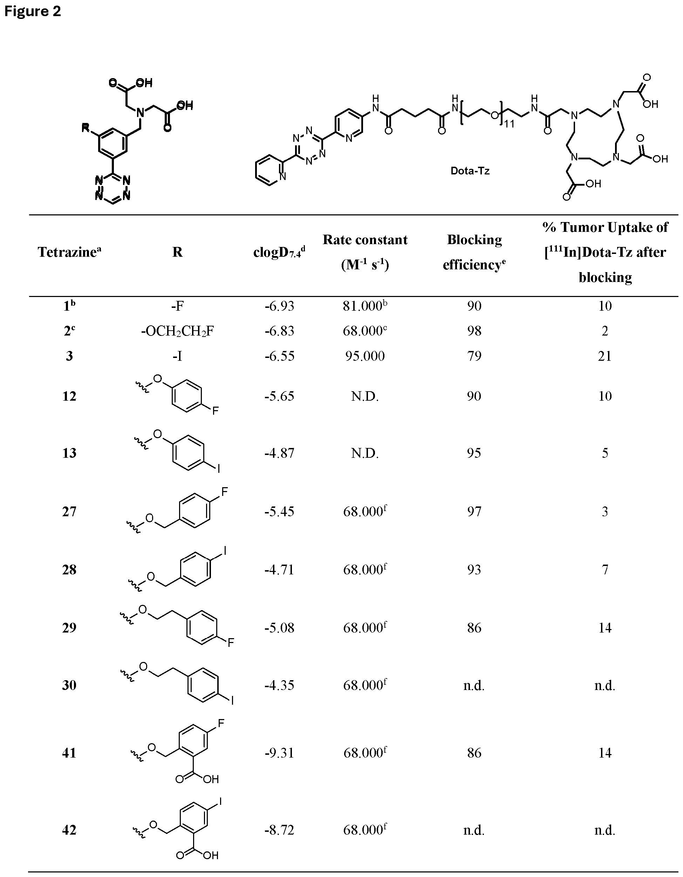

Figure 2. shows the structural scaffolds, calculated physicochemical properties (clogD7.4), and blocking efficiencies of all investigated tetrazine derivatives. The notes in Figure 2 are as follows: ***a*** The compounds were obtained as trifluoroacetate salt. ***b*** Comparable blocking data as disclosed in Preparation of Compounds Labeled with Tririum and Carbon-14, 2009, 1-23*. **c*** Comparable blocking data as disclosed in Shalgunov et al., RSC Medicinal Chemistry 2023, 14, 444-453. ***d*** Calculated distribution coefficient at physiological pH (7.4) in Chemicalize software. ***e*** The blocking effect of non-radiolabeled Tz was determined as the change in tumor uptake of [111 In]4 22 h p.i. Each Tz was administered 1 h prior to [111In]DOTA-Tz, and the uptake was normalized to a group of animals in which no blocking was performed (control). Data represents mean from n = 3 mice/group.

The tetrazines that were studied exhibited a blocking effect ranging from 86 to 97%, as indicated in Figure 2. There were no significant differences between the homologues within the same series. Similarly, there was only a small difference between the fluorine and iodine derivatives. These results confirm the success of our design approach, as previously a significant reduction was observed in blocking efficiency from compound **1** (fluorine) to compound **3** (iodine). However, no such decrease was detected for the new compounds. Interestingly, the addition of an extra carboxylic group did not improve the compound's performance in vivo.
Based on these results, compounds **27** and **28** were selected to be translated as theranostic couple with ¹⁸F and ²¹¹At.

### Example 3 - Radiochemistry

### 3.1 [¹⁸F]Fluoride Production and General Methods

[¹⁸F]Fluoride was produced using a cyclotron CTI Siemens Eclipse, Rigshospitalet, Denmark, by irradiating [¹⁸O]H₂O *via* a (p,n) reaction. Automated synthesis was performed on a Scanys synthesis module (Scansys Laboratorieteknik, Denmark), and analytical HPLC was performed on a Thermo Fisher UltiMate 3000 system equipped with a C18 column (Luna 5 µm C18(2) 100 Å and 150 mm × 4.6 mm). Eluents: A, H₂O with 0.1% TFA and B, MeCN with 0.1% TFA. Gradient was from 100% A to 100% B over 15 min and back to 100% A over 4 min with a flow rate of 1.5 mL/min. Detection was performed by UV absorption at λ = 254 nm on a UVD 170U detector, and radioactivity was analyzed with a flow-through GM tube-based radiodetector (Scansys).

### 3.2 [¹⁸F ]Radiolabeling

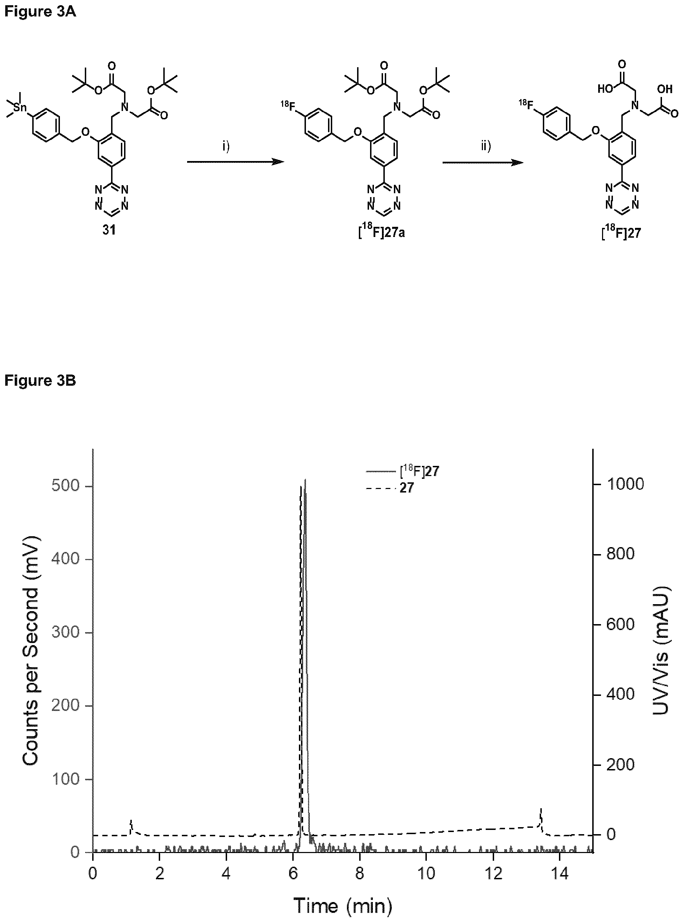

Radiolabeling of [¹⁸F]**27** was carried out using a protection/deprotection strategy in a one-pot, two-step reaction sequence (Figure 3A) to prevent side reactions. Radiolabeling was performed using a copper mediated procedure as previously reported in García-Vázquez et al., Chemical Science 2021, 12, 11668-11675 and in Andersen et al., Molecules 2022, 27*.* Automated synthesis was performed on a Scansys Laboratorieteknik synthesis module housed in a hotcell. An anion exchange cartridge (Sep-Pak Accell Plus QMA Plus Light cartridge, chloride form, Waters) was washed with EtOH (10 mL), 0.5 M K₃PO₄ in H₂O (10 mL), H₂O (10 mL), and dried with air (10 mL). The aqueous [¹⁸F]Fluoride was then passed through this exchange resin and the resin was eluted with 20 mM tetrabutyl ammonium triflate in MeOH (1 mL). The [¹⁸F]TBAF was then dried under a stream of nitrogen and vacuum at 100 °C for 13 min. A solution of the organotin precursor **31** (0.01 mmol), Cu(OTf)₂(Py)₄ (0.015 mmol) in 1 mL DMA (set up shortly before the addition) was added to the reaction vial containing the dried [¹⁸F]TBAF, and the reaction was left at 100 °C for 5 minutes. The solution was then cooled with compressed air down to 55 °C over the term of 2.5 min, before being quenched with 2 mL H₂O + 0.1 % TFA. The cooled reaction mixture was then passed through a Sep-Pak Plus C18 cartridge (SPE), previously preconditioned with EtOH (10 mL), H₂O (10 mL), and air (10 mL). The SPE was then washed with 7 mL H₂O, and dried with air, before being eluted with 3 mL of MeCN into a second reaction vial containing 1 mL TFA. The mixture containing [¹⁸F]**27a** was then heated at 80 °C for 7 min allowing the cleavage of the tert-butyl esters. The reaction mixture was finally concentrated down to 50 - 100 µL at 85 °C under a continuous nitrogen flow for 13 min. The crude reaction mixture was cooled with compressed air down to 30 °C over 4 min. The crude reaction mixture was resolubilized in 2 mL of H₂O + 0.1 % TFA and 1 mL of MeCN and purified by semi-preparative HPLC (Thermo Fisher UltiMate 3000) with a C-18 column (Luna 5 µm C18(2) 100 Å, 250 mm × 10 mm) using an isocratic method (40 % MeCN in H₂O + 0.1 % TFA, flowrate = 4 mL/min). The isolated product eluted after approx. 7.5 min (SI) with a max. activity yield of 143 MBq starting from 16.5 GBq of dry [¹⁸F]TBAF. The product fraction was then diluted in 50 ml H₂O + 0.1 % TFA and passed through a Sep-Pak Light C18 cartridge, which was previously preconditioned with EtOH (10 mL), H₂O (10 mL), and dried with air (10 mL). After the whole product solution had passed through the Sep-Pak Light C18 cartridge, [¹⁸F]**27** was eluted with EtOH (1 mL), dried at 50 °C under a continuous stream of nitrogen for 11 min, and formulated with 0.1 M phosphate buffer (pH 7.4) with a final activity concentration of 70 - 80 MBq/mL, and a max. activity yield of 91.4 MBq after reformulation. The automated synthesis including purification and concentration of [¹⁸F]TBAF, labeling, HPLC separation and formulation was performed over the course of 100 min. [¹⁸F]**27** was afforded in 1.5 % ± 0.6 % (d.c.) over 2 steps, a RCP > 98 % and an Aₘ of 111 GBq/µmol (molar activity of the solution injected into mice).

The identity of [¹⁸F]**27** was determined by comparing the retention time of the product's radio-HPLC trace with the HPLC-UV trace of the respective ¹⁹F-reference **27.** The decay-corrected radiochemical yield (RCY) was determined comparing the activity of dry [¹⁸F]TBAF and that of the product after semi-preparative HPLC purification. The activity of the HPLC-purified product was decay corrected to the time the activity of the dry [¹⁸F]TBAF being measured. The molar activity (Aₘ) of [¹⁸F]**27** was determined by integrating the area of the UV absorbance peak corresponding to the radiolabeled product on the HPLC chromatogram. This area was compared to a standard row of UV peak integrals corresponding to different injected concentrations of **27** (triplicate for each concentration) at the same injection volume, allowing the calculation of the total concentration (in µg/mL or nmol/mL) of Carrier-Tz in the formulated solution. The activity of the HPLC sample of [¹⁸F]**27**, its total volume, as well as the concentration of the Carrier-Tz, allowed the calculation of the Aₘ.

The radiochemical purity (RCP) of the formulated product solution was assessed by integrating the area of the formulated product radio-HPLC trace and comparing its integral to any other radioactive byproducts.

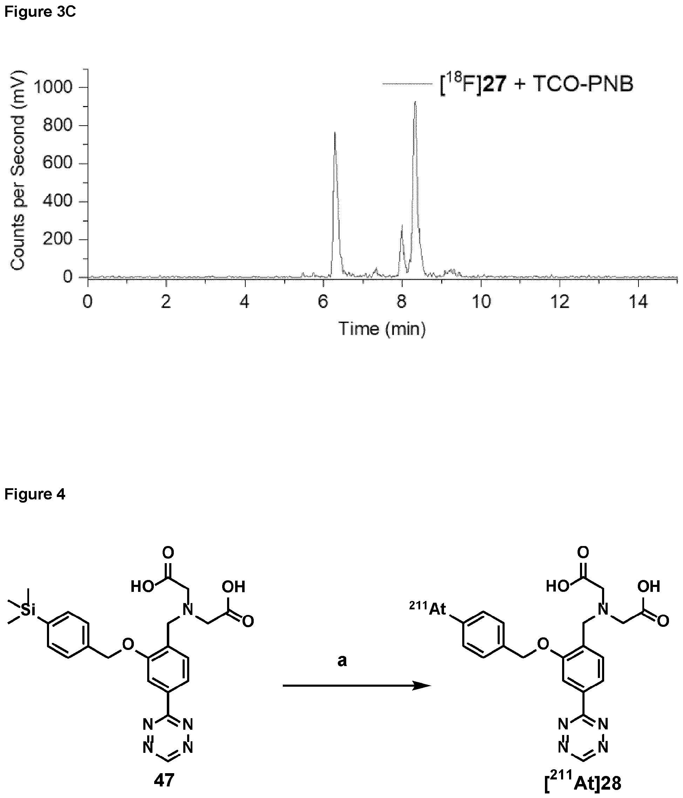

The results are shown in Figure 3A, 3B and 3C. Figure 3A shows the reaction sequence of the radiolabeling of [¹⁸F]**27**. *Reagents and conditions:* i) Conditions for labeling. Cu(OTf)₂(Py)₄, [¹⁸F]TBAF, DMA, 100 °C, 5 min; ii) TFA/MeCN (1/3, 4 mL), 80 °C, 8 min. Figure 3B shows the analytical chromatograms of the formulated [¹⁸F]**27** (Rt: 6.337 min; black graph) and the reference **27** (Rt: 6.223 min; red graph; UV/Vis 254 nm). Figure 3C shows the analytical chromatogram of [¹⁸F]**27** after TCO-PNB addition (Rt: 6.283 min (37.93 %), 7.990 min (9.94 %), 8.330 min (52.13 %)). HPLC conditions: Luna 5 µm C18(2) 100 Å 150 × 4.6 mm eluted with a gradient of MeCN with 0.1 % v/v TFA (solvent B) in water with 0.1 % v/v TFA (solvent A). Gradient conditions: 0-1 min - 0 % B, 1-11 min - 0-100 % B, 11-12 min 100 % B., 12-13min - 100-0 % B, 13-15 min - 0 % B.

[¹⁸F]**27** was labeled in a decay-corrected radiochemical yield (RCY) of 1.5 ± 0.6 % (n = 3) with a radiochemical purity (RCP) of > 98% (n = 3) and a molar activity (A_{M}) of 111 GBq/µmol when starting the synthesis with 16.5 GBq of dried fluoride (Figure 3B) The above values for radiochemical yield (RCY), radiochemical purity (RCP), and molar activity (Aₘ) are given as mean values. The total synthesis time was approximately 100 min including semi-preparative HPLC purification and formulation of the final product. The maximum isolated amount was 143 MBq after semi-preparative HPLC purification.

### 3.3 Tz Core Reactivity test

The reactivity of the tetrazine core was evaluated by reacting [¹⁸F]**27** with *trans-*cyclooctene-p-nitrobenzyl ester (TCO-PNB) and monitoring the reaction by radio-HPLC. The reaction between [¹⁸F]**27** and [²¹¹At]**28** TCO-PNP was performed by mixing the formulated [¹⁸F]**27 or** [²¹¹At]**28** (200 µL) with 5 µL of the commercially available TCO-PNB ester dissolved in DMF (5 mg/mL) in an analytical HPLC vial. The solution was gently shaken and left for 1 min before it was injected on the analytical HPLC for analysis. The result is shown in Figure 3C.

### 3.4 [²¹¹At]Astatine Production

²¹¹At was produced through the ²⁰⁹Bi(α,2n)²¹¹At reaction by irradiating stable bismuth (deposited on an aluminum backing) with 29 MeV α-particles. This reaction was performed using a Scanditronix MC32 cyclotron at the positron emission tomography (PET) and Cyclotron Unit at Copenhagen

University Hospital, Denmark. After production, the irradiated target was transported to the Department of Nuclear Medicine, Sahlgrenska University Hospital, Sweden. The ²¹¹At in the target material was transformed into a chemically useful form by dry distillation as previously reported.⁴³ Radio HPLC analyses were performed on a Jasco LC4000 LC (Jasco Inc.) equipped with a Phenomenex Jupiter Proteo C12 (150 × 4.6 mm, 4 µm - Merck) and a flow-through sodium iodide Nal(TI) well radiodetector. Analyses were perfomed using linear gradients of 0.1% TFA in MiliQ-H₂O (A) and 0.1% TFA, 10% MiliQ-H₂O in MeCN (B).

### 3.5 Radiolabeling of [²¹¹At]28 and synthesis of silyl precursor

For ease of synthesis, the same precursor (**31**) was utilized for the synthesis of [²¹¹At]**28**. The first reaction is the one-pot procedure yielded [²¹¹At]**28a** with an RCC of >90% (Scheme 4). However, the subsequent deprotection of [²¹¹At]**28a** was slow (>3h) and resulted in significant degradation of [²¹¹At]**28**. (RCY 12% - over two steps). For these reasons, we decided to use a new precursor for astatination of **28**, due to its advantage of simplifying the process and saving time. Trimethylsilyl precursors have emerged as a viable option for astatination of compounds via electrophilic desilylation.^{38,39} The main benefit is the possibility of deprotecting the molecules in advance, which helps avoid time-consuming steps on radioactive products. 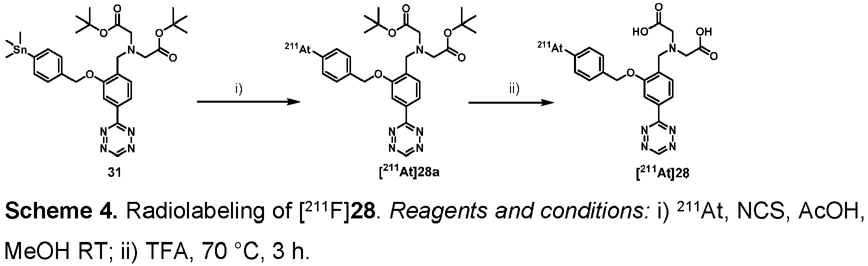 RCY was around 12%; RCP: > 97%.

The silyl precursor **47** was synthesized as shown in Scheme 5. Trimethyl(p-tolyl)silane was brominated at the benzylic position to give **44**. The latter was employed to alkylate **18** giving the intermediate **45**. Tetrazine formation followed by tert-butyl deprotection afforded the desired precursor.

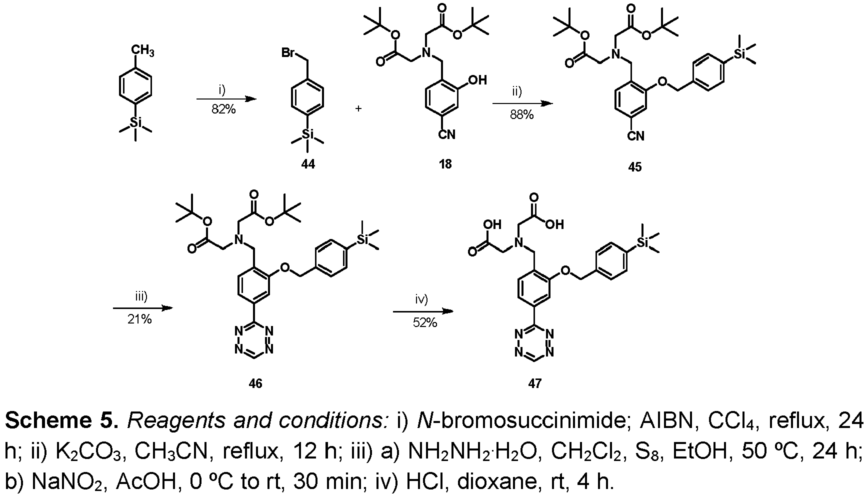

The precursor **47** was then astatinated using conditions reported by Watanabe et al., Organic & Biomolecular Chemistry 2019, 165-171*.* In this experiment, lower precursor loading resulted in a higher RCC. In short, the precursor (47), ²¹¹At, AcOH and NCS were premixed in MeOH. Resulting in the oxidation of the ²¹¹At to ²¹¹AtCl. This was followed by evaporation of the volatiles and subsequently the reaction was redissolved in neat TFA and heated to 70°C, for 10 min.

The reaction mixture was diluted with H₂O and directly submitted to HPLC purification, which yielded [²¹¹At]**28** in a RCY of 93 ± 5 % (n = 3); RCP: > 99%.

The astatination of [²¹¹At]**28** with silyl precursor **47** is shown in Figure 4 wherein a) is the two-step reaction i) ²¹¹At, NCS, AcOH, MeOH RT, and ii) TFA, 70 °C, 10 min.

### 3.6 Radiolabeling of [¹³¹I]28 from silyl precursor 47

The silyl precursor **47** was synthesized as shown in Scheme 5. The iodination procedure is shown in scheme 6.

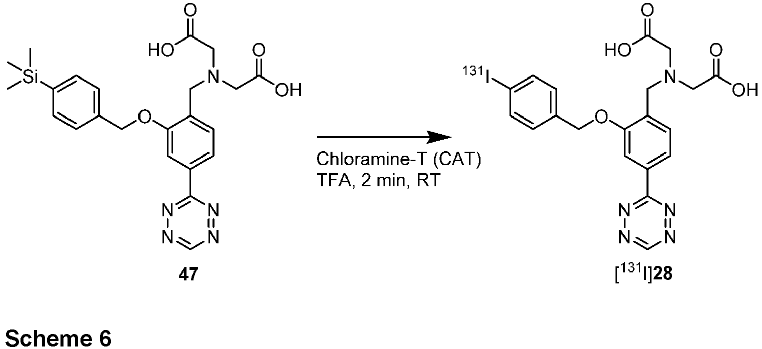

[¹³¹1]Nal (450-550 MBq, 20-25µL in 0.5 M carbonate buffer (pH=10,2-10,8)) was evaporated to dryness with N₂ at 40 °C (ca. 5 min). A solution of precursor **47** (50 µg, 10 µL, 5 mg/mL in MeOH) and chloramine-T (25 µg, 1.25 µL, 20 mg/mL in MeOH) was evaporated to dryness with N₂, redissolved in TFA (100 µL) and added to

[¹³¹1]Nal. After 2 min at r.t., TFA was mostly evaporated with N₂ at 40 °C (ca. 3 min), diluted with 100 µL ACN and 600 µL H₂O and purified via semipreparative HPLC (tᵣₑₜ = 8.5-9.5 min, Luna C18(2) 5µm 250x10 mm column; mobile phase H₂O/ACN + 0.1% TFA (40% ACN in 11 min; flow 4 mL/min). The collected product peak was diluted with ca. 10 mL H₂O and passed through Sep-Pak tC2 Plus Light cartridge (Waters; preconditioning: 2.5 mL EtOH, 5 mL H₂O). The Sep-Pak cartridge was washed with water (1 mL) and eluted with 0.6 mL of EtOH/H₂O (1:1) with gentisic acid (2 mg/mL) or alternatively with 0.6 mL CAN + 0.1% TFA.

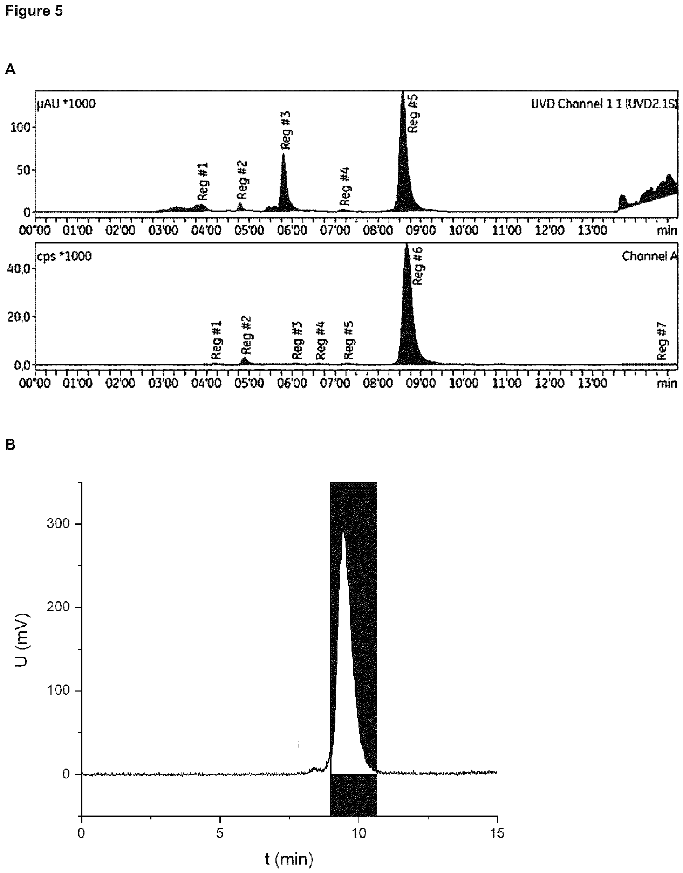

Semipreparative radio-HPLC of [¹³¹I]-**28** is shown in Figure 5A. The HPLC was made using Luna C18(2) 5µm 250x10 mm column; mobile phase H₂O/ACN + 0.1% TFA (40% ACN in 11 min; 100% ACN in 2 min; 40% ACN in 2 min; flow 4 mL/min. (tᵣₑₜ = 8.5-9.5 min).

Analytical radio-HPLC of [¹³¹I]-**28** is shown in Figure 5B. The HPLC was made using Luna C18(2) 5µm 150x4.6 mm column; mobile phase H₂O/ACN + 0.1% TFA (20-50% ACN in 11 min; 100% ACN in 2 min; 20% ACN in 2 min; flow 0.5 mL/min, (tᵣₑₜ = 9.4-9.5 min).

### Example 4 - Pretargeted PET-Imaging of [¹⁸F]27

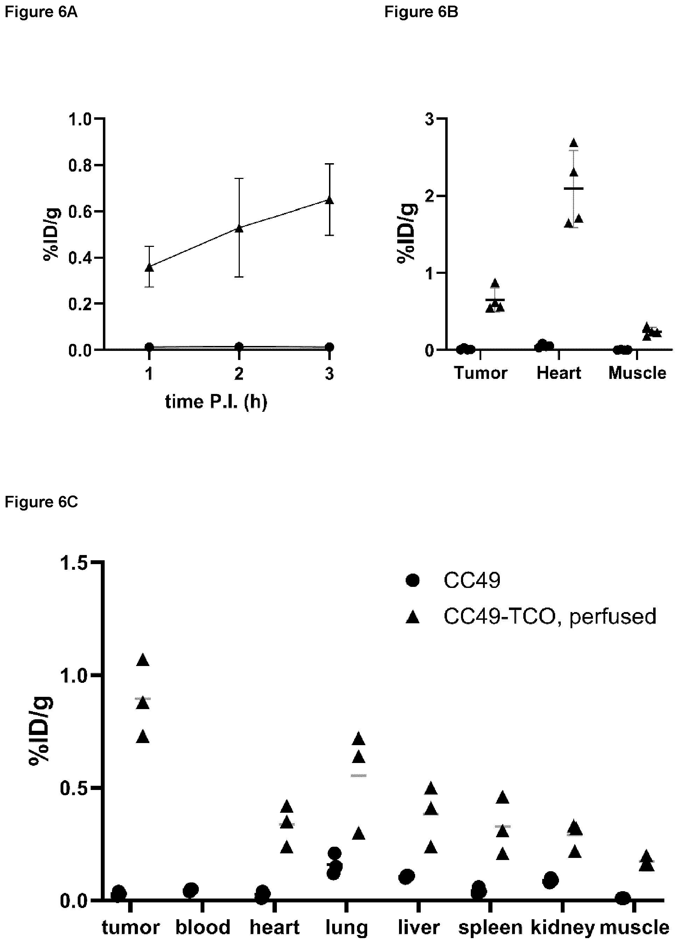

In order to evaluate the ability of [¹⁸F]**27** to be used as a pretargeting imaging agent, a similar setup was used as in the ex vivo blocking study described in Stéen et al., ACS Pharmacology and Translational Science 2021, 824 - 833. All tetrazines displayed rate constant values >55.000 M⁻¹s⁻¹ .Intravenous injection of CC49-TCO preceded [¹⁸F]**27** administration by 72 hours, and subsequent PET/CT scans were performed one, two and three hours later. The control group was given unconjugated mAb CC49 instead of CC49-TCO but was otherwise treated in the same manner. Following PET/CT, the animals were euthanized, and an ex vivo biodistribution was carried out. To determine specific tumor binding, animals pretreated with CC49-TCO were transcardially perfused with saline, as to remove tumor signal arising from resident blood. The results are presented in Figure 6.

Specifically, pretargeted imaging of [¹⁸F]**27** was tested *in vivo* using the TAG-72-targeting antibody CC49 in human colorectal cancer xenograft tumors LS174T. Tumors were established in 7-8 week old female inbred Balb/c nude mice (janvier), and after 7-10 days , the animals were injected i.v. with either 50 µg of TCO-modified CC49 (CC49-TCO, ~7 TCO/mAb, 2 nmol TCO/mouse), or non-modified CC49 (control) (*n* = 4 per group). 72 h later, 4.82±0.64 MBq [¹⁸F]**27** in 100 uL sterile PBS was injected and animals were subsequently PET/CT scaned at 1,2 and 3 hours P.I. (Inveon, Siemens Medical Solutions, USA). 4 mice were scanned simultaneously (ref lewis) by 360 projections at 65 kV x-ray tube voltage, 500 uA anode current, 440 ms exposure time. CT images were downsampled twice. Immediately after CT scan, a 10 min PET scan was initiated, with an energy window of 350-650 keV and 3.438 ns timing window. PET images were reconstructed using the MAP-OSEM algoritm with CT based attenuation and scatter correction. 3D regions of interest were drawn on CT images, and corresponding activities derived from PET images. Mass was assumed to be 1g/mL, and from these data, %ID/g was calculated by decay correcting activities to injection time and normalizing to mass. After the final scan acquisition had completed, mice pretreated with CC49-TCO were transcardially perfused with saline to remove the blood. Control animals were euthanized, and the following organs and tissues resected from all animals for automated gamma counting (Hidex, Finland): Tumor, blood, heart, lung liver, spleen, kidney and muscle. Figure 6A shows the PET-derived biodistribution of [¹⁸F]**27** as %ID/g for tumor uptake at 1, 2 and 3h P.I. and Figure 6B shows the PET-derived biodistribution of [¹⁸F]**27** in tumor, heart (blood surrogate) and muscle tissue %ID/g 3h P.I. Triangles indicate CC49-TCO and circles CC49 control animals. n=4, each group.

The PET/CT data revealed that pre-treated mice with CC49-TCO had a notably higher tumor uptake of 0.65 ± 0.16 % lA/g compared to the control group, which had only 0.01 ± 0.01 %IA/g (mean ± S.D, n = 4) (Figure 6C). The tumor uptake was clearly visible in the imaging. Three hours after injection, image derived tumor-to-muscle (T/M) and tumor-to-blood (T/B) ratios were 2.7 and 0.31 respectively (here, heart ventricle uptake was used as a surrogate for blood uptake). The relatively low T/B ratio, which is a common finding in pretargeted imaging, is likely due to the binding of [¹⁸F]**27** with the remaining CC49-TCO still circulating in the bloodstream. Clearance of CC49-TCO from blood will most likely increase the ratio drastically. Uptake in all other tissues was low. Ex vivo biodistribution confirmed that tumor uptake remained at similar level to that determined by PET after removal of resident blood (Figure 6).

### Example 5 - Pretargeted PET-imaging of Biodistribution of [²¹¹At]28

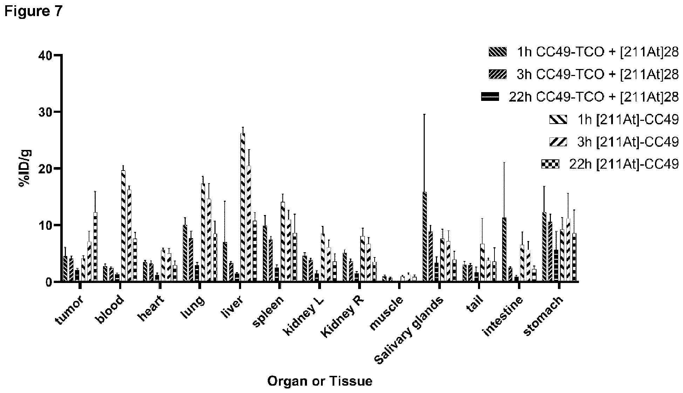

For biodistribution assessment of [²¹¹At]**28** the animal model was established as described above in Example 4. When tumors had reached size of 154.1729 ± 67.39566 they were randomized to six groups (n=5 each). Three groups were pretreated with CC49-TCO 72 hours prior to injection with 100 kBq [²¹¹At]**28** in 100 uL sterile PBS. Separately, three other groups were injected with 100 kBq directly labelled CC49 antibody, labelled by reacting [²¹¹At]28 with CC49-TCO in aqueous solution at room temperature. One, three and 24 hours after tail vein injection of [²¹¹At]28, mice were euthanized and the following organs and tissues resected: tumor, blood, heart, lung, liver, spleen, right and left kidney, muscle, salivary glands, thyroids (throat), tail, intestine and stomach. All organs were weighed and automated gamma counted (Hidex, Finland) using a 60-100 kEv energy window. Standards of 10% injected dose was included for normalization. The Ex vivo biodistribution of [²¹¹At]**28** expressed as %ID/g is shown in Figure 7.

## Claims

1. Tetrazine compound of formula I 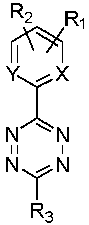 wherein R₁ is 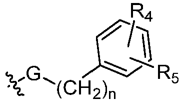
wherein G is selected from -O-, -S-, -NH-, and -N-alkyl-;
n is a digit from 0 to 4;
R₄ is a radioisotope selected from ¹⁸F, ¹⁹F, ⁷⁶Br, ⁷⁷Br, ¹²⁵I, ¹²³I, ¹³¹I, ¹²⁴I, ¹²⁷I and ²¹¹At;
R₅ is -H- or a polar group (PG) selected from: 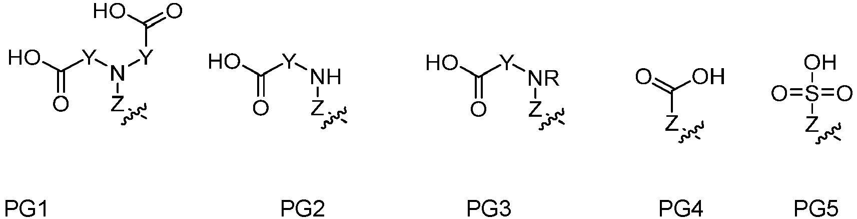
wherein Z is CO(CH₂)ₙ, SO₂(CH₂)ₙ, (CH₂)ₙ or (OCH₂CH2)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 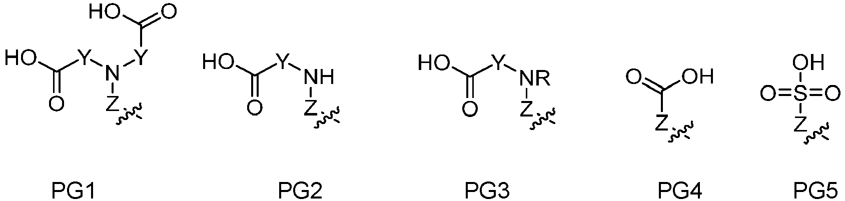
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 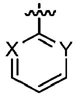
wherein X and Y are independently selected from: -CH₂- and -N-;
and wherein the tetrazine compound of formula I has a lipophilicity of cLogD7.4 < - 0.5.

2. Compound of formula I 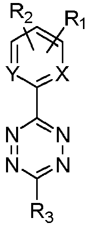 wherein R₁ is 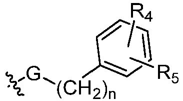
wherein G is selected from -O-, -S-, -NH-, and -N-alkyl-;
n is a digit from 0 to 4;
R₄ is a radioisotope selected from ¹⁸F, ⁷⁶Br, ⁷⁷Br, ¹²⁵I, ¹²³I, ¹³¹I, ¹²⁴I, and ²¹¹At;
R₅ is -H- or a polar group (PG) selected from: 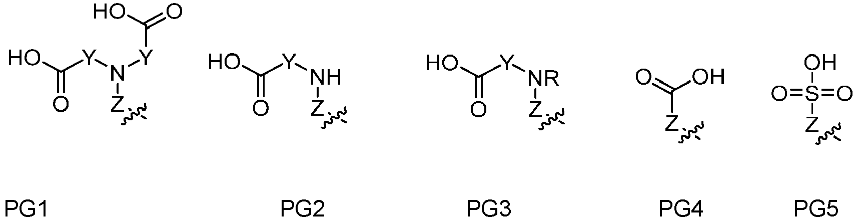
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 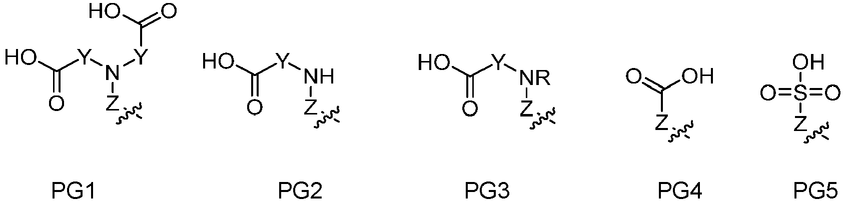
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 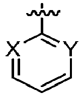
wherein X and Y are independently selected from: -CH₂- and -N-;
and
wherein the tetrazine compound of formula I has a lipophilicity of cLogD7.4 < - 0.5
for use as a medicament in pretargeting based therapy, diagnostic, theranostic or imaging.

3. Compound of formula I 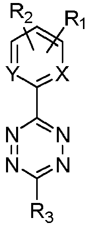 wherein R₁ is 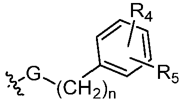
wherein G is selected from -O-, -S-, -NH-, and -N-alkyl-;
n is a digit from 0 to 4;
R₄ is a radioisotope selected from ¹⁸F, ⁷⁶Br, ⁷⁷Br, ¹²⁵I, ¹²³I, ¹³¹I, ¹²⁴I, and ²¹¹At; R₅ is -H- or a polar group (PG) selected from: 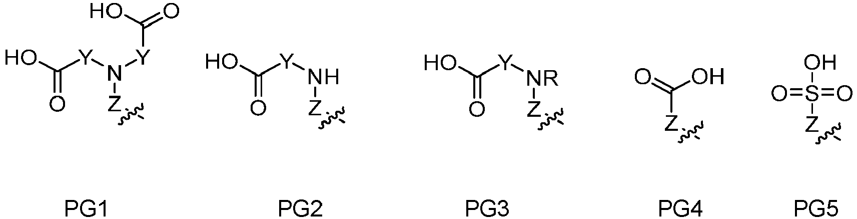
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 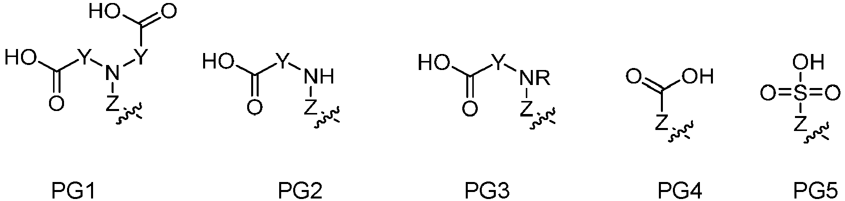
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 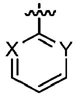
wherein X and Y are independently selected from: -CH₂- and -N-;
and
wherein the tetrazine compound of formula I has a lipophilicity of cLogD7.4 < - 0.5
for use in a method of diagnosing and/or treating cancer diseases, cardiovascular diseases, infectious diseases, neurological disorders, inflammatory diseases, bone disorders, thyroid diseases, renal diseases, or lymphatic disorders.

4. Compound of formula I for use in a method according to claim 3, wherein said method comprises:
a) administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream
c) Administering to the same subject a compound of formula I having complementary inverse electron demand Diels-Alder cycloaddition reactivity to the dienophile.

5. Compound for use in a method according to claim 4, wherein R₄ in formula I is a radioisotope selected from ¹⁸F, ¹²³I, ¹³¹I, ¹²⁴I, and wherein the method is diagnostic further comprising subjecting the subject to PET-scanning when the radioisotope in formula I is ¹⁸F, ⁷⁶Br, or ¹²⁴I, or subjecting the subject to SPECT-scanning when the radioisotope in formula I is ¹²³I or ¹³¹I.

6. Compound for use in a method according to claim 4, wherein R₄ in formula I is a radioisotope selected from ⁷⁷Br,¹²⁵I, ¹³¹I, ²¹¹At, and the method is therapeutic.

7. Compound for use in a method according to claim 4, wherein R₄ in formula I is a radioisotope selected from ¹⁸F, ⁷⁶Br,¹²³I, ¹³¹I, ¹²⁴I, and wherein the method is a theranostic method comprising:
a) administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream,
c) Administering to the same subject a compound of formula I having complementary inverse electron demand Diels-Alder cycloaddition reactivity to the dienophile,
d) Optionally repeating step a) and step b),
e) Administering to the same subject a compound of formula I and having complementary inverse electron demand Diels-Alder cycloaddition reactivity to the dienophile, and wherein R₄ in formula I is a radioisotope selected from ⁷⁷Br,¹²⁵I, ¹³¹I, and ²¹¹At.

8. Compound for use in a method according to claim 4, wherein the method comprises:
a) Administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream,
c) Administering to the same subject as in step a) a compound of formula I having complementary inverse electron demand Diels-Alder cycloaddition reactivity to the dienophile, wherein R₄ in formula I is a radioisotope selected from ¹⁸F, ⁷⁶Br, ¹²³I, ¹³¹I, ¹²⁴I, and subjecting the subject to PET or SPECT,
d) Optionally repeating step a) and step b),
e) Administering to the same subject as in step c) a compound of formula I and having complementary inverse electron demand Diels-Alder cycloaddition reactivity to the dienophile, and wherein R₄ in formula I is a radioisotope selected from ⁷⁷Br,¹²⁵I, ¹³¹I, and ²¹¹At,
f) Administering to the same subject as in step e) a dienophile with inverse electron demand Diels-Alder cycloaddition reactivity being conjugated to a targeting vector targeting specific antigens in the subject,
g) Allowing the targeting vector time to bind to the target, and for unbound targeting vectors to clear from the bloodstream,
h) Administering to the same subject as in step f) a compound of formula I having complementary inverse electron demand Diels-Alder cycloaddition reactivity to the dienophile, wherein R₄ in formula I is a radioisotope selected from ¹⁸F, ⁷⁶Br, ¹²³I, ¹³¹I, ¹²⁴I, and subjecting the subject to PET or SPECT,
i) Optionally, repeating step e) or steps e) to h).

9. Method comprising:
a) administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream
c) Administering to the same subject a compound of formula I: 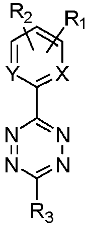 wherein R₁ is 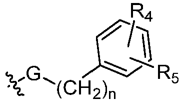
wherein G is selected from -O-, -S-, -NH-, and -N-alkyl-;
n is a digit from 0 to 4;
R₄ is a radioisotope selected from ¹⁸F, ⁷⁶Br, ⁷⁷Br, ¹²⁵I, ¹²³I, ¹³¹I, ¹²⁴I, and ²¹¹At;
R₅ is -H- or a polar group (PG) selected from: 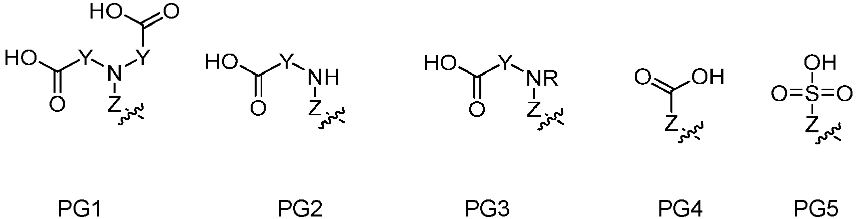
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 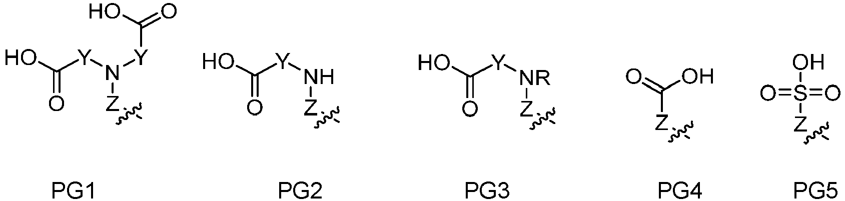
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 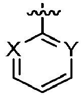
wherein X and Y are independently selected from: -CH₂- and -N-;
and
wherein the tetrazine compound of formula I has a lipophilicity of cLogD7.4 < -0.5, and have complementary inverse electron demand Diels-Alder cycloaddition reactivity to the dienophile.

10. Method according to claim 9, wherein R₄ in formula I is a radioisotope selected from ¹⁸F, ⁷⁶Br, ¹²³I, ¹³¹I, ¹²⁴I, and wherein the method is diagnostic further comprising subjecting the subject to PET-scanning when the radioisotope in formula I is ¹⁸F or ¹²⁴I, or subjecting the subject to SPECT-scanning when the radioisotope in formula I is ¹²³I or ¹³¹I.

11. Method according to claim 9, wherein R₄ in formula I is a radioisotope selected from ¹⁸F, ⁷⁶Br, ¹²³I, ¹³¹I, ¹²⁴I, and wherein the method is a pre-targeting theranostic method further comprising:
d) Optionally repeating step a) and step b)
e) Administering to the same subject a compound of formula I and having complementary inverse electron demand Diels-Alder cycloaddition reactivity to the dienophile, and wherein R₄ in formula I is a radioisotope selected from ⁷⁷Br, ¹²⁵I, ¹³¹I, and ²¹¹At.

12. Method according to claim 9, comprising:
a) Administering to a subject a dienophile with inverse electron demand Diels-Alder cycloaddition reactivity being conjugated to a targeting vector targeting specific antigens in the subject,
b) Allowing the targeting vector time to bind to the target, and for unbound targeting vectors to clear from the bloodstream,
c) Administering to the same subject as in step a) a compound of formula I having complementary inverse electron demand Diels-Alder cycloaddition reactivity to the dienophile, wherein R₄ in formula I is a radioisotope selected from ¹⁸F, ⁷⁶Br, ¹²³I, ¹³¹I, ¹²⁴I, and subjecting the subject to PET or SPECT,
d) Optionally repeating step a) and step b),
e) Administering to the same subject as in step c) a compound of formula I and having complementary inverse electron demand Diels-Alder cycloaddition reactivity to the dienophile, and wherein R₄ in formula I is a radioisotope selected from ⁷⁷Br, ¹²⁵I, ¹³¹I, and ²¹¹At,
f) Administering to the same subject as in step e) a dienophile with inverse electron demand Diels-Alder cycloaddition reactivity being conjugated to a targeting vector targeting specific antigens in the subject,
g) Allowing the targeting vector time to bind to the target, and for unbound targeting vectors to clear from the bloodstream,
h) Administering to the same subject as in step f) a compound of formula I having complementary inverse electron demand Diels-Alder cycloaddition reactivity to the dienophile, wherein R₄ in formula I is a radioisotope selected from ¹⁸F, ⁷⁶Br, ¹²³I, ¹³¹I, ¹²⁴I, and subjecting the subject to PET or SPECT,
i) Optionally, repeating step e) or steps e) to h).

13. Precursor of the Tetrazine compound according to claim 1 of formula II: 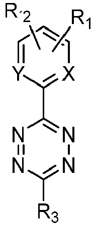 wherein R₁ is 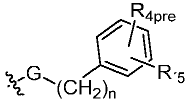
wherein G is selected from -O-, -S-, -NH-, and -N-alkyl-;
n is a digit from 0 to 4;
R₄ₚᵣₑ is a group selected from T₃Sn, T₃Si or T₃Ge, wherein T is a; straight chain or branched alkyl group, independently selected from methyl, ethyl, propyl, butyl, pentyl, hexyl, heptyl, octyl, nonyl, and decyl,
R'₅ is -H- or a polar group (PG) selected from: 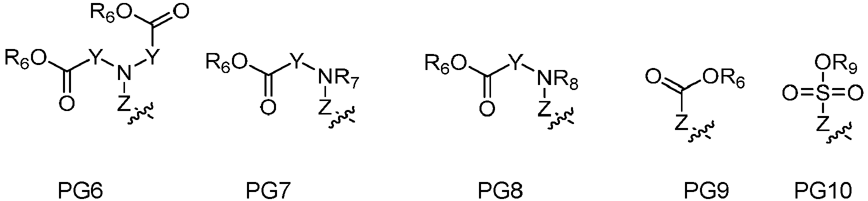
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or (OCH₂CH₂)ₙOCH₂COOC(CH₃)₃, wherein n is a digit from 0 to 3;
R₉ is selected from -H, neopentyl, and α-trifluoromethylbenzyl;
and wherein the curly sign indicates the link to the aromatic ring,
R'₂ is -H- or a polar group (PG) selected from: 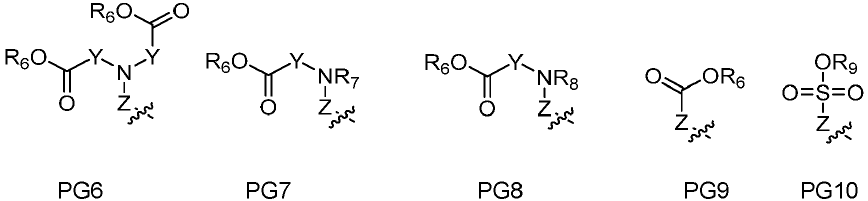
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or (OCH₂CH₂)ₙOCH₂COOC(CH₃)₃, wherein n is a digit from 0 to 3;
R₉ is selected from -H, neopentyl, and α-trifluoromethylbenzyl;
and wherein the curly sign indicates the link to the aromatic ring
R₃ is -H or 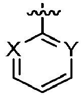
wherein X and Y are independently selected from: -CH₂- and -N-, and wherein the curly sign indicates the link to the aromatic ring.

14. A kit comprising the tetrazine compound according to formula I: 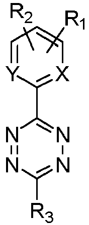 wherein R₁ is 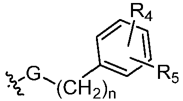
wherein G is selected from -O-, -S-, -NH-, and -N-alkyl-;
n is a digit from 0 to 4;
R₄ is a radioisotope selected from ¹⁸F, ¹⁹F, ⁷⁶Br, ⁷⁷Br, ¹²⁵I, ¹²³I, ¹³¹I, ¹²⁴I, ¹²⁷I and ²¹¹At
R₅ is -H- or a polar group (PG) selected from: 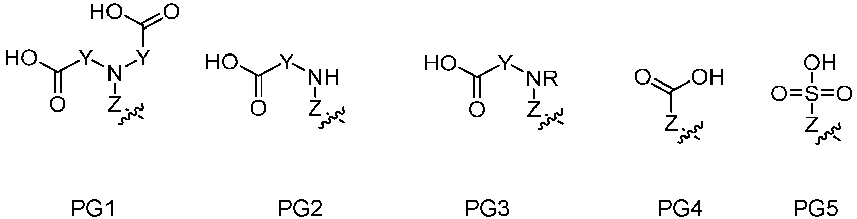
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n is a digit from 0 to 3; and
wherein R is (CH₂)ₙCH₃, (OCH₂CH₂)ₙOH, CO(CH₂)ₙCOOH, (OCH₂CH₂)ₙOCH₂COOH and n is a digit from 0 to 3;
and wherein the curly sign indicates the link to the aromatic ring;
R₂ is -H- or a polar group (PG) selected from: 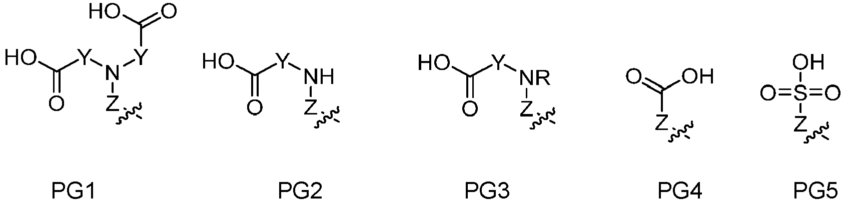
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring;
R₃ is -H or 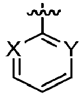
wherein X and Y are independently selected from: -CH₂- and -N-;
and
wherein the tetrazine compound of formula I has a lipophilicity of cLogD7.4 < -0.5;
or a precursor of formula I having the structure of formula II: 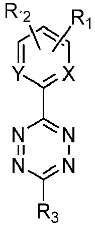 wherein R₁ is 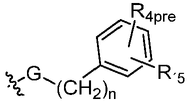
wherein G is selected from -O-, -S-, -NH-, and -N-alkyl-;
n is a digit from 0 to 4;
R₄ₚᵣₑ is a group selected from T₃Sn, T₃Si or T₃Ge, wherein T is a; straight chain or branched alkyl group, independently selected from methyl, ethyl, propyl, butyl, pentyl, hexyl, heptyl, octyl, nonyl, and decyl,
R'₅ is -H- or a polar group (PG) selected from: 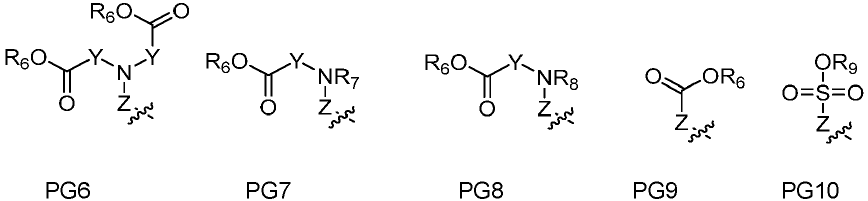
wherein Z is CO(CH₂)ₙ, SO₂(CH₂)ₙ, (CH₂)ₙ or (OCH₂CH₂)ₙ and n = 0, 1, 2 or 3; and wherein Y is (CH₂)ₙ, (OCH₂CH₂)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, (OCH₂CH₂)ₙOCH₂COOC(CH₃)₃, and n is a digit from 0 to 3;
R₉ is selected from -H, neopentyl, and α-trifluoromethylbenzyl;
and wherein the curly sign indicates the link to the aromatic ring,
R'₂ is -H- or a polar group (PG) selected from: 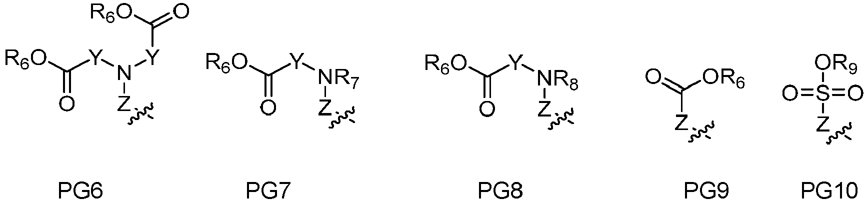
wherein Z is CO(CH₂)ₙ, SO₂(CH₂)ₙ, (CH₂)ₙ or (OCH₂CH₂)ₙ and n = 0, 1, 2 or 3; and
wherein Y is (CH₂)ₙ, (OCH₂CH₂)ₙ or CO(CH₂)ₙ and n is a digit from 0 to 3;
and wherein R₆ is selected from -H, tert-butyl, and trityl;
R₇ is -H or Boc;
R₈ is (CH₂)ₙCH₃, (OCH₂CH₂)ₙOH, CO(CH₂)ₙCOOH, (OCH₂CH₂)ₙOCH₂COOH, (OCH₂CH₂)ₙOSi(CH₃)₂C(CH₃)₃, CO(CH₂)ₙCOOC(CH₃)₃, or (OCH₂CH₂)ₙOCH₂COOC(CH₃)₃, wherein n is a digit from 0 to 3;
R₉ is selected from -H, neopentyl, and α-trifluoromethylbenzyl;
and wherein the curly sign indicates the link to the aromatic ring,
R₃ is -H or 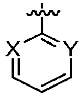
wherein X and Y are independently selected from: -CH₂- and -N-, and wherein the curly sign indicates the link to the aromatic ring;
and a dienophile having complementary inverse electron demand Diels-Alder cycloaddition reactivity.

15. A kit according to claim 14 wherein said dienophile having complementary inverse electron demand Diels-Alder cycloaddition reactivity is conjugated to a targeting vector, such as a targeting vector selected from an antibody, a nanobody, a polymer, a nanomedicine, a cell, a protein, a peptide, or a small molecule.
